# EUROPEAN PATENT SPECIFICATION

(11) **EP 2 321 414 B1**
(45) Date of publication and mention of the grant of the patent: **10.01.2018**
(21) Application number: 09790796.8
(22) Date of filing: 24.07.2009
(51) Int. Cl.: C12N 15/113, C07H 21/02

(54) **ENHANCEMENT OF SIRNA SILENCING ACTIVITY USING UNIVERSAL BASES OR MISMATCHES IN THE SENSE STRAND**
VERBESSERUNG DER SIRNA-SILENCING-AKTIVITÄT UNTER VERWENDUNG UNIVERSELLER BASEN ODER FEHLPAARUNGEN IM SENSE-STRANG
AMÉLIORATION DE L'ACTIVITÉ D'EXTINCTION D'ARNSI UTILISANT DES BASES UNIVERSELLES OU DES NON-APPARIEMENTS DANS LE BRIN SENS

(30) Priority: 25.07.2008 US 83763 P
(43) Date of publication of application: 18.05.2011
(73) Proprietor: Alnylam Pharmaceuticals, Inc., Cambridge, MA 02142 (US)
(72) Inventor: MAIER, Martin, Cambridge, MA 02142 (US); ADDEPALLI, Haripriya, Cambridge, MA 02142 (US); MANOHARAN, Muthiah, Cambridge, MA 02142 (US)
(74) Representative: Müller-Boré & Partner Patentanwälte PartG mbB
(86) International application number: PCT/US2009/051648
(87) International publication number: WO 2010/011895

(56) References cited:
- WO-A2-2007/053696
- WO-A2-2009/120878
- US-A1- 2007 054 279
- XIA JIE ET AL: "Gene silencing activity of siRNAs with a ribo-difluorotoluyl nucleotide" ACS CHEMICAL BIOLOGY, vol. 1, no. 3, 17 April 2006 (2006-04-17), pages 176-183, XP002554979
- JUDGE ET AL: "Design of Noninflammatory Synthetic siRNA Mediating Potent Gene Silencing in Vivo" MOLECULAR THERAPY, vol. 13, no. 3, 1 March 2006 (2006-03-01), pages 494-505, XP005326762 ISSN: 1525-0016
- MUHONEN PIRKKO ET AL: "RNA interference tolerates 2'-fluoro modifications at the Argonaute2 cleavage site." CHEMISTRY & BIODIVERSITY, vol. 4, no. 5, May 2007 (2007-05), pages 858-873, XP002554980 ISSN: 1612-1880
- PRAKASH T P ET AL: "Positional effect of chemical modifications on short interference RNA activity in mammalian cells" JOURNAL OF MEDICINAL CHEMISTRY, AMERICAN CHEMICAL SOCIETY, WASHINGTON, US, vol. 48, no. 13, 27 May 2005 (2005-05-27), pages 4247-4253, XP002404764 ISSN: 0022-2623

## Description

The present invention relates to double-stranded iRNA agents with increased RNAi silencing activity, wherein the iRNA agents comprise two 21 nucleotide-long strands, wherein the strands form a double-stranded region of 19 consecutive base pairs having a two-nucleotide overhang at the 3'-end, wherein the cleavage site region corresponds to positions 9-12 from the 5'-end of the sense strand, said double-stranded iRNA agents comprising:
a. an antisense strand that is complementary to a target gene; and
b. a sense strand that is complementary to said antisense strand and comprises at least one of the following modified nucleotides in the following positions from the 5' end:
   (i) a ribonebularine in position 9;
   (ii) a ribo-2-aminopurine in position 12; and
   (iii) an abasic nucleotide in position 10.

Oligonucleotides and their analogs have been developed for various uses in molecular biology, including uses as probes, primers, linkers, adapters, and gene fragments. In a number of these applications, the oligonucleotides specifically hybridize to a target nucleic acid sequence. Hybridization is the sequence specific hydrogen bonding of oligonucleotides via Watson-Crick and/or Hoogsteen base pairs to RNA or DNA. The bases of such base pairs are said to be complementary to one another.

Double-stranded RNA molecules (dsRNA) can block gene expression by virtue of a highly conserved regulatory mechanism known as RNA interference (RNAi). Briefly, RNA III Dicer, an enzyme, processes dsRNA into small interfering RNA (also sometimes called short interfering RNA or siRNA) of approximately 22 nucleotides. One strand of the siRNA (the "antisense strand") then serves as a guide sequence to induce cleavage by an RNA-induced silencing complex, RISC, of messenger RNAs (mRNAs) including a nucleotide sequence which is at least partially complementary to the antisense strand. The antisense strand is not cleaved or otherwise degraded in this process, and the RISC including the antisense strand can subsequently affect the cleavage of further mRNAs.

During the RISC assembly process the passenger (or sense) strand is generally cleaved between positions 9 and 10, and subsequently separated from the complementary guide (or antisense) strand to generate the active RISC complex. (See Matranga, C. et al. (2005) Passenger-Strand Cleavage Facilitates Assembly of siRNA into Ago2-Containing RNAi Enzyme Complexes. Cell 123, 607-620.) The passenger strand is cleaved during the course of RISC assembly, and certain chemical modifications at this putative cleavage site in the passenger strand can severely impair silencing activity. (See Leuschner P.J.F., Ameres S.L., et al. (2006). Cleavage of the siRNA passenger strand during RISC assembly in human cells. EMBO reports 7, 314-20.) It is greatly desired that oligonucleotides be able to be synthesized to have customized properties which are tailored for particular uses. Described herein is the placement of nucleotides bearing certain base modifications, such as the universal bases 2,4-difluorotoluyl or 5-nitroindole, in the central region of the sense strand (*e.g.,* region around the cleavage site, e.g. 2 nucleotides on both side of the cleavage site, e.g. nucleotides 9 to 12) or the placement of one or more mismatches in this central region or both, so as to improve the potency of siRNA, including the silencing activity of siRNA.

In this context, WO 2007/053696 A2 describes compositions and methods for modulating the expression of influenza viral genes by chemically modified oligonucleotides. Further, US 2007/0054279 A1 describes double-stranded oligonucleotides comprising at least one ligand tethered to an altered or non-natural nucleobase.

The present invention relates to a double-stranded iRNA agent with increased RNAi silencing activity, wherein the iRNA agent comprises two 21 nucleotide-long strands, wherein the strands form a double-stranded region of 19 consecutive base pairs having a two-nucleotide overhang at the 3'-end, wherein the cleavage site region corresponds to positions 9-12 from the 5'-end of the sense strand, said double-stranded iRNA agent comprising:
a. an antisense strand that is complementary to a target gene; and
b. a sense strand that is complementary to said antisense strand and comprises at least one of the following modified nucleotides in the following positions from the 5' end:
   (i) a ribonebularine in position 9;
   (ii) a ribo-2-aminopurine in position 12; and
   (iii) an abasic nucleotide in position 10.

The figures show:
**Figure 1** depicts graphically the results from assays of silencing of firefly expression by modified siRNA (mismatch walk on Luc sense strand, position 9-12) compared to parent duplex AD-1000 (average of three plates).
**Figure 2** depicts graphically the results from assays of silencing of firefly expression by modified siRNA (mismatch walk on Luc sense strand, position 1-4) compared to parent duplex AD-1000 (average of 3 plates).
**Figure 3** depicts graphically the results from assays of silencing of firefly expression by modified siRNA (mismatch walk on Luc sense strand, position 4-7) compared to parent duplex AD-1000 (average of three plates).
**Figure 4** depicts graphically the results from assays of silencing of firefly expression by modified siRNA (mismatch walk in Luc sense strand, position 7-8 and 13-14) compared to parent duplex AD-1000 (average of 3 plates).
**Figure 5** depicts graphically the results from assays of silencing of firefly expression by modified siRNA (mismatch walk on Luc sense strand, position 15-18) compared to parent duplex AD-1000 (average of three plates).
**Figure 6** depicts graphically the results from assays of silencing of firefly expression by modified siRNA (mismatch walk on Luc sense strand, position 18-19) compared to parent duplex AD-1000 (average of 3 plates).
**Figure 7** depicts graphically the results from assays of silencing of firefly expression by modified siRNA (2,4-difluorotoluyl ribonucleotide at position 10 and 11 in the Luc sense strand) compared to parent duplex AD-1000 (average of three plates).
**Figure 8** depicts graphically the results from assays of silencing of firefly expression by modified siRNA (2,4-difluorotoluyl ribonucleotide at position 9 and 12 in the Luc sense strand) compared to parent duplex (AD-1000) in HeLa Dual Lus cells.
**Figure 9** depicts graphically the results from assays of silencing of firefly expression by modified siRNA (2,4-difluorotoluyl deoxyribonucleotide at position 9-12 in the Luc sense strand) compared to parent duplex (AD-1000) in HeLa Dual Lus cells.
**Figure 10** depicts graphically the results from assays of silencing of firefly expression by modified siRNA (5-nitroindole ribo-and deoxyribonucleotide at position 9-12 in the Luc sense strand) compared to parent duplex (AD-1000) in HeLa Dual Lus cells.
**Figure 11** depicts graphically the results from assays of silencing of firefly expression by modified siRNA (ribo- and deoxyribonebularine at position 9-12 in the Luc sense strand) compared to parent duplex (AD-1000) in HeLa Dual Lus cells.
**Figure 12** depicts graphically the results from assays of silencing of firefly expression by modified siRNA (ribo- and deoxyriboinosine at position 9-12 in the Luc sense strand) compared to parent duplex (AD-1000) in HeLa Dual Lus cells.
**Figure 13** depicts graphically the results from assays of silencing of firefly expression by modified siRNA (ribo- and deoxyrib-2-aminopurine at position 9-12 in the Luc sense strand) compared to parent duplex (AD-1000) in HeLa Dual Lus cells.
**Figure 14** depicts IC₅₀ values across all modifications plotted against thermal stability of the corresponding siRNA duplexes.
**Figure 15** depicts graphically the results from assays of silencing of firefly expression by modified siRNA (siRNAs containing the abasic modification 2-hydroxymethyl-tetrahydrofurane-3-phosphate at position 9-12 in the strand) compared to parent duplex (AD-1000) in HeLa Dual Lus cells, plus corresponding IC₅₀ values.
**Figure 16** depicts graphically the results from assays of silencing of firefly expression by modified siRNA (siRNAs containing a bulge at (a) positions 9-11 and (b) position 12 in the sense strand) compared to parent duplex (AD-1000) in HeLa Dual Lus cells.
**Figure 17** depicts graphically the results from assays of dose-dependent silencing of PTEN in HeLa cells with siRNAs containing mismatch base pairings and 2,4-difluorotoluyl ribonucleotide at position 9-10 and 9-12 of the sense strand, respectively, compared to parent duplex (AD-19044).

The present invention is further described with reference to the following general embodiments.

One aspect described herein provides siRNA and siNA compositions containing modified nucleotides or mismatched base pairings, particularly in the sense strand of a double stranded siNA, as well as methods for inhibiting the expression of a target gene in a cell, tissue or mammal using these compositions. Described herein are also compositions and methods for treating diseases in a mammal caused by the aberrant expression of a target gene, or a mutant form thereof, using siRNA compositions.

Described herein are nucleic acid-containing compositions that target specific mRNA sequences for removal by RISC. In particular are compositions containing double stranded iRNA agents that contain either a nucleobase modification (such as a universal base) or a mismatched base pairing. Without being limited by theory, it is believed that the presence of the nucleobase modifications or mismatches facilitates sense strand removal during RISC assembly, possibly through local destabilization of the duplex at the putative cleavage site.

In one aspect the, described herein is a double stranded iRNA agent with increased RNAi silencing activity comprising (a) an antisense strand which is complimentary to a target gene; (b) a sense strand which is complimentary to said antisense strand and comprises at least one modified nucleobase in the region corresponding to the target cleavage site region; and wherein said increased RNAi silencing activity is relative the corresponding unmodified RNAi agent as determined by comparing their respective IC₅₀ values measured either *in vitro* or *in vivo.*

In one embodiment, the modified nucleobase comprising nucleotide further comprises at least one modification chosen from a group of sugar modifications and backbone modifications described herein. In one embodiment, the nucleotide comprising the modified nucleobase is a 2'-deoxy nucleotide.

In one embodiment, the modified nucleobase is at the first position of the cleavage site region from the 5'-end of the sense strand.

In one embodiment, the modified nucleobase is at the second position of the cleavage site region from the 5'-end of the sense strand.

In one embodiment, the modified nucleobase is at the third position of the cleavage site region from the 5'-end of the sense strand.

In one embodiment, the modified nucleobase is at the fourth position of the cleavage site region from the 5'-end of the sense strand.

In another aspect, described herein is a double stranded iRNA agent with increased RNAi silencing activity comprising (a) an antisense strand which is complimentary to a target gene; (b) a sense strand which is complimentary to said antisense strand and comprises one or two or more mismatch base parings with the antisense strand in the region corresponding to the target cleavage site region; and wherein said increased RNAi silencing activity is relative the corresponding unmodified RNAi agent as determined by comparing their respective IC₅₀ values measured either *in vitro* or *in vivo.*

In one embodiment, the modified nucleobase comprising nucleotide further comprises at least one modification chosen from a group of sugar modification and backbone modification described herein. In one embodiment, the nucleotide comprising the modified nucleobase is a 2'-deoxy nucleotide.

In one embodiment, the mismatch is at the first position of the cleavage site region from the 5'-end of the sense strand.

In one embodiment, the mismatch is at the second position of the cleavage site region from the 5'-end of the sense strand.

In one embodiment, the mismatch is at the third position of the cleavage site region from the 5'-end of the sense strand.

In one embodiment, the mismatch is at the fourth position of the cleavage site region from the 5'-end of the sense strand.

The "target cleavage site" herein means the backbone linkage in the target gene or the sense strand that is cleaved by the RISC mechanism by utilizing the iRNA agent. And the target cleavage site region comprises at least one or at least two nucleotides on both side of the cleavage site. For the sense strand, the target cleavage site is the backbone linkage in the sense strand that would get cleaved if the sense strand itself was the target to be cleaved by the RNAi mechanism. The target cleavage site can be determined using methods known in the art, for example the 5'-RACE assay as detailed in Soutschek et al., Nature (2004) 432, 173-178. As is well understood in the art, the cleavage site region for a conical double stranded RNAi agent comprising two 21-nucleotides long strands (wherein the strands form a double stranded region of 19 consecutive basepairs having 2-nucleotide single stranded overhangs at the 3'-ends), the cleavage site region corresponds to positions 9-12 from the 5'-end of the sense strand.

In another aspect the, the invention provides a double stranded iRNA agent with increased RNAi silencing activity comprising (a) an antisense strand which is complimentary to a target gene; (b) a sense strand which is complimentary to said antisense strand and comprises at least one modification in the region corresponding to the target cleavage site region; and wherein said increased RNAi silencing activity is relative the corresponding unmodified RNAi agent as determined by comparing their respective IC₅₀ values measured either *in vitro* or *in vivo* and said modification locally destabilizes the duplex.

In one embodiment, the modified nucleobase comprising nucleotide further comprises at least one modification chosen from a group of sugar modification and backbone modification described herein. In one embodiment, the nucleotide comprising the modified nucleobase is a 2'-deoxy nucleotide.

In one embodiment, the local destabilization modification is at the first position of the cleavage site region from the 5'-end of the sense strand.

In one embodiment, the local destabilization modification is at the second position of the cleavage site region from the 5'-end of the sense strand.

In one embodiment, the local destabilization modification is at the third position of the cleavage site region from the 5'-end of the sense strand.

In one embodiment, the local destabilization modification is at the fourth position of the cleavage site region from the 5'-end of the sense strand.

### Definitions

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains.

The phrase "antisense strand" as used herein, refers to a polynucleotide that is substantially or 100% complementary to a target nucleic acid of interest. An antisense strand may comprise a polynucleotide that is RNA, DNA or chimeric RNA/DNA. For example, an antisense strand may be complementary, in whole or in part, to a molecule of messenger RNA, an RNA sequence that is not mRNA (e.g., microRNA, piwiRNA, tRNA, rRNA and hnRNA) or a sequence of DNA that is either coding or non-coding. The phrase "antisense strand" includes the antisense region of both polynucleotides that are formed from two separate strands, as well as unimolecular polynucleotides that are capable of forming hairpin structures. The terms "antisense strand" and "guide strand" are used interchangeably herein.

The phrase "sense strand" refers to a polynucleotide that has the same nucleotide sequence, in whole or in part, as a target nucleic acid such as a messenger RNA or a sequence of DNA. The sense strand is not incorporated into the functional riboprotein RISC. The terms "sense strand" and "passenger strand" are used interchangeably herein.

The term "duplex" includes a region of complementarity between two regions of two or more polynucleotides that comprise separate strands, such as a sense strand and an antisense strand, or between two regions of a single contiguous polynucleotide.

The term "complementary" refers to the ability of polynucleotides to form base pairs with one another. Base pairs are typically formed by hydrogen bonds between nucleotide units in antiparallel polynucleotide strands. Complementary polynucleotide strands can base pair in the Watson-Crick manner (*e.g.,* a to t, a to u, c to g), or in any other manner that allows for the formation of stable duplexes. "Perfect complementarity" or 100% complementarity refers to the situation in which each nucleotide unit of one polynucleotide strand can hydrogen bond with each nucleotide unit of a second polynucleotide strand. Less than perfect complementarity refers to the situation in which some, but not all, nucleotide units of two strands can hydrogen bond with each other. "Substantial complementarity" refers to polynucleotide strands exhibiting 90% or greater complementarity, excluding regions of the polynucleotide strands, such as overhangs, that are selected so as to be noncomplementary.

The phrase "from the 5'-end of the sense strand" includes the relative position of a given nucleotide in the sense strand with respect to the nucleotide of the sense strand that is located at the 5' most position of that strand.

The phrase "first 5' terminal nucleotide" includes first 5' terminal antisense nucleotides and first 5' terminal antisense nucleotides. "First 5' terminal antisense nucleotide" refers to the nucleotide of the antisense strand that is located at the 5' most position of that strand with respect to the bases of the antisense strand that have corresponding complementary bases on the sense strand. Thus, in a double stranded polynucleotide that is made of two separate strands, it refers to the 5' most base other than bases that are part of any 5' overhang on the antisense strand. When the first 5' terminal antisense nucleotide is part of a hairpin molecule, the term "terminal" refers to the 5' most relative position within the antisense region and thus is the 5" most nucleotide of the antisense region. The phrase "first 5" terminal sense nucleotide" is defined in reference to the antisense nucleotide. In molecules comprising two separate strands, it refers to the nucleotide of the sense strand that is located at the 5' most position of that strand with respect to the bases of the sense strand that have corresponding complementary bases on the antisense strand. Thus, in a double stranded polynucleotide that is made of two separate strands, it is the 5' most base other than bases that are part of any 5' overhang on the sense strand.

The term "nucleotide" includes a ribonucleotide or a deoxyribonucleotide or modified form thereof, as well as an analog thereof. Nucleotides include species that comprise purines, *e.g*., adenine, hypoxanthine, guanine, and their derivatives and analogs, as well as pyrimidines, *e.g.,* cytosine, uracil, thymine, and their derivatives and analogs.

The term "pseudouracil" or "5-uracil" refers to 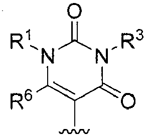 when R¹, R³ and R⁶ are hydrogen. Substituted pseudouracils are defined as follows: when R¹ is not hydrogen, it is a 1-substituted pseudouracil; when R³ is not hydrogen, it is a 3-substituted pseudouracil; and when R⁶ is not hydrogen, it is a 6-substituted uracil. The terms "2-(thio)pseudouracil", "4-(thio)pseudouracil" and "2,4-(dithio)pseudouracil" refer to 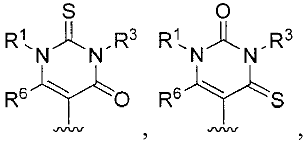 and 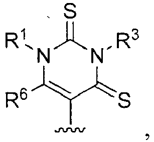 respectively. Suitable R¹, R³ and R⁶ include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, ureido or conjugate groups.

The phrases "2'-modification" and "2'-modified nucleotide" refer to a nucleotide unit having a sugar moiety, for example a ribosyl moiety, that is modified at the 2'-position such that the hydroxyl group (2'-OH) is replaced by, for example, -F, -H, -CH₃, -CH₂CH₃, -OCH₃, -OCH₂CH₃, -OCH₂CH₂OMe, -OCH₂C(=O)NHMe, -OCH₂-(4'-C) (a so-called "LNA sugar modifications"), or -OCH₂CH₂-(4'-C) (a so-called "ENA sugar modification"). For example, the phrases "2'-fluoro modification" and "2'-fluoro modified nucleotide" refer to a nucleotide unit having a sugar moiety, for example a ribosyl moiety, that is modified at the 2'-position such that the hydroxyl group (2'-OH) is replaced by a fluoro group (2'-F). U.S. Pat. Nos. 6,262,241, and 5,459,255, are drawn to, *inter alia,* methods of synthesizing 2'-fluoro modified nucleotides and oligonucleotides.

The phrase "phosphorothioate internucleotide linkage" refers to the replacement of a P=O group with a P=S group, and includes phosphorodithioate internucleoside linkages. One, some or all of the internucleotide linkages that are present in the oligonucleotide can be phosphorothioate internucleotide linkages. U.S. Pat. Nos. 6,143,881, 5,587,361 and 5,599,797, are drawn to, *inter alia,* oligonucleotides having phosphorothioate linkages.

The term "1,3-(diaza)-2-(oxo)-phenoxazin-1-yl" as used herein refers to 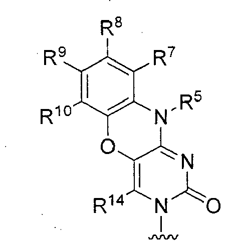 when R³, R⁷, R⁸, R⁹, R¹⁰, and R¹⁴ are hydrogen. Substituted 1,3-(diaza)-2-(oxo)-phenoxazin-1-yl are numbered as are the R groups (e.g., a 7-substituted 1,3-(diaza)-2-(oxo)-phenoxazin-1-yl is a compound wherein R⁷ is not hydrogen). The term "1-(aza)-2-(thio)-3-(aza)-phenoxazin-1-yl'' as used herein refers to 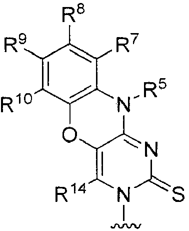 when R³, R⁷, , R⁸ R⁹, R¹⁰, and R¹⁴ are hydrogen. The term "1,3-(diaza)-2-(oxo)-phenthiazin-1-yl" as used herein refers to 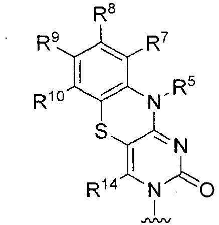 when R³, R⁷, R⁸, R⁹, R¹⁰, and R¹⁴ are hydrogen. The term "1-(aza)-2-(thio)-3-(aza)-phenthiazin-1-yl" as used herein refers to 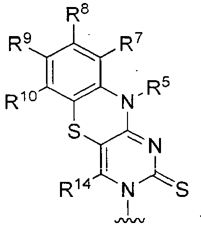 when R³, R⁷, R⁸, R⁹, R¹⁰, and R¹⁴ are hydrogen. Suitable R⁵, R⁷ to R¹⁰, and R¹⁴ include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, ureido or conjugate groups.

As used herein, "aminoalkylhydroxy" refers to -O-alkyl-amino (*e.g.,* -OCH₂CH₂NH₂). As used herein, "guanidiniumalkylhydroxy" refers to -O-alkyl-guanidinium (*e.g.,* -OCH₂CH₂N(H)C(=NH)NH₂).

As used herein, "aminocarbonylethylenyl" refers to 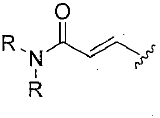 (*e.g*., when both R are hydrogen, 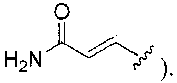 As used herein, "aminoalkylaminocarbonylethylenyl" refers to 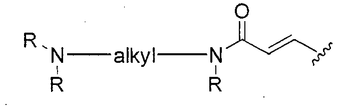 (e.g., when all three R are hydrogen, 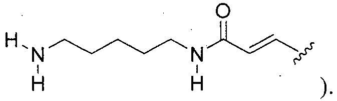 Suitable R include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, ureido or conjugate groups.

The term "1,3,5-(triaza)-2,6-(dioxa)-naphthalene" as used herein refers to 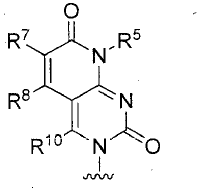 when R⁵, R⁷, R⁸ and R¹⁰ are hydrogen. Suitable R⁵, R⁷, R⁸ and R¹⁰ include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, ureido or conjugate groups.

The term "off-target" and the phrase "off-target effects" refer to any instance in which an siRNA or shRNA directed against a given target causes an unintended affect by interacting either directly or indirectly with another mRNA sequence, a DNA sequence or a cellular protein or other moiety. For example, an "off-target effect" may occur when there is a simultaneous degradation of other transcripts due to partial homology or complementarity between that other transcript and the sense and/or antisense strand of the siRNA or shRNA

The phrase "pharmaceutically acceptable carrier or diluent" includes compositions that facilitate the introduction of nucleic acid therapeutics such as siRNA, dsRNA, dsDNA, shRNA, microRNA, antimicroRNA, antagomir, antimir, antisense, aptamer or dsRNA/DNA hybrids into a cell and includes but is not limited to solvents or dispersants, coatings, anti-infective agents, isotonic agents, and agents that mediate absorption time or release of the inventive polynucleotides and double stranded polynucleotides. The phrase "pharmaceutically acceptable" includes approval by a regulatory agency of a government, for example, the U.S. federal government, a non-U.S. government, or a U.S. state government, or inclusion in a listing in the U.S. Pharmacopeia or any other generally recognized pharmacopeia for use in animals, including in humans.

The terms "silence" and "inhibit the expression of" and related terms and phrases, refer to the at least partial suppression of the expression of a gene targeted by an siRNA or siNA, as manifested by a reduction of the amount of mRNA transcribed from the target gene which may be isolated from a first cell or group of cells in which the target gene is transcribed and which has or have been treated such that the expression of the target gene is inhibited, as compared to a second cell or group of cells substantially identical to the first cell or group of cells but which has or have not been so treated (*i.e*., control cells).

The term "halo" refers to any radical of fluorine, chlorine, bromine or iodine. The term "alkyl" refers to saturated and unsaturated non-aromatic hydrocarbon chains that may be a straight chain or branched chain, containing the indicated number of carbon atoms (these include without limitation propyl, allyl, or propargyl), which may be optionally inserted with N, O, or S. For example, C₁-C₁₀ indicates that the group may have from 1 to 10 (inclusive) carbon atoms in it. The term "alkoxy" refers to an -O-alkyl radical. The term "alkylene" refers to a divalent alkyl (*i.e.,* -R-). The term "alkylenedioxo" refers to a divalent species of the structure -O-R-O-, in which R represents an alkylene. The term "aminoalkyl" refers to an alkyl substituted with an amino. The term "mercapto" refers to an -SH radical. The term "thioalkoxy" refers to an -S-alkyl radical.

The term "aryl" refers to a 6-carbon monocyclic or 10-carbon bicyclic aromatic ring system wherein 0, 1, 2, 3, or 4 atoms of each ring may be substituted by a substituent. Examples of aryl groups include phenyl, naphthyl and the like. The term "arylalkyl" or the term "aralkyl" refers to alkyl substituted with an aryl. The term "arylalkoxy" refers to an alkoxy substituted with aryl.

The term "cycloalkyl" as employed herein includes saturated and partially unsaturated cyclic hydrocarbon groups having 3 to 12 carbons, for example, 3 to 8 carbons, and, for example, 3 to 6 carbons, wherein the cycloalkyl group additionally may be optionally substituted. Cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (*e.g.,* carbon atoms and 1-3, 1-6, or 1-9 heteroatoms ofN, O, or S if monocyclic, bicyclic, or tricyclic, respectively), wherein 0, 1, 2, 3, or 4 atoms of each ring may be substituted by a substituent. Examples of heteroaryl groups include pyridyl, furyl or furanyl, imidazolyl, benzimidazolyl, pyrimidinyl, thiophenyl or thienyl, quinolinyl, indolyl, thiazolyl, and the like. The term "heteroarylalkyl" or the term "heteroaralkyl" refers to an alkyl substituted with a heteroaryl. The term "heteroarylalkoxy" refers to an alkoxy substituted with heteroaryl.

The term "heterocyclyl" refers to a nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (*e.g.,* carbon atoms and 1-3, 1-6, or 1-9 heteroatoms ofN, O, or S if monocyclic, bicyclic, or tricyclic, respectively), wherein 0, 1, 2 or 3 atoms of each ring may be substituted by a substituent. Examples of heterocyclyl groups include piperazinyl, pyrrolidinyl, dioxanyl, morpholinyl, tetrahydrofuranyl, and the like.

The term "oxo" refers to an oxygen atom, which forms a carbonyl when attached to carbon, an N-oxide when attached to nitrogen, and a sulfoxide or sulfone when attached to sulfur. The term "thio" refers to a sulfur atom, which forms a thiocarbonyl when attached to a carbon.

The term "acyl" refers to an alkylcarbonyl, cycloalkylcarbonyl, arylcarbonyl, heterocyclylcarbonyl, or heteroarylcarbonyl substituent, any of which may be further substituted by substituents.

The term "substituents" refers to a group "substituted" on an alkyl, cycloalkyl, aryl, heterocyclyl, or heteroaryl group at any atom of that group. Suitable substituents include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, ureido or conjugate groups.

In many cases, protecting groups are used during preparation of the compounds of the invention. As used herein, the term "protected" means that the indicated moiety has a protecting group appended thereon. In some certain embodiments described herein, compounds contain one or more protecting groups. A wide variety of protecting groups can be employed in the methods described herein. In general, protecting groups render chemical functionalities inert to specific reaction conditions, and can be appended to and removed from such functionalities in a molecule without substantially damaging the remainder of the molecule.

Representative hydroxyl protecting groups, for example, are disclosed by Beaucage et al. (Tetrahedron 1992, 48, 2223-2311). Further hydroxyl protecting groups, as well as other representative protecting groups, are disclosed in Greene and Wuts, Protective Groups in Organic Synthesis, Chapter 2, 2d ed., John Wiley & Sons, New York, 1991, and Oligonucleotides And Analogues A Practical Approach, Ekstein, F. Ed., IRL Press, N.Y, 1991.

Examples of hydroxyl protecting groups include, but are not limited to, t-butyl, t-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, 2,6-dichlorobenzyl, diphenylmethyl, p,p'-dinitrobenzhydryl, p-nitrobenzyl, triphenylmethyl, trimethylsilyl, triethylsilyl, t-butyldimethylsilyl, t-butyldiphenylsilyl, triphenylsilyl, benzoylformate, acetate, chloroacetate, trichloro acetate, trifluoroacetate, pivaloate, benzoate, p-phenylbenzoate, 9-fluorenylmethyl carbonate, mesylate and tosylate.

Amino-protecting groups stable to acid treatment are selectively removed with base treatment, and are used to make reactive amino groups selectively available for substitution. Examples of such groups are the Fmoc (E. Atherton and R. C. Sheppard in The Peptides, S. Udenfriend, J. Meienhofer, Eds., Academic Press, Orlando, 1987, volume 9, p.1) and various substituted sulfonylethyl carbamates exemplified by the Nsc group (Samukov et al., Tetrahedron Lett. 1994, 35, 7821; Verhart and Tesser, Rec. Trav. Chim. Pays-Bas 1987, 107, 621).

Additional amino-protecting groups include, but are not limited to, carbamate protecting groups, such as 2-trimethylsilylethoxycarbonyl (Teoc), 1-methyl-1-(4-biphenylyl)ethoxycarbonyl (Bpoc), t-butoxycarbonyl (BOC), allyloxycarbonyl (Alloc), 9-fluorenylmethyloxycarbonyl (Fmoc), and benzyloxycarbonyl (Cbz); amide protecting groups, such as formyl, acetyl, trihaloacetyl, benzoyl, and nitrophenylacetyl; sulfonamide protecting groups, such as 2-nitrobenzenesulfonyl; and imine and cyclic imide protecting groups, such as phthalimido and dithiasuccinoyl. Equivalents of these amino-protecting groups are also encompassed by the compounds and methods described herein.

### siRNA Compositions

Provided herein are siRNA compositions containing one or more short interfering ribonucleic acid (siRNA) molecules. These siRNAs can be single stranded or double stranded. Generally, each siRNA strand will be from about 10 in length (*e.g.,* 10, 11, 12, 13, 14, 15, 16, 17, 18, 19, 20, 21, 22, 23, 24 25 or more) to about 35 nucleotides in length (*e.g.,* 26, 27, 28, 29, 30, 31, 32, 33, 34, 35, 36, 37, 38, 39, 40 or more). Preferably, each strand is from about 19 to about 29 nucleotides in length.

Double stranded siRNA ("dsiRNA") compositions contain two single strands with at least substantial complementarity. For example, the first and second strands are each about 19 to about 29 nucleotides in length, and are capable of forming a duplex of between 17 and 25 base pairs. Regions of the strands, such as overhangs, are generally selected so as to be noncomplementary, and are not included in the formed duplex. siRNA compositions may contain one or two strands that have one or more terminal deoxythymidine (dT) nucleotide bases. Generally, these dT nucleotides are included in the overhang region and do not form or contribute to a duplex structure.

Described herein are single stranded RNA molecules bearing certain base modifications, such as the universal bases 2,4-difluorotoluyl or 5-nitroindole. Additional non-limiting base modifications are provided herein, such as in Examples 1 and 2. These modifications are generally in the central region of the sense strand (*e.g.,* region corresponding to the target cleavage site, e.g., nucleotides 9 to 12). Also described are single stranded RNA molecules bearing one or more mismatched nucleotides, such that binding of the single stranded RNA molecule to a second single stranded RNA molecule occurs at less than 100% of the nucleotides.

A "single strand siRNA compound" or a "single stranded siRNA compound" as used herein, is an siRNA compound which is made up of a single molecule. It may include a duplexed region, formed by intra-strand pairing, *e.g.,* it may be, or include, a hairpin or pan-handle structure. Single strand siRNA compounds may be antisense with regard to the target molecule. In certain embodiments single strand siRNA compounds are 5' phosphorylated or include a phosphoryl analog at the 5' prime terminus. 5'-phosphate modifications include those which are compatible with RISC mediated gene silencing. Suitable modifications include: 5'-monophosphate ((HO)₂(O)P-O-5'); 5'-diphosphate ((HO)₂(O)P-O-P(HO)(O)-O-5'); 5'-triphosphate ((HO)₂(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)₂(S)P-O-5'); 5'-monodithiophosphate (phosphorodithioate; (HO)(HS)(S)P-O-5'), 5'-phosphorothiolate ((HO)₂(O)P-S-5'); any additional combination of oxygen/sulfur replaced monophosphate, diphosphate and triphosphates (*e.g*., 5'-alpha-thiotriphosphate, 5'-gamma-thiotriphosphate, etc.), 5'-phosphoramidates ((HO)₂(O)P-NH-5', (HO)(NH₂)(O)P-O-5'), 5'-alkylphosphonates (R = alkyl = methyl, ethyl, isopropyl, propyl, etc., *e.g.,* RP(OH)(O)-O-5'-, (HO)₂(O)P-5'-CH₂-), 5'-alkyletherphosphonates (R = alkylether = methoxymethyl (MeOCH₂-), ethoxymethyl, etc., *e.g.,* RP(OH)(O)-O-5'-). (These modifications can also be used with the antisense strand of a double stranded iRNA.)

A single strand siRNA compound may be sufficiently long that it can enter the RISC and participate in RISC mediated cleavage of a target mRNA. A single strand siRNA compound is at least 14, and in other embodiments at least 15, 20, 25, 29, 35, 40, or 50 nucleotides in length. In certain embodiments, it is less than 200, 100, or 60 nucleotides in length.

Hairpin siRNA compounds will have a duplex region equal to or at least 17, 18, 19, 29, 21, 22, 23, 24, or 25 nucleotide pairs. The duplex region will may be equal to or less than 200, 100, or 50, in length. In certain embodiments, ranges for the duplex region are 15-30, 17 to 23, 19 to 23, and 19 to 21 nucleotides pairs in length. The hairpin may have a single strand overhang or terminal unpaired region, in some embodiments at the 3', and in certain embodiments on the antisense side of the hairpin. In some embodiments, the overhangs are 2-3 nucleotides in length.

A "double stranded siRNA compound" as used herein, is an siRNA compound which includes more than one, and in some cases two, strands in which interchain hybridization can form a region of duplex structure.

The antisense strand of a double stranded siRNA compound may be equal to or at least, 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to 21 nucleotides in length.

The sense strand of a double stranded siRNA compound may be equal to or at least 14, 15, 16 17, 18, 19, 25, 29, 40, or 60 nucleotides in length. It may be equal to or less than 200, 100, or 50, nucleotides in length. Ranges may be 17 to 25, 19 to 23, and 19 to 21 nucleotides in length.

The double strand portion of a double stranded siRNA compound may be equal to or at least, 14, 15, 16 17, 18, 19, 20, 21, 22, 23, 24, 25, 29, 40, or 60 nucleotide pairs in length. It may be equal to or less than 200, 100, or 50, nucleotides pairs in length. Ranges may be 15-30, 17 to 23, 19 to 23, and 19 to 21 nucleotides pairs in length.

In many embodiments, the ds siRNA compound is sufficiently large that it can be cleaved by an endogenous molecule, *e.g.,* by Dicer, to produce smaller ds siRNA compounds, *e.g.,* siRNAs agents

It may be desirable to modify one or both of the antisense and sense strands of a double strand siRNA compound. In some cases they will have the same modification or the same class of modification but in other cases the sense and antisense strand will have different modifications, *e.g.,* in some cases it is desirable to modify only the sense strand. It may be desirable to modify only the sense strand, *e.g*., to inactivate it, *e.g.,* the sense strand can be modified in order to inactivate the sense strand and prevent formation of an active siRNA/protein or RISC. This can be accomplished by a modification which prevents 5'-phosphorylation of the sense strand, *e.g.,* by modification with a 5'-O-methyl ribonucleotide (see Nykänen et al., (2001) ATP requirements and small interfering RNA structure in the RNA interference pathway. Cell 107, 309-321.) Other modifications which prevent phosphorylation can also be used, *e.g.,* simply substituting the 5'-OH by H rather than O-Me. Alternatively, a large bulky group may be added to the 5'-phosphate turning it into a phosphodiester linkage, though this may be less desirable as phosphodiesterases can cleave such a linkage and release a functional siRNA 5'-end. Antisense strand modifications include 5' phosphorylation as well as any of the other 5' modifications discussed herein, particularly the 5' modifications discussed above in the section on single stranded iRNA molecules.

The sense and antisense strands may be chosen such that the ds siRNA compound includes a single strand or unpaired region at one or both ends of the molecule. Thus, a ds siRNA compound may contain sense and antisense strands, paired to contain an overhang, *e.g.,* one or two 5' or 3' overhangs, or a 3' overhang of 2-3 nucleotides. Many embodiments will have a 3' overhang. Certain ssiRNA compounds will have single-stranded overhangs, in some embodiments 3' overhangs, of 1 or 2 or 3 nucleotides in length at each end. The overhangs can be the result of one strand being longer than the other, or the result of two strands of the same length being staggered. 5' ends may be phosphorylated.

In some embodiments, the length for the duplexed region is between 15 and 30, or 18, 19, 20, 21, 22, and 23 nucleotides in length, *e.g.,* in the ssiRNA compound range discussed above. ssiRNA compounds can resemble in length and structure the natural Dicer processed products from long dsiRNAs. Embodiments in which the two strands of the ssiRNA compound are linked, *e.g.,* covalently linked are also included. Hairpin, or other single strand structures which provide the required double stranded region, and a 3' overhang are also described herein.

The isolated siRNA compounds described herein, including ds siRNA compounds and ssiRNA compounds can mediate silencing of a target RNA, *e.g.,* mRNA, *e.g.,* a transcript of a gene that encodes a protein. For convenience, such mRNA is also referred to herein as mRNA to be silenced. Such a gene is also referred to as a target gene. In general, the RNA to be silenced is an endogenous gene or a pathogen gene. In addition, RNAs other than mRNA, *e.g.,* tRNAs, and viral RNAs, can also be targeted.

As used herein, the phrase "mediates RNAi" refers to the ability to silence, in a sequence specific manner, a target RNA. While not wishing to be bound by theory, it is believed that silencing uses the RNAi machinery or process and a guide RNA, *e.g.,* an ssiRNA compound of 21 to 23 nucleotides.

As used herein, "specifically hybridizable" and "complementary" are terms which are used to indicate a sufficient degree of complementarity such that stable and specific binding occurs between a compound described herein and a target RNA molecule. Specific binding requires a sufficient degree of complementarity to avoid non-specific binding of the oligomeric compound to non-target sequences under conditions in which specific binding is desired, *i.e.,* under physiological conditions in the case of *in vivo* assays or therapeutic treatment, or in the case of *in vitro* assays, under conditions in which the assays are performed. The non-target sequences typically differ by at least 5 nucleotides.

In one embodiment, an siRNA compound is "sufficiently complementary" to a target RNA, *e.g.,* a target mRNA, such that the siRNA compound silences production of protein encoded by the target mRNA. In another embodiment, the siRNA compound is "exactly complementary" to a target RNA, *e.g*., the target RNA and the siRNA compound anneal, for example to form a hybrid made exclusively of Watson-Crick base pairs in the region of exact complementarity. A "sufficiently complementary" target RNA can include an internal region (*e.g.,* of at least 10 nucleotides) that is exactly complementary to a target RNA. Moreover, in some embodiments, the siRNA compound specifically discriminates a single-nucleotide difference. In this case, the siRNA compound only mediates RNAi if exact complementary is found in the region (*e.g.,* within 7 nucleotides of) the single-nucleotide difference.

As used herein, the term "oligonucleotide" refers to a nucleic acid molecule (RNA or DNA) for example of length less than 100, 200, 300, or 400 nucleotides.

RNA agents discussed herein include unmodified RNA as well as RNA which have been modified, *e.g.,* to improve efficacy, and polymers of nucleoside surrogates. Unmodified RNA refers to a molecule in which the components of the nucleic acid, namely sugars, bases, and phosphate moieties, are the same or essentially the same as that which occur in nature, for example as occur naturally in the human body. The art has often referred to rare or unusual, but naturally occurring, RNAs as modified RNAs, see, *e.g.,* Limbach et al., (1994) Summary: the modified nucleosides of RNA, Nucleic Acids Res. 22: 2183-2196. Such rare or unusual RNAs, often termed modified RNAs (apparently because they are typically the result of a post transcriptionally modification) are within the term unmodified RNA, as used herein. Modified RNA refers to a molecule in which one or more of the components of the nucleic acid, namely sugars, bases, and phosphate moieties, are different from that which occur in nature, for example, different from that which occurs in the human body. While they are referred to as modified "RNAs," they will of course, because of the modification, include molecules which are not RNAs. Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, *e.g.,* non-charged mimics of the ribophosphate backbone. Examples of all of the above are discussed herein.

Much of the discussion below refers to single strand molecules. In many embodiments described herein, a double stranded siRNA compound, *e.g.,* a partially double stranded siRNA compound, is envisioned. Thus, it is understood that that double stranded structures (*e.g.,* where two separate molecules are contacted to form the double stranded region or where the double stranded region is formed by intramolecular pairing (*e.g.,* a hairpin structure)) made of the single stranded structures described below are described herein. Lengths are described elsewhere herein.

As nucleic acids are polymers of subunits, many of the modifications described below occur at a position which is repeated within a nucleic acid, *e.g.,* a modification of a base, or a phosphate moiety, or the a non-linking O of a phosphate moiety. In some cases the modification will occur at all of the subject positions in the nucleic acid but in many cases it will not. By way of example, a modification may only occur at a 3' or 5' terminal position, may only occur in a terminal regions, *e.g.,* at a position on a terminal nucleotide or in the last 2, 3, 4, 5, or 10 nucleotides of a strand. A modification may occur in a double strand region, a single strand region, or in both the first strand and the second strand. A modification may occur only in the double strand region of an RNA or may only occur in a single strand region of an RNA. *E.g.,* a phosphorothioate modification at a non-linking O position may only occur at one or both termini, may only occur in a terminal regions, *e.g.,* at a position on a terminal nucleotide or in the last 2, 3, 4, 5, or 10 nucleotides of a strand, or may occur in double strand and single strand regions, particularly at termini. The 5' end or ends can be phosphorylated.

In some embodiments it is possible, *e.g.,* to enhance stability, to include particular bases in overhangs, or to include modified nucleotides or nucleotide surrogates, in single strand overhangs, *e.g.,* in a 5' or 3' overhang, or in both the first strand and the second strand. *E.g.,* it can be desirable to include purine nucleotides in overhangs. In some embodiments all or some of the bases in a 3' or 5' overhang will be modified, *e.g.,* with a modification described herein. Modifications can include, *e.g.,* the use of modifications at the 2' OH group of the ribose sugar, *e.g.,* the use of deoxyribonucleotides, *e.g.,* deoxythymidine, instead of ribonucleotides, and modifications in the phosphate group, *e.g.,* phosphothioate modifications. Overhangs need not be homologous with the target sequence.

Modifications and nucleotide surrogates are discussed below, with reference to scaffold i shown below.

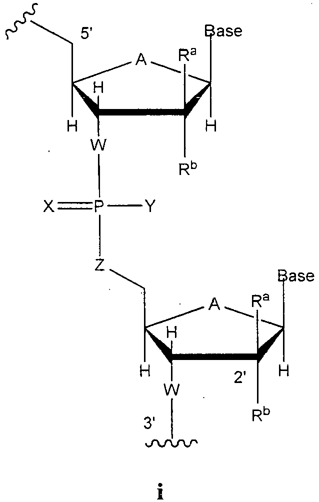

The scaffold i presented above represents a portion of a ribonucleic acid. The basic components are the ribose sugar, the base, the terminal phosphates, and phosphate internucleotide linkers. Where the bases are naturally occurring bases, *e.g.,* adenine, uracil, guanine or cytosine, the sugars are the unmodified 2' hydroxyl ribose sugar (A is O, R^{a} is H, and R^{b} is OH) and W, X, Y, and Z are all O, formula **i** represents a naturally occurring unmodified oligoribonucleotide.

Unmodified oligoribonucleotides may be less than optimal in some applications, *e.g.,* unmodified oligoribonucleotides can be prone to degradation by *e.g.,* cellular nucleases. Nucleases can hydrolyze nucleic acid phosphodiester bonds. However, chemical modifications to one or more of the above RNA components can confer improved properties, and, *e.g.,* can render oligoribonucleotides more stable to nucleases.

Modified nucleic acids and nucleotide surrogates can include one or more of:
(i) alteration, *e.g.,* replacement, of one or both of the non-linking (X and Y) phosphate oxygens and/or of one or more of the linking (W and Z) phosphate oxygens (When the phosphate is in the terminal position, one of the positions W or Z will not link the phosphate to an additional element in a naturally occurring ribonucleic acid. However, for simplicity of terminology, except where otherwise noted, the W position at the 5' end of a nucleic acid and the terminal Z position at the 3' end of a nucleic acid, are within the term "linking phosphate oxygens" as used herein);
(ii) alteration, *e.g.,* replacement, of a constituent of the ribose sugar, *e.g.,* of the 2' hydroxyl on the ribose sugar (i.e., R^{b} to -F and/or A to S);
(iii) wholesale replacement of the phosphate moiety with "dephospho" linkers;
(iv) modification or replacement of a naturally occurring base with a non-natural base;
(v) replacement or modification of the ribose-phosphate backbone;
(vi) modification of the 3' end or 5' end of the RNA, *e.g.,* removal, modification or replacement of a terminal phosphate group or conjugation of a moiety, *e.g.,* a fluorescently labeled moiety, to either the 3" or 5' end of RNA; and
(vii) modification of the sugar (e.g., six membered rings).

The terms replacement, modification, alteration, and the like, as used in this context, do not imply any process limitation, *e.g.,* modification does not mean that one must start with a reference or naturally occurring ribonucleic acid and modify it to produce a modified ribonucleic acid bur rather modified simply indicates a difference from a naturally occurring molecule.

It is understood that the actual electronic structure of some chemical entities cannot be adequately represented by only one canonical form (*i.e.,* Lewis structure). While not wishing to be bound by theory, the actual structure can instead be some hybrid or weighted average of two or more canonical forms, known collectively as resonance forms or structures. Resonance structures are not discrete chemical entities and exist only on paper. They differ from one another only in the placement or "localization" of the bonding and nonbonding electrons for a particular chemical entity. It can be possible for one resonance structure to contribute to a greater extent to the hybrid than the others. Thus, the written and graphical descriptions of the embodiments described herein are made in terms of what the art recognizes as the predominant resonance form for a particular species. For example, any phosphoroamidate (replacement of a nonlinking oxygen with nitrogen) would be represented by X = O and Y = N.

Specific modifications are discussed in more detail below.

### The Phosphate Group

The phosphate group is a negatively charged species. The charge is distributed equally over the two non-linking oxygen atoms (*i.e.,* X and Y in Formula 1 above). However, the phosphate group can be modified by replacing one of the oxygens with a different substituent. One result of this modification to RNA phosphate backbones can be increased resistance of the oligoribonucleotide to nucleolytic breakdown. Thus while not wishing to be bound by theory, it can be desirable in some embodiments to introduce alterations which result in either an uncharged linker or a charged linker with unsymmetrical charge distribution.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. Unlike the situation where only one of X or Y is altered, the phosphorus center in the phosphorodithioates is achiral which precludes the formation of oligoribonucleotides diastereomers. Diastereomer formation can result in a preparation in which the individual diastereomers exhibit varying resistance to nucleases. Further, the hybridization affinity of RNA containing chiral phosphate groups can be lower relative to the corresponding unmodified RNA species. Thus, while not wishing to be bound by theory, modifications to both X and Y which eliminate the chiral center, *e.g.,* phosphorodithioate formation, may be desirable in that they cannot produce diastereomer mixtures. Thus, X can be any one of S, Se, B, C, H, N, or OR (R is alkyl or aryl). Thus Y can be any one of S, Se, B, C, H, N, or OR (R is alkyl or aryl). Replacement of X and/or Y with sulfur is possible.

The phosphate linker can also be modified by replacement of a linking oxygen (*i.e.,* W or Z in Formula 1) with nitrogen (bridged phosphoroamidates), sulfur (bridged phosphorothioates) and carbon (bridged methylenephosphonates). The replacement can occur at a terminal oxygen (position W (3') or position Z (5'). Replacement of W with carbon or Z with nitrogen is possible.

Candidate agents can be evaluated for suitability as described below.

### The Sugar Group

A modified RNA can include modification of all or some of the sugar groups of the ribonucleic acid. For example, the 2' hydroxyl group (OH) can be modified or replaced with a number of different "oxy" or "deoxy" substituents. While not being bound by theory, enhanced stability is expected since the hydroxyl can no longer be deprotonated to form a 2' alkoxide ion. The 2' alkoxide can catalyze degradation by intramolecular nucleophilic attack on the linker phosphorus atom. Again, while not wishing to be bound by theory, it can be desirable to some embodiments to introduce alterations in which alkoxide formation at the 2' position is not possible.

Examples of "oxy"-2' hydroxyl group modifications include alkoxy or aryloxy (OR, *e.g.,* R = H, alkyl, cycloalkyl, aryl, aralkyl, heteroaryl or sugar); polyethyleneglycols (PEG), O(CH₂CH₂O)ₙCH₂CH₂OR; "locked" nucleic acids (LNA) in which the 2' hydroxyl is connected, *e.g.,* by a methylene bridge, to the 4' carbon of the same ribose sugar; O-amine (amine = NH₂; alkylamino, dialkylamino, heterocyclyl, arylamino, diaryl amino, heteroaryl amino, or diheteroaryl amino, ethylene diamine, polyamino) and aminoalkoxy, O(CH₂)ₙamine, (*e.g*., amine =NH₂; alkylamino, dialkylamino, heterocyclyl, arylamino, diaryl amino, heteroaryl amino, or diheteroaryl amino, ethylene diamine, polyamino). It is noteworthy that oligonucleotides containing only the methoxyethyl group (MOE), (OCH₂CH₂OCH₃, a PEG derivative), exhibit nuclease stabilities comparable to those modified with the robust phosphorothioate modification.

"Deoxy" modifications include hydrogen (*i.e.,* deoxyribose sugars, which are of particular relevance to the overhang portions of partially ds RNA); halo (*e.g*., fluoro); amino (*e.g.,* NH₂; alkylamino, dialkylamino, heterocyclyl, arylamino, diaryl amino, heteroalyl amino, diheteroaryl amino, or amino acid); NH(CH₂CH₂NH)ₙCH₂CH₂-amine (amine = NH₂; alkylamino, dialkylamino, heterocyclyl, arylamino, diaryl amino, heteroaryl amino, or diheteroaryl amino), NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino functionality. Other substituents of certain embodiments include 2'-methoxyethyl, 2'-OCH₃, 2'-O-allyl, 2'-C- allyl, and 2'-fluoro.

The sugar group can also contain one or more carbons that possess the opposite stereochemical configuration than that of the corresponding carbon in ribose. Thus, a modified RNA can include nucleotides containing *e.g.,* arabinose, as the sugar.

Modified RNA's can also include "abasic" sugars, which lack a nucleobase at C-1'. These abasic sugars can also be further contain modifications at one or more of the constituent sugar atoms.

To maximize nuclease resistance, the 2' modifications can be used in combination with one or more phosphate linker modifications (*e.g.,* phosphorothioate). The so-called "chimeric" oligonucleotides are those that contain two or more different modifications.

The natural sugar ring may also be expanded to a six-membered ring. In addition, the oxygen in the sugar may be replaced with a sulfur.

Candidate modifications can be evaluated as described below.

### Replacement of the Phosphate Group

The phosphate group can be replaced by non-phosphorus containing connectors. While not wishing to be bound by theory, it is believed that since the charged phosphodiester group is the reaction center in nucleolytic degradation, its replacement with neutral structural mimics should impart enhanced nuclease stability. Again, while not wishing to be bound by theory, it can be desirable, in some embodiment, to introduce alterations in which the charged phosphate group is replaced by a neutral moiety.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In certain embodiments, replacements may include the methylenecarbonylamino and methylenemethylimino groups.

Candidate modifications can be evaluated as described below.

### Replacement of Ribophosphate Backbone

Oligonucleotide- mimicking scaffolds can also be constructed wherein the phosphate linker and ribose sugar are replaced by nuclease resistant nucleoside or nucleotide surrogates. While not wishing to be bound by theory, it is believed that the absence of a repetitively charged backbone diminishes binding to proteins that recognize polyanions (*e.g.,* nucleases). Again, while not wishing to be bound by theory, it can be desirable in some embodiment, to introduce alterations in which the bases are tethered by a neutral surrogate backbone.

Examples include the mophilino, cyclobutyl, pyrrolidine and peptide nucleic acid (PNA) nucleoside surrogates. In certain embodiments, PNA surrogates may be used.

Candidate modifications can be evaluated as described below.

### Terminal Modifications

The 3' and 5' ends of an oligonucleotide can be modified. Such modifications can be at the 3' end, 5' end or both ends of the molecule. They can include modification or replacement of an entire terminal phosphate or of one or more of the atoms of the phosphate group. For example, the 3' and 5' ends of an oligonucleotide can be conjugated to other functional molecular entities such as labeling moieties, *e.g.,* fluorophores (*e.g.,* pyrene, TAMRA, fluorescein, Cy3 or Cy5 dyes) or protecting groups (based *e.g.,* on sulfur, silicon, boron or ester). The functional molecular entities can be attached to the sugar through a phosphate group and/or a spacer. The terminal atom of the spacer can connect to or replace the linking atom of the phosphate group or the C-3' or C-5' O, N, S or C group of the sugar. Alternatively, the spacer can connect to or replace the terminal atom of a nucleotide surrogate (*e.g.,* PNAs). These spacers or linkers can include *e.g.,* -(CH₂)ₙ-, -(CH₂)ₙN-, -(CH₂)ₙO-, -(CH₂)ₙS-, -O(CH₂CH₂O)ₙCH₂CH₂O- (*e.g.,* n = 3 or 6), abasic sugars, amide, carboxy, amine, oxyamine, oxyimine, thioether, disulfide, thiourea, sulfonamide, or morpholino, or biotin and fluorescein reagents. When a spacer/phosphate-functional molecular entity-spacer/phosphate array is interposed between two strands of siRNA compounds, this array can substitute for a hairpin RNA loop in a hairpin-type RNA agent. The 3' end can be an -OH group. While not wishing to be bound by theory, it is believed that conjugation of certain moieties can improve transport, hybridization, and specificity properties. Again, while not wishing to be bound by theory, it may be desirable to introduce terminal alterations that improve nuclease resistance. Other examples of terminal modifications include dyes, intercalating agents (*e.g.,* acridines), cross-linkers (*e.g.,* psoralene, mitomycin C), porphyrins (TPPC4, texaphyrin, Sapphyrin), polycyclic aromatic hydrocarbons (*e.g.,* phenazine, dihydrophenazine), artificial endonucleases (*e.g.,* EDTA), lipophilic carriers (*e.g*., cholesterol, cholic acid, adamantane acetic acid, 1-pyrene butyric acid, dihydrotestosterone, 1,3-bis-O(hexadecyl)glycerol, geranyloxyhexyl group, hexadecylglycerol, borneol, menthol, 1,3-propanediol, heptadecyl group, palmitic acid, myristic acid,03-(oleoyl)lithocholic acid, O3-(oleoyl)cholenic acid, dimethoxytrityl, or phenoxazin-1-yl)and peptide conjugates (*e.g*., antennapedia peptide, Tat peptide), alkylating agents, phosphate, amino, mercapto, PEG (*e.g*., PEG-40K), MPEG, [MPEG]₂, polyamino, alkyl, substituted alkyl, radiolabeled markers, enzymes, haptens (*e.g.,* biotin), transport/absorption facilitators (*e.g.,* aspirin, vitamin E, folic acid), synthetic ribonucleases (*e.g.,* imidazole, bisimidazole, histamine, imidazole clusters, acridine-imidazole conjugates, Eu³⁺ complexes of tetraazamacrocycles).

Terminal modifications can be added for a number of reasons, including as discussed elsewhere herein to modulate activity or to modulate resistance to degradation. Terminal modifications useful for modulating activity include modification of the 5' end with phosphate or phosphate analogs. For example, in certain embodiments siRNA compounds, especially antisense strands, are 5' phosphorylated or include a phosphoryl analog at the 5' prime terminus. 5'-phosphate modifications include those which are compatible with RISC mediated gene silencing. Suitable modifications include: 5'-monophosphate ((HO)₂(O)P-O-5'); 5'-diphosphate ((HO)₂(O)P-O-P(HO)(O)-O-5'); 5'-triphosphate ((HO)₂(O)P-O-(HO)(O)P-O-P(HO)(O)-O-5'); 5'-guanosine cap (7-methylated or non-methylated) (7m-G-O-5'-(HO)(O)P-O-(HO)(O)P-OP(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)₂(S)P-O-5'); 5'-monodithiophosphate (phosphorodithioate; (HO)(HS)(S)P-O-5'), 5'-phosphorothiolate ((HO)₂(O)P-S-5'); any additional combination of oxygen/sulfur replaced monophosphate, diphosphate and triphosphates (*e.g.,* 5'-alpha-thiotriphosphate, 5'-gamma-thiotriphosphate, etc.), 5'-phosphoramidates ((HO)₂(O)P-NH-5', (HO)(NH₂)(O)P-O-5'), 5'-alkylphosphonates (R=alkyl=methyl, ethyl, isopropyl, propyl, etc., *e.g.,* RP(OH)(O)-O-5'-, (OH)2(O)P-5'-CH₂-), 5'-alkyletherphosphonates (R=alkylether=methoxymethyl (MeOCH₂-), ethoxymethyl, etc., *e.g.,* RP(OH)(O)-O-5'-).

Terminal modifications can also be useful for monitoring distribution, and in such cases the groups to be added may include fluorophores, *e.g.,* fluorescein or an Alexa dye, *e.g*., Alexa 488. Terminal modifications can also be useful for enhancing uptake, useful modifications for this include cholesterol. Terminal modifications can also be useful for cross-linking an RNA agent to another moiety; modifications useful for this include mitomycin C.

Candidate modifications can be evaluated as described below.

### The Bases

Adenine, guanine, cytosine and uracil are the most common bases found in RNA. These bases can be modified or replaced to provide RNAs having improved properties. For example, nuclease resistant oligoribonucleotides can be prepared with these bases or with synthetic and natural nucleobases (*e.g.,* inosine, thymine, xanthine, hypoxanthine, nubularine, isoguanisine, or tubercidine) and any one of the above modifications. Alternatively, substituted or modified analogs of any of the above bases and "universal bases" can be employed. Examples include 2-(halo)adenine, 2-(alkyl)adenine, 2-(propyl)adenine, 2-(amino)adenine. 2-(aminoalkyl)adenine, 2-(aminopropyl)adenine, 2-(methylthio)-N⁶-(isopentenyl)adenine, 6-(alkyl)adenine, 6-(methyl)adenine, 7-(deaza)adenine, 8-(alkenyl)adenine, 8-(alkyl)adenine, 8-(alkynyl)adenine, 8-(amino)adenine, 8-(halo)adenine, 8-(hydroxyl)adenine, 8-(thioalkyl)adenine, 8-(thiol)adenine, N⁶-(isopentyl)adenine, N⁶-(methyl)adenine, N⁶, N⁶-(dimethyl)adenine, 2-(alkyl)guanine,2-(propyl)guanine, 6-(alkyl)guanine, 6-(methyl)guanine, 7-(alkyl)guanine, 7-(methyl)guanine, 7-(deaza)guanine, 8-(alkyl)guanine, 8-(alkenyl)guanine, 8-(alkynyl)guanine, 8-(amino)guanine, 8-(halo)guanine, 8-(hydroxyl)guanine, 8-(thioalkyl)guanine, 8-(thiol)guanine, N-(methyl)guanine, 2-(thio)cytosine, 3-(deaza)-5-(aza)cytosine, 3-(alkyl)cytosine, 3-(methyl)cytosine, 5-(alkyl)cytosine, 5-(alkynyl)cytosine, 5-(halo)cytosine, 5₋(methyl)cytosine, 5-(propynyl)cytosine, 5-(propynyl)cytosine, 5-(trifluoromethyl)cytosine, 6-(azo)cytosine, N⁴-(acetyl)cytosine, 3-(3-amino-3-carboxypropyl)uracil, 2-(thio)uracil,5-(methyl)-2-(thio)uracil, 5-(methylaminomethyl)-2-(thio)uracil, 4-(thio)uracil, 5-(methyl)-4-(thio)uracil, 5-(methylaminomethyl)-4-(thio)uracil, 5-(methyl)-2,4-(dithio)uracil, 5-(methylaminomethyl)-2,4-(dithio)uracil, 5-(2-aminopropyl)uracil, 5-(alkyl)uracil, 5-(alkynyl)uracil, 5-(allylamino)uracil, 5-(aminoallyl)uracil, 5-(aminoalkyl)uracil, 5-(guanidiniumalkyl)uracil, 5-(1,3-diazole-1-alkyl)uracil, 5-(cyanoalkyl)uracil, 5-(dialkylaminoalkyl)uracil, 5-(dimethylaminoalkyl)uracil, 5-(halo)uracil, 5-(methoxy)uracil, uracil-5-oxyacetic acid, 5-(methoxycarbonylmethyl)-2-(thio)uracil, 5-(methoxycarbonyl-methyl)uracil, 5-(propynyl)uracil, 5-(propynyl)uracil, 5-(trifluoromethyl)uracil, 6-(azo)uracil, dihydrouracil, N³-(methyl)uracil, 5-uracil (*i.e.,* pseudouracil), 2-(thio)pseudouracil,4-(thio)pseudouraci1,2,4-(dithio)psuedouracil,5-(alkyl)pseudouracil, 5-(methyl)pseudouracil, 5-(alkyl)-2-(thio)pseudouracil, 5-(methyl)-2-(thio)pseudouracil, 5-(alkyl)-4-(thio)pseudouracil, 5-(methyl)-4-(thio)pseudouracil, 5-(alkyl)-2,4-(dithio)pseudouracil, 5-(methyl)-2,4-(dithio)pseudouracil, 1-substituted pseudouracil, 1-substituted 2(thio)-pseudouracil, 1-substituted 4-(thio)pseudouracil, 1-substituted 2,4-(dithio)pseudouracil, 1-(aminocarbonylethylenyl)-pseudouracil, 1-(aminocarbonylethylenyl)-2(thio)-pseudouracil, 1-(aminocarbonylethylenyl)-4-(thio)pseudouracil, 1-(aminocarbonylethylenyl)-2,4-(dithio)pseudouracil, 1-(aminoalkylaminocarbonylethylenyl)-pseudouracil, 1-(aminoalkylamino-carbonylethylenyl)-2(thio)-pseudouracil, 1-(aminoalkylaminocarbonylethylenyl)-4-(thio)pseudouracil, 1-(aminoalkylaminocarbonylethylenyl)-2,4-(dithio)pseudouracil, 1,3-(diaza)-2-(oxo)-phenoxazin-1-yl, 1-(aza)-2-(thio)-3-(aza)-phenoxazin-1-yl, 1,3-(diaza)-2-(oxo)-phenthiazin-1-yl, 1-(aza)-2-(thio)-3-(aza)-phenthiazin-1-yl, 7-substituted 1,3-(diaza)-2-(oxo)-phenoxazin-1-yl, 7-substituted 1-(aza)-2-(thio)-3-(aza)-phenoxazin-1-yl, 7-substituted 1,3-(diaza)-2-(oxo)-phenthiazin-1-yl, 7-substituted 1-(aza)-2-(thio)-3-(aza)-phenthiazin-1-yl, 7-(aminoalkylhydroxy)-1,3-(diaza)-2-(oxo)-phenoxazin-1-yl, 7-(aminoalkylhydroxy)-1-(aza)-2-(thio)-3-(aza)-phenoxazin-1-yl, 7-(aminoalkylhydroxy)-1,3-(diaza)-2-(oxo)-phenthiazin-1-yl, 7-(aminoalkylhydroxy)-1-(aza)-2-(thio)-3-(aza)-phenthiazin-1-yl, 7-(guanidiniumalkylhydroxy)-1,3-(diaza)-2-(oxo)-phenoxazin-1-yl, 7-(guanidiniumalkylhydroxy)=1-(aza)-2-(thio)-3-(aza)-phenoxazin-1-yl, 7-(guanidiniumalkyl-hydroxy)-1,3-(diaza)-2-(oxo)-phenthiazin-1-yl, 7-(guanidiniumalkylhydroxy)-1-(aza)-2-(thio)-3-(aza)-phenthiazin-1-yl, 1,3,5-(triaza)-2,6-(dioxa)-naphthalene, inosine, xanthine, hypoxanthine, nubularine, tubercidine, isoguanisine, inosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, 3-(methyl)isocarbostyrilyl, 5-(methyl)isocarbostyrilyl, 3-(methyl)-7-(propynyl)isocarbostyrilyl, 7-(aza)indolyl, 6-(methyl)-7-(aza)indolyl, imidizopyridinyl, 9-(methyl)-imidizopyridinyl, pyrrolopyrizinyl, isocarbostyrilyl, 7-(propynyl)isocarbostyrilyl, propynyl-7-(aza)indolyl, 2,4,5-(trimethyl)phenyl, 4-(methyl)indolyl, 4,6-(dimethyl)indolyl, phenyl, napthalenyl, anthracenyl, phenanthracenyl; pyrenyl, stilbenyl, tetracenyl, pentacenyl, difluorotolyl, 4-(fluoro)-6-(methyl)benzimidazole, 4-(methyl)benzimidazole, 6-(azo)thymine, 2-pyridinone, 5-nitroindole, 3-nitropyrrole, 6-(aza)pyrimidine, 2-(amino)purine, 2,6-(diamino)purine, 5-substituted pyrimidines, N²-substituted purines, N⁶-substituted purines, O⁶-substituted purines, substituted 1,2,4-triazoles, or any O-alkylated or N-alkylated derivatives thereof;

Further purines and pyrimidines include those disclosed in U.S. Pat. No. 3,687,808, those disclosed in the Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613.

Generally, base changes are not used for promoting stability, but they can be useful for other reasons, *e.g.,* some, *e.g.,* 2,6-diaminopurine and 2 amino purine, are fluorescent. Modified bases can reduce target specificity. This may be taken into consideration in the design of siRNA compounds.

Candidate modifications can be evaluated as described below.

### Cationic Groups

Modifications can also include attachment of one or more cationic groups to the sugar, base, and/or the phosphorus atom of a phosphate or modified phosphate backbone moiety. A cationic group can be attached to any atom capable of substitution on a natural, unusual or universal base. A preferred position is one that does not interfere with hybridization, i.e., does not interfere with the hydrogen bonding interactions needed for base pairing. A cationic group can be attached e.g., through the C2' position of a sugar or analogous position in a cyclic or acyclic sugar surrogate. Cationic groups can include e.g., protonated amino groups, derived from e.g., O-AMINE (AMINE = NH₂; alkylamino, dialkylamino, heterocyclyl, arylamino, diaryl amino, heteroaryl amino, or diheteroaryl amino, ethylene diamine, polyamino); aminoalkoxy, e.g., O(CH₂)ₙAMINE, (*e.g.,* AMINE = NH₂; alkylamino, dialkylamino, heterocyclyl, arylamino, diaryl amino, heteroaryl amino, or diheteroaryl amino, ethylene diamine, polyamino); amino (*e.g.* NH₂; alkylamino, dialkylamino, heterocyclyl, arylamino, diaryl amino, heteroaryl amino, diheteroaryl amino, or amino acid); or NH(CH₂CH₂NH)ₙCH₂CH₂-AMINE (AMINE = NH₂; alkylamino, dialkylamino, heterocyclyl, arylamino, diaryl amino, heteroaryl amino, or diheteroaryl amino).

### Exemplary Modifications and Placement within an iRNA agent

Some modifications may preferably be included on an iRNA agent at a particular location, e.g., at an internal position of a strand, or on the 5' or 3' end of a strand of an iRNA agent. A preferred location of a modification on an iRNA agent, may confer preferred properties on the agent. For example, preferred locations of particular modifications may confer optimum gene silencing properties, or increased resistance to endonuclease or exonuclease activity. A modification described herein and below may be the sole modification, or the sole type of modification included on multiple ribonucleotides, or a modification can be combined with one or more other modifications described herein and below. For example, a modification on one strand of a multi-strand iRNA agent can be different than a modification on another strand of the multi-strand iRNA agent. Similarly, two different modifications on one strand can differ from a modification on a different strand of the iRNA agent. Other additional unique modifications, without limitation, can be incorporates into strands of the iRNA agent.

An iRNA agent may include a backbone modification to any nucleotide on an iRNA strand. For example, an iRNA agent may include a phosphorothioate linkage or P-alkyl modification in the linkages between one or more nucleotides of an iRNA agent. The nucleotides can be terminal nucleotides, e.g., nucleotides at the last position of a sense or antisense strand, or internal nucleotides.

An iRNA agent can include a sugar modification, e.g., a 2' or 3' sugar modification. Exemplary sugar modifications include, for example, a 2'-O-methylated nucleotide, a 2'-deoxy nucleotide, (e.g., a 2'-deoxyfluoro nucleotide), a 2'-O-methoxyethyl nucleotide, a 2'-O-NMA, a 2'-DMAEOE, a 2'-aminopropyl, 2'-hydroxy, or a 2'-ara-fluoro or a locked nucleic acid (LNA), extended nucleic acid (ENA), hexose nucleic acid (HNA), or cyclohexene nucleic acid (CeNA). A 2' modification is preferably 2'-OMe, and more preferably, 2'-deoxyfluoro. When the modification is 2'-OMe, the modification is preferably on the sense strands. When the modification is a 2'-fluoro, and the modification may be on any strand of the iRNA agent. A 2'-ara-fluoro modification will preferably be on the sense strands of the iRNA agent. An iRNA agent may include a 3' sugar modification, e.g., a 3'-OMe modification. Preferably a 3'-OMe modification is on the sense strand of the iRNA agent.

An iRNA agent may include a 5'-methyl-pyrimidine (e.g., a 5'-methyl-uridine modification or a 5'-methyl-cytodine) modification.
The modifications described herein can be combined onto a single iRNA agent. For example, an iRNA agent may have a phosphorothioate linkage and a 2' sugar modification; e.g., a 2'-OMe or 2'-F modification. In another example, an iRNA agent may include at least one 5-Me-pyrimidine and a 2'-sugar modification, e.g., a 2'-F or 2'-OMe modification.

An iRNA agent may include a nucleobase modification, such as a cationic modification, such as a 3-abasic cationic modification. The cationic modification can be e.g., an alkylamino-dT (e.g., a C6 amino-dT), an allylamino conjugate, a pyrrolidine conjugate, a pthalamido, a porphyrin, or a hydroxyprolinol conjugate, on one or more of the terminal nucleotides of the iRNA agent. When an alkylamino-dT conjugate is attached to the terminal nucleotide of an iRNA agent, the conjugate is preferably attached to the 3' end of the sense or antisense strand of an iRNA agent. When a pyrrolidine linker is attached to the terminal nucleotide of an iRNA agent, the linker is preferably attached to the 3'- or 5'-end of the sense strand, or the 3'-end of the antisense strand. When a pyrrolidine linker is attached to the terminal nucleotide of an iRNA agent, the linker is preferably on the 3'- or 5'-end of the sense strand, and not on the 5'-end of the antisense strand.

An iRNA agent may include at least one conjugate, such as a lipophile, a terpene, a protein binding agent, a vitamin, a carbohydrate, or a peptide. For example, the conjugate can be naproxen, nitroindole (or another conjugate that contributes to stacking interactions), folate, ibuprofen, or a C5 pyrimidine linker. The conjugate can also be a glyceride lipid conjugate (e.g., a dialkyl glyceride derivatives), vitamin E conjugate, or a thio-cholesterol. In generally, and except where noted to the contrary below, when a conjugate is on the terminal nucleotide of a sense or antisense strand, the conjugate is preferably on the 5' or 3' end of the sense strand or on the 5' end of the antisense strand, and preferably the conjugate is not on the 3' end of the antisense strand.

When the conjugate is naproxen, and the conjugate is on the terminal nucleotide of a sense or antisense strand, the conjugate is preferably on the 5' or 3' end of the sense or antisense strands. When the conjugate is cholesterol, and the conjugate is on the terminal nucleotide of a sense or antisense strand, the cholesterol conjugate is preferably on the 5' or 3' end of the sense strand and preferably not present on the antisense strand. Cholesterol may be conjugated to the iRNA agent by a pyrrolidine linker, serinol linker, hydroxyprolinol linker, or disulfide linkage. A dU-cholesterol conjugate may also be conjugated to the iRNA agent by a disulfide linkage. When the conjugate is cholanic acid, and the conjugate is on the terminal nucleotide of a sense or antisense strand, the cholanic acid is preferably attached to the 5' or 3' end of the sense strand, or the 3' end of the antisense strand. In one embodiment, the cholanic acid is attached to the 3' end of the sense strand and the 3' end of the antisense strand.

One or more nucleotides of an iRNA agent may have a 2'-5' linkage. Preferably, the 2'-5' linkage is on the sense strand. When the 2'-5' linkage is on the terminal nucleotide of an iRNA agent, the 2'-5' linkage occurs on the 5' end of the sense strand.
The iRNA agent may include an L-sugar, preferably on the sense strand, and not on the antisense strand.

The iRNA agent may include a methylphosphonate modification. When the methylphosphonate is on the terminal nucleotide of an iRNA agent, the methylphosphonate is at the 3' end of the sense or antisense strands of the iRNA agent.

An iRNA agent may be modified by replacing one or more ribonucleotides with deoxyribonucleotides. Preferably, adjacent deoxyribonucleotides are joined by phosphorothioate linkages, and the iRNA agent does not include more than four consecutive deoxyribonucleotides on the sense or the antisense strands.

An iRNA agent may include a difluorotoluyl (DFT) modification, e.g., 2,4-difluorotoluyl uracil, or a guanidine to inosine substitution.

The iRNA agent may include at least one 5'-uridine-adenine-3' (5'-UA-3') dinucleotide wherein the uridine is a 2'-modified nucleotide, or a terminal 5'-uridine-guanine-3' (5'-UG-3') dinucleotide, wherein the 5'-uridine is a 2'-modified nucleotide, or a terminal 5'-cytidine-adenine-3' (5'-CA-3') dinucleotide, wherein the 5'-cytidine is a 2'-modified nucleotide, or a terminal 5'-uridine-uridine-3' (5'-UU-3') dinucleotide, wherein the 5'-uridine is a 2'-modified nucleotide, or a terminal 5'-cytidine-cytidine-3' (5'-CC-3') dinucleotide, wherein the 5'-cytidine is a 2'-modified nucleotide, or a terminal 5'-cytidine-uridine-3' (5'-CU-3') dinucleotide, wherein the 5'-cytidine is a 2'-modified nucleotide, or a terminal 5'-uridine-cytidine-3' (5'-UC-3') dinucleotide, wherein the 5'-uridine is a 2'-modified nucleotide. The chemically modified nucleotide in the iRNA agent may be a 2'-O-methylated nucleotide. In some embodiments, the modified nucleotide can be a 2'-deoxy nucleotide, a 2'-deoxyfluoro nucleotide, a 2'-O-methoxyethyl nucleotide, a 2'-O-NMA, a 2'-DMAEOE, a 2'-aminopropyl, 2'-hydroxy, or a 2'-ara-fluoro, or a locked nucleic acid (LNA), extended nucleic acid (ENA), hexose nucleic acid (HNA), or cyclohexene nucleic acid (CeNA). The iRNA agents including these modifications are particularly stabilized against exonuclease activity, when the modified dinucleotide occurs on a terminal end of the sense or antisense strand of an iRNA agent, and are otherwise particularly stabilized against endonuclease activity.

An iRNA agent may have a single overhang, e.g., one end of the iRNA agent has a 3' or 5' overhang and the other end of the iRNA agent is a blunt end, or the iRNA agent may have a double overhang, e.g., both ends of the iRNA agent have a 3' or 5' overhang, such as a dinucleotide overhang. In another alternative, both ends of the iRNA agent may have blunt ends. The unpaired nucleotides may have at least one phosphorothioate dinucleotide linkage, and at least one of the unpaired nucleotides may be chemically modified in the 2'-position. The doublestrand region of the iRNA agent may include phosphorothioate dinucleotide linkages on one or both of the sense and antisense strands. Various strands of the multi-strand iRNA agent may be connected with a linker, e.g., a chemical linker such as hexaethylene glycol linker, a poly-(oxyphosphinico-oxy-1,3-propandiol) linker, an allyl linker, or a polyethylene glycol linker.

### Nuclease resistant monomers

An iRNA agent can include monomers which have been modified so as to inhibit degradation, e.g., by nucleases, e.g., endonucleases or exonucleases, found in the body of a subject. These monomers are referred to herein as NRMs, or nuclease resistance promoting monomers or modifications. In many cases these modifications will modulate other properties of the iRNA agent as well, e.g., the ability to interact with a protein, e.g., a transport protein, e.g., serum albumin, or a member of the RISC (RNA-induced Silencing Complex), or the ability of the first and second sequences to form a duplex with one another or to form a duplex with another sequence, e.g., a target molecule.

While not wishing to be bound by theory, it is believed that modifications of the sugar, base, and/or phosphate backbone in an iRNA agent can enhance endonuclease and exonuclease resistance, and can enhance interactions with transporter proteins and one or more of the functional components of the RISC complex. Preferred modifications are those that increase exonuclease and endonuclease resistance and thus prolong the half-life of the iRNA agent prior to interaction with the RISC complex, but at the same time do not render the iRNA agent resistant to endonuclease activity in the RISC complex. Again, while not wishing to be bound by any theory, it is believed that placement of the modifications at or near the 3' and/or 5' end of antisense strands can result in iRNA agents that meet the preferred nuclease resistance criteria delineated above. Again, still while not wishing to be bound by any theory, it is believed that placement of the modifications at e.g., the middle of a sense strand can result in iRNA agents that are relatively less likely to undergo off-targeting.

Modifications described herein can be incorporated into any RNA and RNA-like molecule described herein, e.g., an iRNA agent, a carrier oligonucleotide. An iRNA agent may include a duplex comprising a hybridized sense and antisense strand, in which the antisense strand and/or the sense strand may include one or more of the modifications described herein. The anti sense strand may include modifications at the 3' end and/or the 5' end and/or at one or more positions that occur 1-6 (e.g., 1-5, 1-4, 1-3, 1-2) nucleotides from either end of the strand. The sense strand may include modifications at the 3' end and/or the 5' end and/or at any one of the intervening positions between the two ends of the strand. The iRNA agent may also include a duplex comprising two hybridized antisense strands. The first and/or the second antisense strand may include one or more of the modifications described herein. Thus, one and/or both antisense strands may include modifications at the 3' end and/or the 5' end and/or at one or more positions that occur 1-6 (e.g., 1-5, 1-4, 1-3, 1-2) nucleotides from either end of the strand. Particular configurations are discussed below.

Modifications that can be useful for producing iRNA agents that meet the preferred nuclease resistance criteria delineated above can include one or more of the following chemical and/or stereochemical modifications of the sugar, base, and/or phosphate backbone:
(i) chiral (Sp) thioates. Thus, preferred NRMs include nucleotide dimers with an enriched or pure for a particular chiral form of a modified phosphate group containing a heteroatom at the nonbridging position, e.g., Sp or Rp, where this is the position normally occupied by the oxygen. The heteroatom can be S, Se, Nr₂, or Br3. When the heteroatom is S, enriched or chirally pure Sp linkage is preferred. Enriched means at least 70, 80, 90, 95, or 99% of the preferred form. Such NRMs are discussed in more detail below;
(ii) attachment of one or more cationic groups to the sugar, base, and/or the phosphorus atom of a phosphate or modified phosphate backbone moiety. Thus, preferred NRMs include monomers at the terminal position derivatized at a cationic group. As the 5' end of an antisense sequence should have a terminal -OH or phosphate group this NRM is preferably not used at the 5' end of an anti-sense sequence. The group should be attached at a position on the base which minimizes interference with H bond formation and hybridization, e.g., away from the face which interacts with the complementary base on the other strand, e.g, at the 5' position of a pyrimidine or a 7-position of a purine. These are discussed in more detail below;
(iii) nonphosphate linkages at the termini. Thus, preferred NRMs include Non-phosphate linkages, e.g., a linkage of 4 atoms which confers greater resistance to cleavage than does a phosphate bond. Examples include 3' CH2-NCH₃-O-CH2-5' and 3' CH2-NH-(O=)-CH2-5'.;
(iv) 3'-bridging thiophosphates and 5'-bridging thiophosphates. Thus, preferred NRM's can included these structures;
(v) L-RNA, 2'-5' linkages, inverted linkages, a-nucleosides. Thus, other preferred NRM's include: L nucleosides and dimeric nucleotides derived from L-nucleosides; 2'-5' phosphate, non-phosphate and modified phosphate linkages (e.g., thiophosphates, phosphoramidates and boronophosphates); dimers having inverted linkages, e.g., 3'-3' or 5'-5' linkages; monomers having an alpha linkage at the 1' site on the sugar, e.g., the structures described herein having an alpha linkage;
(vi) conjugate groups. Thus, preferred NRM's can include e.g., a targeting moiety or a conjugated ligand described herein conjugated with the monomer, e.g., through the sugar , base, or backbone;
(vii) abasic linkages. Thus, preferred NRM's can include an abasic monomer, e.g., an abasic monomer as described herein (e.g., a nucleobaseless monomer); an aromatic or heterocyclic or polyheterocyclic aromatic monomer as described herein.; and
(viii) 5'-phosphonates and 5'-phosphate prodrugs. Thus, preferred NRM's include monomers, preferably at the terminal position, e.g., the 5' position, in which one or more atoms of the phosphate group is derivatized with a protecting group, which protecting group or groups, are removed as a result of the action of a component in the subject's body, e.g, a carboxyesterase or an enzyme present in the subject's body. E.g., a phosphate prodrug in which a carboxy esterase cleaves the protected molecule resulting in the production of a thioate anion which attacks a carbon adjacent to the O of a phosphate and resulting in the production of an unprotected phosphate.

One or more different NRM modifications can be introduced into an iRNA agent or into a sequence of an iRNA agent. An NRM modification can be used more than once in a sequence or in an iRNA agent. As some NRM's interfere with hybridization the total number incorporated, should be such that acceptable levels of iRNA agent duplex formation are maintained.

In some embodiments NRM modifications are introduced into the terminal the cleavage site or in the cleavage region of a sequence (a sense strand or sequence) which does not target a desired sequence or gene in the subject. This can reduce off-target silencing.

### Evaluation of Candidate RNAs

One can evaluate a candidate RNA agent, *e.g.,* a modified RNA, for a selected property by exposing the agent or modified molecule and a control molecule to the appropriate conditions and evaluating for the presence of the selected property. For example, resistance to a degradent can be evaluated as follows. A candidate modified RNA (and a control molecule, usually the unmodified form) can be exposed to degradative conditions, *e.g.,* exposed to a milieu, which includes a degradative agent, *e.g.,* a nuclease. *E.g.,* one can use a biological sample, *e.g.,* one that is similar to a milieu, which might be encountered, in therapeutic use, *e.g.,* blood or a cellular fraction, *e.g.,* a cell-free homogenate or disrupted cells. The candidate and control could then be evaluated for resistance to degradation by any of a number of approaches. For example, the candidate and control could be labeled prior to exposure, with, *e.g.,* a radioactive or enzymatic label, or a fluorescent label, such as Cy3 or Cy5. Control and modified RNA's can be incubated with the degradative agent, and optionally a control, *e.g.,* an inactivated, *e.g.,* heat inactivated, degradative agent. A physical parameter, *e.g.,* size, of the modified and control molecules are then determined. They can be determined by a physical method, *e.g.,* by polyacrylamide gel electrophoresis or a sizing column, to assess whether the molecule has maintained its original length, or assessed functionally. Alternatively, Northern blot analysis can be used to assay the length of an unlabeled modified molecule.

A functional assay can also be used to evaluate the candidate agent. A functional assay can be applied initially or after an earlier non-functional assay, (*e.g.,* assay for resistance to degradation) to determine if the modification alters the ability of the molecule to silence gene expression. For example, a cell, *e.g.,* a mammalian cell, such as a mouse or human cell, can be co-transfected with a plasmid expressing a fluorescent protein, *e.g.,* GFP, and a candidate RNA agent homologous to the transcript encoding the fluorescent protein (see, *e.g.,* WO 00/44914). For example, a modified dsiRNA homologous to the GFP mRNA can be assayed for the ability to inhibit GFP expression by monitoring for a decrease in cell fluorescence, as compared to a control cell, in which the transfection did not include the candidate dsiRNA, *e.g.,* controls with no agent added and/or controls with a non-modified RNA added. Efficacy of the candidate agent on gene expression can be assessed by comparing cell fluorescence in the presence of the modified and unmodified dssiRNA compounds.

In an alternative functional assay, a candidate dssiRNA compound homologous to an endogenous mouse gene, for example, a maternally expressed gene, such as *c-mos,* can be injected into an immature mouse oocyte to assess the ability of the agent to inhibit gene expression *in vivo* (see, *e.g*., WO 01/36646). A phenotype of the oocyte, *e.g.,* the ability to maintain arrest in metaphase II, can be monitored as an indicator that the agent is inhibiting expression. For example, cleavage of *c-mos* mRNA by a dssiRNA compound would cause the oocyte to exit metaphase arrest and initiate parthenogenetic development (Colledge et al. Nature 370: 65-68, 1994; Hashimoto et al. Nature, 370:68-71, 1994). The effect of the modified agent on target RNA levels can be verified by Northern blot to assay for a decrease in the level of target mRNA, or by Western blot to assay for a decrease in the level of target protein, as compared to a negative control. Controls can include cells in which with no agent is added and/or cells in which a non-modified RNA is added.

### Ligands

A wide variety of entities, such as targeting moieties, endosomolytic agents and PK modulating entities, can be coupled to the iRNA agents at various places, for example at the 3'-end, 5'-end, both the 3'- and 5'-end, internally or a combination of them. Only one or both strands of an iRNA agent can comprise one or more ligand in addition to the modifications described herein. Preferred methods of conjugation, preferred monomers for conjugation and preferred ligands are described in copending United States Patent applications #10/916,185, filed August 10, 2004; #10/946,873, filed September 21, 2004; #10/985,426, filed November 9, 2004; #11/833,934, filed August 3, 2007; #11/115,989, filed April 27, 2005; #11/119,533, filed April 29, 2005 and #11/197,753, filed August 4, 2005. Further preferred ligands and ligand conjugated monomers are described in the United States provisional application #60/992,309 filed December 4, 2007 and #61/013,597 filed December 13, 2007.

### Physiological Effects

The siRNA compounds described herein can be designed such that determining therapeutic toxicity is made easier by the complementarity of the siRNA with both a human and a non-human animal sequence. By these methods, an siRNA can consist of a sequence that is fully complementary to a nucleic acid sequence from a human *and* a nucleic acid sequence from at least one non-human animal, *e.g.,* a non-human mammal, such as a rodent, ruminant or primate. For example, the non-human mammal can be a mouse, rat, dog, pig, goat, sheep, cow, monkey, Pan paniscus, Pan troglodytes, Macaca mulatto, or Cynomolgus monkey. The sequence of the siRNA compound could be complementary to sequences within homologous genes, *e.g.,* oncogenes or tumor suppressor genes, of the non-human mammal and the human. By determining the toxicity of the siRNA compound in the non-human mammal, one can extrapolate the toxicity of the siRNA compound in a human. For a more strenuous toxicity test, the siRNA can be complementary to a human and more than one, *e.g.,* two or three or more, non-human animals.

The methods described herein can be used to correlate any physiological effect of an siRNA compound on a human, *e.g.,* any unwanted effect, such as a toxic effect, or any positive, or desired effect.

### Increasing Cellular Uptake of siRNAs

Described herein are various siRNA compositions that contain covalently attached conjugates that increase cellular uptake and/or intracellular targeting of the siRNAs.

Additionally provided are methods described herein that include administering an siRNA compound and a drug that affects the uptake of the siRNA into the cell. The drug can be administered before, after, or at the same time that the siRNA compound is administered. The drug can be covalently or non-covalently linked to the siRNA compound. The drug can be, for example, a lipopolysaccharide, an activator of p38 MAP kinase, or an activator of NF-κB. The drug can have a transient effect on the cell. The drug can increase the uptake of the siRNA compound into the cell, for example, by disrupting the cell's cytoskeleton, *e.g.,* by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin. The drug can also increase the uptake of the siRNA compound into a given cell or tissue by activating an inflammatory response, for example. Exemplary drugs that would have such an effect include tumor necrosis factor alpha (TNFalpha), interleukin-1 beta, a CpG motif, gamma interferon or more generally an agent that activates a toll-like receptor.

### siRNA Production

An siRNA can be produced, *e.g.,* in bulk, by a variety of methods. Exemplary methods include: organic synthesis and RNA cleavage, *e.g., in vitro* cleavage.

*Organic Synthesis.* An siRNA can be made by separately synthesizing a single stranded RNA molecule, or each respective strand of a double-stranded RNA molecule, after which the component strands can then be annealed.

A large bioreactor, *e.g.,* the OligoPilot II from Pharmacia Biotec AB (Uppsala Sweden), can be used to produce a large amount of a particular RNA strand for a given siRNA. The OligoPilotII reactor can efficiently couple a nucleotide using only a 1.5 molar excess of a phosphoramidite nucleotide. To make an RNA strand, ribonucleotides amidites are used. Standard cycles of monomer addition can be used to synthesize the 21 to 23 nucleotide strand for the siRNA. Typically, the two complementary strands are produced separately and then annealed, *e.g.,* after release from the solid support and deprotection.

Organic synthesis can be used to produce a discrete siRNA species. The complementary of the species to a particular target gene can be precisely specified. For example, the species may be complementary to a region that includes a polymorphism, *e.g.,* a single nucleotide polymorphism. Further the location of the polymorphism can be precisely defined. In some embodiments, the polymorphism is located in an internal region, *e.g*., at least 4, 5, 7, or 9 nucleotides from one or both of the termini.

*dsiRNA Cleavage.* siRNAs can also be made by cleaving a larger siRNA. The cleavage can be mediated *in vitro* or *in vivo.* For example, to produce iRNAs by cleavage *in vitro,* the following method can be used:

*In vitro* transcription. dsiRNA is produced by transcribing a nucleic acid (DNA) segment in both directions. For example, the HiScribe™ RNAi transcription kit (New England Biolabs) provides a vector and a method for producing a dsiRNA for a nucleic acid segment that is cloned into the vector at a position flanked on either side by a T7 promoter. Separate templates are generated for T7 transcription of the two complementary strands for the dsiRNA. The templates are transcribed *in vitro* by addition of T7 RNA polymerase and dsiRNA is produced. Similar methods using PCR and/or other RNA polymerases (*e.g.,* T3 or SP6 polymerase) can also be dotoxins that may contaminate preparations of the recombinant enzymes.

*In Vitro Cleavage.* In one embodiment, RNA generated by this method is carefully purified to remove endsiRNA is cleaved *in vitro* into siRNAs, for example, using a Dicer or comparable RNAse III-based activity. For example, the dsiRNA can be incubated in an *in vitro* extract from Drosophila or using purified components, *e.g.,* a purified RNAse or RISC complex (RNA-induced silencing complex). See, *e.g.,* Ketting et al. Genes Dev 2001 Oct 15;15(20):2654-9. and Hammond Science 2001 Aug 10;293(5532):1146-50.

dsiRNA cleavage generally produces a plurality of siRNA species, each being a particular 21 to 23 nt fragment of a source dsiRNA molecule. For example, siRNAs that include sequences complementary to overlapping regions and adjacent regions of a source dsiRNA molecule may be present.

Regardless of the method of synthesis, the siRNA preparation can be prepared in a solution (*e.g.,* an aqueous and/or organic solution) that is appropriate for formulation. For example, the siRNA preparation can be precipitated and redissolved in pure double-distilled water, and lyophilized. The dried siRNA can then be resuspended in a solution appropriate for the intended formulation process.

### Formulations

The siRNA compounds described herein can be formulated for administration to a subject It is understood that these formulations, compositions and methods can be practiced with modified siRNA compounds, and such practice is described herein.

A formulated siRNA composition can assume a variety of states. In some examples, the composition is at least partially crystalline, uniformly crystalline, and/or anhydrous (*e.g.,* less than 80, 50, 30, 20, or 10% water). In another example, the siRNA is in an aqueous phase, *e.g.,* in a solution that includes water.

The aqueous phase or the crystalline compositions can, *e.g.,* be incorporated into a delivery vehicle, *e.g.,* a liposome (particularly for the aqueous phase) or a particle (*e.g.,* a microparticle as can be appropriate for a crystalline composition). Generally, the siRNA composition is formulated in a manner that is compatible with the intended method of administration, as described herein. For example, in particular embodiments the composition is prepared by at least one of the following methods: spray drying, lyophilization, vacuum drying, evaporation, fluid bed drying, or a combination of these techniques; or sonication with a lipid, freeze-drying, condensation and other self-assembly.

A siRNA preparation can be formulated in combination with another agent_{,} *e.g*., another therapeutic agent or an agent that stabilizes an siRNA, *e.g.,* a protein that complexes with siRNA to form an iRNP. Still other agents include chelators, *e.g.,* EDTA *(e.g.,* to remove divalent cations such as Mg²⁺), salts, RNAse inhibitors (*e.g.,* a broad specificity RNAse inhibitor such as RNAsin) and so forth.

In one embodiment, the siRNA preparation includes another siNA compound, *e.g.,* a second siRNA that can mediate RNAi with respect to a second gene, or with respect to the same gene. Still other preparation can include at least 3,5, ten, twenty, fifty, or a hundred or more different siRNA species. Such siRNAs can mediate RNAi with respect to a similar number of different genes.

In one embodiment, the siRNA preparation includes at least a second therapeutic agent (*e.g*., an agent other than an RNA or a DNA). For example, an siRNA composition for the treatment of a viral disease, *e.g.,* HIV, might include a known antiviral agent (*e.g.,* a protease inhibitor or reverse transcriptase inhibitor). In another example, an siRNA composition for the treatment of a cancer might further comprise a chemotherapeutic agent.

Exemplary formulations are discussed below.

*Liposomes.* For ease of exposition the formulations, compositions and methods in this section are discussed largely with regard to unmodified siRNA compounds. It may be understood, however, that these formulations, compositions and methods can be practiced with other siRNA compounds, *e.g.,* modified siRNAs, and such practice is described herein. An siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) preparation can be formulated for delivery in a membranous molecular assembly, *e.g.,* a liposome or a micelle. As used herein, the term "liposome" refers to a vesicle composed of amphiphilic lipids arranged in at least one bilayer, *e.g.,* one bilayer or a plurality of bilayers. Liposomes include unilamellar and multilamellar vesicles that have a membrane formed from a lipophilic material and an aqueous interior. The aqueous portion contains the siRNA composition. The lipophilic material isolates the aqueous interior from an aqueous exterior, which typically does not include the siRNA composition, although in some examples, it may. Liposomes are useful for the transfer and delivery of active ingredients to the site of action. Because the liposomal membrane is structurally similar to biological membranes, when liposomes are applied to a tissue, the liposomal bilayer fuses with bilayer of the cellular membranes. As the merging of the liposome and cell progresses, the internal aqueous contents that include the siRNA are delivered into the cell where the siRNA can specifically bind to a target RNA and can mediate RNAi. In some cases the liposomes are also specifically targeted, *e.g.,* to direct the siRNA to particular cell types.

A liposome containing an siRNA can be prepared by a variety of methods. In one example, the lipid component of a liposome is dissolved in a detergent so that micelles are formed with the lipid component. For example, the lipid component can be an amphipathic cationic lipid or lipid conjugate. The detergent can have a high critical micelle concentration and may be nonionic. Exemplary detergents include cholate, CHAPS, octylglucoside, deoxycholate, and lauroyl sarcosine. The siRNA preparation is then added to the micelles that include the lipid component. The cationic groups on the lipid interact with the siRNA and condense around the siRNA to form a liposome. After condensation, the detergent is removed, *e.g.,* by dialysis, to yield a liposomal preparation of siRNA.

If necessary a carrier compound that assists in condensation can be added during the condensation reaction, *e.g*., by controlled addition. For example, the carrier compound can be a polymer other than a nucleic acid (*e.g.,* spermine or spermidine). pH can also adjusted to favor condensation.

Further description of methods for producing stable polynucleotide delivery vehicles, which incorporate a polynucleotide/cationic lipid complex as structural components of the delivery vehicle, are described in, *e.g.,* WO 96/37194. Liposome formation can also include one or more aspects of exemplary methods described in Felgner, P. L. et al., Proc. Natl. Acad. Sci., USA 8:7413-7417, 1987; U.S. Pat. No. 4,897,355; U.S. Pat. No. 5,171,678; Bangham, et al. M. Mol. Biol. 23:238, 1965; Olson, et al. Biochim. Biophys. Acta 557:9, 1979; Szoka, et al. Proc. Natl. Acad. Sci. 75: 4194, 1978; Mayhew, et al. Biochim. Biophys. Acta 775:169, 1984; Kim, et al. Biochim. Biophys. Acta 728:339, 1983; and Fukunaga, et al. Endocrinol. 115:757, 1984. Commonly used techniques for preparing lipid aggregates of appropriate size for use as delivery vehicles include sonication and freeze-thaw plus extrusion (see, *e.g.,* Mayer, et al. Biochim. Biophys. Acta 858:161, 1986). Microfluidization can be used when consistently small (50 to 200 nm) and relatively uniform aggregates are desired (Mayhew, et al. Biochim. Biophys. Acta 775:169, 1984). These methods are readily adapted to packaging siRNA preparations into liposomes.

Liposomes that are pH-sensitive or negatively-charged entrap nucleic acid molecules rather than complex with them. Since both the nucleic acid molecules and the lipid are similarly charged, repulsion rather than complex formation occurs. Nevertheless, some nucleic acid molecules are entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 19, (1992) 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Examples of other methods to introduce liposomes into cells *in vitro* and *in vivo* include U.S. Pat. No. 5,283,185; U.S. Pat. No. 5,171,678; WO 94/00569; WO 93/24640; WO 91/16024; Felgner, J. Biol. Chem. 269:2550, 1994; Nabel, Proc. Natl. Acad. Sci. 90:11307, 1993; Nabel, Human Gene Ther. 3:649, 1992; Gershon, Biochem. 32:7143, 1993; and Strauss EMBO J. 11:417, 1992.

In one embodiment, cationic liposomes are used. Cationic liposomes possess the advantage of being able to fuse to the cell membrane. Non-cationic liposomes, although not able to fuse as efficiently with the plasma membrane, are taken up by macrophages *in vivo* and can be used to deliver siRNAs to macrophages.

Further advantages of liposomes include: liposomes obtained from natural phospholipids are biocompatible and biodegradable; liposomes can incorporate a wide range of water and lipid soluble drugs; liposomes can protect encapsulated siRNAs in their internal compartments from metabolism and degradation (Rosoff, in "Pharmaceutical Dosage Forms," Lieberman, Rieger and Banker (Eds.), 1988, volume 1, p. 245). Important considerations in the preparation of liposome formulations are the lipid surface charge, vesicle size and the aqueous volume of the liposomes.

A positively charged synthetic cationic lipid, N-[1-(2,3-dioleyloxy)propyl]-N,N,N-trimethylammonium chloride (DOTMA) can be used to form small liposomes that interact spontaneously with nucleic acid to form lipid-nucleic acid complexes which are capable of fusing with the negatively charged lipids of the cell membranes of tissue culture cells, resulting in delivery of siRNA (see, *e.g.,* Felgner, P. L. et al., Proc. Natl. Acad. Sci., USA 8:7413-7417, 1987 and U.S. Pat. No. 4,897,355 for a description of DOTMA and its use with DNA).

A DOTMA analogue, 1,2-bis(oleoyloxy)-3-(trimethylammonia)propane (DOTAP) can be used in combination with a phospholipid to form DNA-complexing vesicles. Lipofectin™ Bethesda Research Laboratories, Gaithersburg, Md.) is an effective agent for the delivery of highly anionic nucleic acids into living tissue culture cells that comprise positively charged DOTMA liposomes which interact spontaneously with negatively charged polynucleotides to form complexes. When enough positively charged liposomes are used, the net charge on the resulting complexes is also positive. Positively charged complexes prepared in this way spontaneously attach to negatively charged cell surfaces, fuse with the plasma membrane, and efficiently deliver functional nucleic acids into, for example, tissue culture cells. Another commercially available cationic lipid, 1,2-bis(oleoyloxy)-3,3-(trimethylammonia)propane ("DOTAP") (Boehringer Mannheim, Indianapolis, Indiana) differs from DOTMA in that the oleoyl moieties are linked by ester, rather than ether linkages.

Other reported cationic lipid compounds include those that have been conjugated to a variety of moieties including, for example, carboxyspermine which has been conjugated to one of two types of lipids and includes compounds such as 5-carboxyspermylglycine dioctaoleoylamide ("DOGS") (Transfectam™, Promega, Madison, Wisconsin) and dipalmitoylphosphatidylethanolamine 5-carboxyspermyl-amide ("DPPES") (see, *e.g.,* U.S. Pat. No. 5,171,678).

Another cationic lipid conjugate includes derivatization of the lipid with cholesterol ("DC-Chol") which has been formulated into liposomes in combination with DOPE (See, Gao, X. and Huang, L., Biochim. Biophys. Res. Commun. 179:280, 1991). Lipopolylysine, made by conjugating polylysine to DOPE, has been reported to be effective for transfection in the presence of serum (Zhou, X. et al., Biochim. Biophys. Acta 1065:8, 1991). For certain cell lines, these liposomes containing conjugated cationic lipids, are said to exhibit lower toxicity and provide more efficient transfection than the DOTMA-containing compositions. Other commercially available cationic lipid products include DMRIE and DMRIE-HP (Vical, La Jolla, California) and Lipofectamine (DOSPA) (Life Technology, Inc., Gaithersburg, Maryland). Other cationic lipids suitable for the delivery of oligonucleotides are described in WO 98/39359 and WO 96/37194.

Liposomal formulations are particularly suited for topical administration, liposomes present several advantages over other formulations. Such advantages include reduced side effects related to high systemic absorption of the administered drug, increased accumulation of the administered drug at the desired target, and the ability to administer siRNA, into the skin. In some implementations, liposomes are used for delivering siRNA to epidermal cells and also to enhance the penetration of siRNA into dermal tissues, *e.g.,* into skin. For example, the liposomes can be applied topically. Topical delivery of drugs formulated as liposomes to the skin has been documented (see, *e.g*., Weiner et al., Journal of Drug Targeting, 1992, vol. 2,405-410 and du Plessis et al., Antiviral Research, 18, 1992, 259-265; Mannino, R. J. and Fould-Fogerite, S., Biotechniques 6:682-690, 1988; Itani, T. et al. Gene 56:267-276. 1987; Nicolau, C. et al. Meth. Enz. 149:157-176, 1987; Straubinger, R. M. and Papahadjopoulos, D. Meth. Enz. 101:512-527, 1983; Wang, C. Y. and Huang, L., Proc. Natl. Acad. Sci. USA 84:7851-7855, 1987).

Non-ionic liposomal systems have also been examined to determine their utility in the delivery of drugs to the skin, in particular systems comprising non-ionic surfactant and cholesterol. Non-ionic liposomal formulations comprising Novasome I (glyceryl dilaurate/cholesterol/polyoxyethylene-10-stearyl ether) and Novasome II (glyceryl distearate/ cholesterol/polyoxyethylene-10-stearyl ether) were used to deliver a drug into the dermis of mouse skin. Such formulations with siRNA are useful for treating a dermatological disorder.

Liposomes that include siRNA can be made highly deformable. Such deformability can enable the liposomes to penetrate through pore that are smaller than the average radius of the liposome. For example, transfersomes are a type of deformable liposomes. Transferosomes can be made by adding surface edge activators, usually surfactants, to a standard liposomal composition. Transfersomes that include siRNA can be delivered, for example, subcutaneously by infection in order to deliver siRNA to keratinocytes in the skin. In order to cross intact mammalian skin, lipid vesicles must pass through a series of fine pores, each with a diameter less than 50 nm, under the influence of a suitable transdermal gradient. In addition, due to the lipid properties, these transferosomes can be self-optimizing (adaptive to the shape of pores, *e.g.,* in the skin), self-repairing, and can frequently reach their targets without fragmenting, and often self-loading.

Other formulations are described in United States provisional application serial nos. 61/018,616, filed January 2, 2008; 6-1/018,611, filed January 2, 2008; 61/039,748, filed March 26, 2008; 61/047,087, filed April 22, 2008 and 61/051,528, filed May 8, 2008. PCT application no PCT/US2007/080331, filed October 3, 2007 also describes respective formulations.

*Surfactants.* For ease of exposition the formulations, compositions and methods in this section are discussed largely with regard to unmodified siRNA compounds. It may be understood, however, that these formulations, compositions and methods can be practiced with other siRNA compounds, *e.g.,* modified siRNA compounds, and such practice is described herein. Surfactants find wide application in formulations such as emulsions (including microemulsions) and liposomes (see above). siRNA (or a precursor, *e.g.,* a larger dsiRNA which can be processed into a siRNA, or a DNA which encodes a siRNA or precursor) compositions can include a surfactant. In one embodiment, the siRNA is formulated as an emulsion that includes a surfactant. The most common way of classifying and ranking the properties of the many different types of surfactants, both natural and synthetic, is by the use of the hydrophile/lipophile balance (HLB). The nature of the hydrophilic group provides the most useful means for categorizing the different surfactants used in formulations (Rieger, in "Pharmaceutical Dosage Forms," Marcel Dekker, Inc., New York, NY, 1988, p. 285).

If the surfactant molecule is not ionized, it is classified as a nonionic surfactant. Nonionic surfactants find wide application in pharmaceutical products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in "Pharmaceutical Dosage Forms," Marcel Dekker, Inc., New York, NY, 1988, p. 285).

*Micelles and other Membranous Formulations.* For ease of exposition the micelles and other formulations, compositions and methods in this section are discussed largely with regard to unmodified siRNA compounds. It may be understood, however, that these micelles and other formulations, compositions and methods can be practiced with other siRNA compounds, *e.g.,* modified siRNA compounds, and such practice is described herein. The siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g*., a double-stranded siRNA compound, or ssiRNA compound, or precursor thereof)) composition can be provided as a micellar formulation. "Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic.

A mixed micellar formulation suitable for delivery through transdermal membranes may be prepared by mixing an aqueous solution of the siRNA composition, an alkali metal C₈ to C₂₂ alkyl sulphate, and a micelle forming compounds. Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerin, polyglycerin, lysine, polylysine, triolein, polyoxyethylene ethers and analogues thereof, polidocanol alkyl ethers and analogues thereof, chenodeoxycholate, deoxycholate, and mixtures thereof. The micelle forming compounds may be added at the same time or after addition of the alkali metal alkyl sulphate. Mixed micelles will form with substantially any kind of mixing of the ingredients but vigorous mixing in order to provide smaller size micelles.

In one method a first micellar composition is prepared which contains the siRNA composition and at least the alkali metal alkyl sulphate. The first micellar composition is then mixed with at least three micelle forming compounds to form a mixed micellar composition. In another method, the micellar composition is prepared by mixing the siRNA composition, the alkali metal alkyl sulphate and at least one of the micelle forming compounds, followed by addition of the remaining micelle forming compounds, with vigorous mixing.

Phenol and/or m-cresol may be added to the mixed micellar composition to stabilize the formulation and protect against bacterial growth. Alternatively, phenol and/or m-cresol may be added with the micelle forming ingredients. An isotonic agent such as glycerin may also be added after formation of the mixed micellar composition.

For delivery of the micellar formulation as a spray, the formulation can be put into an aerosol dispenser and the dispenser is charged with a propellant. The propellant, which is under pressure, is in liquid form in the dispenser. The ratios of the ingredients are adjusted so that the aqueous and propellant phases become one, *i.e.,* there is one phase. If there are two phases, it is necessary to shake the dispenser prior to dispensing a portion of the contents, *e.g.,* through a metered valve. The dispensed dose of pharmaceutical agent is propelled from the metered valve in a fine spray.

Propellants may include hydrogen-containing chlorofluorocarbons, hydrogen-containing fluorocarbons, dimethyl ether and diethyl ether. In certain embodiments, HFA 134a (1,1,1,2 tetrafluoroethane) may be used.

The specific concentrations of the essential ingredients can be determined by relatively straightforward experimentation. For absorption through the oral cavities, it is often desirable to increase, *e.g.,* at least double or triple, the dosage for through injection or administration through the gastrointestinal tract.

*Particles.* For ease of exposition the particles, formulations, compositions and methods in this section are discussed largely with regard to modified siRNA compounds. It may be understood, however, that these particles, formulations, compositions and methods can be practiced with other siRNA compounds, *e.g.,* unmodified siRNA compounds, and such practice is described herein. In another embodiment, an siRNA compound, *e.g.,* a double-stranded siRNA compound, or ssiRNA compound, (*e.g*., a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) preparations may be incorporated into a particle, *e.g.,* a microparticles. Microparticles can be produced by spray-drying, but may also be produced by other methods including lyophilization, evaporation, fluid bed drying, vacuum drying, or combination of these techniques. See below for further description.

Sustained-Release Formulations. An siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) described herein can be formulated for controlled, *e.g.,* slow release. Controlled release can be achieved by disposing the siRNA within a structure or substance which impedes its release. *E.g.,* siRNA can be disposed within a porous matrix or in an erodable matrix, either of which allow release of the siRNA over a period of time.

Polymeric particles, *e.g.,* polymeric in microparticles can be used as a sustained-release reservoir of siRNA that is taken up by cells only released from the microparticle through biodegradation. The polymeric particles in this embodiment should therefore be large enough to preclude phagocytosis (*e.g.,* larger than 10 µm or larger than 20 µm). Such particles can be produced by the same methods to make smaller particles, but with less vigorous mixing of the first and second emulsions. That is to say, a lower homogenization speed, vortex mixing speed, or sonication setting can be used to obtain particles having a diameter around 100 µm rather than 10 µm. The time of mixing also can be altered.

Larger microparticles can be formulated as a suspension, a powder, or an implantable solid, to be delivered by intramuscular, subcutaneous, intradermal, intravenous, or intraperitoneal injection; via inhalation (intranasal or intrapulmonary); orally; or by implantation. These particles are useful for delivery of any siRNA when slow release over a relatively long term is desired. The rate of degradation, and consequently of release, varies with the polymeric formulation.

Microparticles may include pores, voids, hollows, defects or other interstitial spaces that allow the fluid suspension medium to freely permeate or perfuse the particulate boundary. For example, the perforated microstructures can be used to form hollow, porous spray dried microspheres.

Polymeric particles containing siRNA (*e.g*., a siRNA) can be made using a double emulsion technique, for instance. First, the polymer is dissolved in an organic solvent. A polymer may be polylactic-co-glycolic acid (PLGA), with a lactic/glycolic acid weight ratio of 65:35, 50:50, or 75:25. Next, a sample of nucleic acid suspended in aqueous solution is added to the polymer solution and the two solutions are mixed to form a first emulsion. The solutions can be mixed by vortexing or shaking, and in the mixture can be sonicated. Any method by which the nucleic acid receives the least amount of damage in the form of nicking, shearing, or degradation, while still allowing the formation of an appropriate emulsion is possible. For example, acceptable results can be obtained with a Vibra-cell model VC-250 sonicator with a 1/8'' microtip probe, at setting #3.

*Spray Drying.* An siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof)) can be prepared by spray drying. Spray dried siRNA can be administered to a subject or be subjected to further formulation. A pharmaceutical composition of siRNA can be prepared by spray drying a homogeneous aqueous mixture that includes a siRNA under conditions sufficient to provide a dispersible powdered composition, *e.g.,* a pharmaceutical composition. The material for spray drying can also include one or more of: a pharmaceutically acceptable excipient, or a dispersibility-enhancing amount of a physiologically acceptable, water-soluble protein. The spray-dried product can be a dispersible powder that includes the siRNA.

Spray drying is a process that converts a liquid or slurry material to a dried particulate form. Spray drying can be used to provide powdered material for various administrative routes including inhalation. See, for example, M. Sacchetti and M. M. Van Oort in: Inhalation Aerosols: Physical and Biological Basis for Therapy, A. J. Hickey, ed. Marcel Dekkar, New York, 1996.

Spray drying can include atomizing a solution, emulsion, or suspension to form a fine mist of droplets and drying the droplets. The mist can be projected into a drying chamber (*e.g.,* a vessel, tank, tubing, or coil) where it contacts a drying gas. The mist can include solid or liquid pore forming agents. The solvent and pore forming agents evaporate from the droplets into the drying gas to solidify the droplets, simultaneously forming pores throughout the solid. The solid (typically in a powder, particulate form) then is separated from the drying gas and collected.

Spray drying includes bringing together a highly dispersed liquid, and a sufficient volume of air (*e.g.,* hot air) to produce evaporation and drying of the liquid droplets. The preparation to be spray dried can be any solution, course suspension, slurry, colloidal dispersion, or paste that may be atomized using the selected spray drying apparatus. Typically, the feed is sprayed into a current of warm filtered air that evaporates the solvent and conveys the dried product to a collector. The spent air is then exhausted with the solvent. Several different types of apparatus may be used to provide the desired product. For example, commercial spray dryers manufactured by Buchi Ltd. or Niro Corp. can effectively produce particles of desired size.

Spray-dried powdered particles can be approximately spherical in shape, nearly uniform in size and frequently hollow. There may be some degree of irregularity in shape depending upon the incorporated medicament and the spray drying conditions. In many instances the dispersion stability of spray-dried microspheres appears to be more effective if an inflating agent (or blowing agent) is used in their production. Certain embodiments may comprise an emulsion with an inflating agent as the disperse or continuous phase (the other phase being aqueous in nature). An inflating agent may be dispersed with a surfactant solution, using, for instance, a commercially available microfluidizer at a pressure of about 5000 to 15,000 psi. This process forms an emulsion, which may be stabilized by an incorporated surfactant, typically comprising submicron droplets of water immiscible blowing agent dispersed in an aqueous continuous phase. The formation of such dispersions using this and other techniques are common and well known to those in the art. The blowing agent may be a fluorinated compound (*e.g.,* perfluorohexane, perfluorooctyl bromide, perfluorodecalin, perfluorobutyl ethane) which vaporizes during the spray-drying process, leaving behind generally hollow, porous aerodynamically light microspheres. As will be discussed in more detail below, other suitable blowing agents include chloroform, freons, and hydrocarbons. Nitrogen gas and carbon dioxide are also contemplated as a suitable blowing agent.

Although the perforated microstructures may be formed using a blowing agent as described above, it will be appreciated that, in some instances, no blowing agent is required and an aqueous dispersion of the medicament and surfactant(s) are spray dried directly. In such cases, the formulation may be amenable to process conditions (*e.g.,* elevated temperatures) that generally lead to the formation of hollow, relatively porous microparticles. Moreover, the medicament may possess special physicochemical properties (*e.g.,* high crystallinity, elevated melting temperature, surface activity, etc.) that make it particularly suitable for use in such techniques.

The perforated microstructures may optionally be associated with, or comprise, one or more surfactants. Moreover, miscible surfactants may optionally be combined with the suspension medium liquid phase. It will be appreciated by those skilled in the art that the use of surfactants may further increase dispersion stability, simplify formulation procedures or increase bioavailability upon administration. Of course combinations of surfactants, including the use of one or more in the liquid phase and one or more associated with the perforated microstructures are contemplated herein. By "associated with or comprise" it is meant that the structural matrix or perforated microstructure may incorporate, adsorb, absorb, be coated with or be formed by the surfactant.

Surfactants suitable for use include any compound or composition that aids in the formation and maintenance of the stabilized respiratory dispersions by forming a layer at the interface between the structural matrix and the suspension medium. The surfactant may comprise a single compound or any combination of compounds, such as in the case of co-surfactants. Particularly certain surfactants are substantially insoluble in the propellant, nonfluorinated, and selected from the group consisting of saturated and unsaturated lipids, nonionic detergents, nonionic block copolymers, ionic surfactants, and combinations of such agents. It may be emphasized that, in addition to the aforementioned surfactants, suitable (*i.e.,* biocompatible) fluorinated surfactants are compatible with the teachings herein and may be used to provide the desired stabilized preparations.

Lipids, including phospholipids, from both natural and synthetic sources may be used in varying concentrations to form a structural matrix. Generally, compatible lipids comprise those that have a gel to liquid crystal phase transition greater than about 40° C. In certain embodiments, the incorporated lipids are relatively long chain (*i.e.,* C₆ -C₂₂) saturated lipids and may comprise phospholipids. Exemplary phospholipids useful in the disclosed stabilized preparations comprise egg phosphatidylcholine, dilauroylphosphatidylcholine, dioleylphosphatidylcholine, dipalmitoylphosphatidyl-choline, disteroylphosphatidylcholine, short-chain phosphatidylcholines, phosphatidylethanolamine, dioleylphosphatidylethanolamine, phosphatidylserine, phosphatidylglycerol, phosphatidylinositol, glycolipids, ganglioside GM1, sphingomyelin, phosphatidic acid, cardiolipin; lipids bearing polymer chains such as, polyethylene glycol, chitin, hyaluronic acid, or polyvinylpyrrolidone; lipids bearing sulfonated mono-, di-, and polysaccharides; fatty acids such as palmitic acid, stearic acid, and oleic acid; cholesterol, cholesterol esters, and cholesterol hemisuccinate. Due to their excellent biocompatibility characteristics, phospholipids and combinations of phospholipids and poloxamers are particularly suitable for use in the stabilized dispersions disclosed herein.

Compatible nonionic detergents comprise: sorbitan esters including sorbitan trioleate (Spans™ 85), sorbitan sesquioleate, sorbitan monooleate, sorbitan monolaurate, polyoxyethylene (20) sorbitan monolaurate, and polyoxyethylene (20) sorbitan monooleate, oleyl polyoxyethylene (2) ether, stearyl polyoxyethylene (2) ether, lauryl polyoxyethylene (4) ether, glycerol esters, and sucrose esters. Other suitable nonionic detergents can be easily identified using McCutcheon's Emulsifiers and Detergents (McPublishing Co., Glen Rock, N.J.). Certain block copolymers include diblock and triblock copolymers of polyoxyethylene and polyoxypropylene, including poloxamer 188 (Pluronic F68), poloxamer 407 (Pluronic F-127), and poloxamer 338. Ionic surfactants such as sodium sulfosuccinate, and fatty acid soaps may also be utilized. In certain embodiments, the microstructures may comprise oleic acid or its alkali salt.

In addition to the aforementioned surfactants, cationic surfactants or lipids may be used, especially in the case of delivery of an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof). Examples of suitable cationic lipids include: DOTMA, N-[-(2,3-dioleyloxy)propyl]-N,N,N-trimethylammonium-chloride; DOTAP,1,2-dioleyloxy-3-(trimethylammonio)propane; and DOTB, 1,2-dioleyl-3-(4'-trimethylammonio)butanoyl-sn-glycerol. Polycationic amino acids such as polylysine, and polyarginine are also contemplated.

For the spraying process, such spraying methods as rotary atomization, pressure atomization and two-fluid atomization can be used. Examples of the devices used in these processes include "Parubisu [phonetic rendering] Mini-Spray GA-32" and "Parubisu Spray Drier DL-41", manufactured by Yamato Chemical Co., or "Spray Drier CL-8," "Spray Drier L-8," "Spray Drier FL-12," "Spray Drier FL-16" or "Spray Drier FL-20," manufactured by Okawara Kakoki Co., can be used for the method of spraying using rotary-disk atomizer.

While no particular restrictions are placed on the gas used to dry the sprayed material, it is recommended to use air, nitrogen gas or an inert gas. The temperature of the inlet of the gas used to dry the sprayed materials such that it does not cause heat deactivation of the sprayed material. The range of temperatures may vary between about 50°C to about 200°C, for example, between about 50°C and 100°C. The temperature of the outlet gas used to dry the sprayed material, may vary between about 0°C and about 150°C, for example, between 0°C and 90°C, and for example between 0°C and 60°C.

The spray drying is done under conditions that result in substantially amorphous powder of homogeneous constitution having a particle size that is respirable, a low moisture content and flow characteristics that allow for ready aerosolization. In some cases, the particle size of the resulting powder is such that more than about 98% of the mass is in particles having a diameter of about 10 µm or less with about 90% of the mass being in particles having a diameter less than 5 µm. Alternatively, about 95% of the mass will have particles with a diameter of less than 10 µm with about 80% of the mass of the particles having a diameter of less than 5 µm.

The dispersible pharmaceutical-based dry powders that include the siRNA preparation may optionally be combined with pharmaceutical carriers or excipients which are suitable for respiratory and pulmonary administration. Such carriers may serve simply as bulking agents when it is desired to reduce the siRNA concentration in the powder which is being delivered to a patient, but may also serve to enhance the stability of the siRNA compositions and to improve the dispersibility of the powder within a powder dispersion device in order to provide more efficient and reproducible delivery of the siRNA and to improve handling characteristics of the siRNA such as flowability and consistency to facilitate manufacturing and powder filling.

Such carrier materials may be combined with the drug prior to spray drying, *i.e.,* by adding the carrier material to the purified bulk solution. In that way, the carrier particles will be formed simultaneously with the drug particles to produce a homogeneous powder. Alternatively, the carriers may be separately prepared in a dry powder form and combined with the dry powder drug by blending. The powder carriers will usually be crystalline (to avoid water absorption), but might in some cases be amorphous or mixtures of crystalline and amorphous. The size of the carrier particles may be selected to improve the flowability of the drug powder, typically being in the range from 25 µm to 100 µm. A carrier material may be crystalline lactose having a size in the above-stated range.

Powders prepared by any of the above methods will be collected from the spray dryer in a conventional manner for subsequent use. For use as pharmaceuticals and other purposes, it will frequently be desirable to disrupt any agglomerates which may have formed by screening or other conventional techniques. For pharmaceutical uses, the dry powder formulations will usually be measured into a single dose, and the single dose sealed into a package. Such packages are particularly useful for dispersion in dry powder inhalers, as described in detail below. Alternatively, the powders may be packaged in multiple-dose containers.

Methods for spray drying hydrophobic and other drugs and components are described in U.S. Pat. Nos. 5,000,888; 5,026,550; 4,670,419, 4,540,602; and 4,486,435. Bloch and Speison (1983) Pharm. Acta Helv 58:14-22 teaches spray drying of hydrochlorothiazide and chlorthalidone (lipophilic drugs) and a hydrophilic adjuvant (pentaerythritol) in azeotropic solvents of dioxane-water and 2-ethoxyethanol-water. A number of Japanese Patent application Abstracts relate to spray drying of hydrophilic-hydrophobic product combinations, including JP 806766; JP 7242568; JP 7101884; JP 7101883; JP 71018982; JP 7101881; and JP 4036233. Other foreign patent publications relevant to spray drying hydrophilic-hydrophobic product combinations include FR 2594693; DE 2209477; and WO 88/07870.

In one aspect, described herein is a spray-dried siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) composition suitable for inhalation by a subject, including: (a) a therapeutically effective amount of a siRNA compound suitable for treating a condition in the subject by inhalation; (b) a pharmaceutically acceptable excipient selected from the group consisting of carbohydrates and amino acids; and (c) optionally, a dispersibility-enhancing amount of a physiologically-acceptable, water-soluble polypeptide.

In one embodiment, the excipient is a carbohydrate. The carbohydrate can be selected from the group consisting of monosaccharides, disaccharides, trisaccharides, and polysaccharides. In some embodiments the carbohydrate is a monosaccharide selected from the group consisting of dextrose, galactose, mannitol, D-mannose, sorbitol, and sorbose. In another embodiment the carbohydrate is a disaccharide selected from the group consisting of lactose, maltose, sucrose, and trehalose.

In another embodiment, the excipient is an amino acid. In one embodiment, the amino acid is a hydrophobic amino acid. In some embodiments the hydrophobic amino acid is selected from the group consisting of alanine, isoleucine, leucine, methionine, phenylalanine, proline, tryptophan, and valine. In yet another embodiment the amino acid is a polar amino acid. In some embodiments the amino acid is selected from the group consisting of arginine, histidine, lysine, cysteine, glycine, glutamine, serine, threonine, tyrosine, aspartic acid and glutamic acid.

In one embodiment, the dispersibility-enhancing polypeptide is selected from the group consisting of human serum albumin, α-lactalbumin, trypsinogen, and polyalanine.

In one embodiment, the spray-dried siRNA compound composition includes particles having a mass median diameter (MMD) of less than 10 microns. In another embodiment, the spray-dried siRNA compound composition includes particles having a mass median diameter of less than 5 microns. In yet another embodiment the spray-dried siRNA compound composition includes particles having a mass median aerodynamic diameter (MMAD) of less than 5 microns.

*Lyophilization.* An siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) preparation can be made by lyophilization. Lyophilization is a freeze-drying process in which water is sublimed from the composition after it is frozen. The particular advantage associated with the lyophilization process is that biologicals and pharmaceuticals that are relatively unstable in an aqueous solution can be dried without elevated temperatures (thereby eliminating the adverse thermal effects), and then stored in a dry state where there are few stability problems. Such techniques are particularly compatible with the incorporation of nucleic acids in perforated microstructures without compromising physiological activity. Methods for providing lyophilized particulates are known to those of skill in the art and it would clearly not require undue experimentation to provide dispersion compatible microstructures in accordance with the teachings herein. Accordingly, to the extent that lyophilization processes may be used to provide microstructures having the desired porosity and size, they are conformance with the teachings herein and are expressly contemplated herein.

### Pharmaceutical Compositions

In one aspect, described herein is a pharmaceutical composition that includes an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) including a nucleotide sequence complementary to a target RNA, *e.g.,* substantially and/or exactly complementary. The target RNA can be a transcript of an endogenous human gene. In one embodiment, the siRNA compound (a) is 19-25 nucleotides long, for example, 21-23 nucleotides, (b) is complementary to an endogenous target RNA, and, optionally, (c) includes at least one 3' overhang 1-5 nt long. In one embodiment, the pharmaceutical composition can be an emulsion, microemulsion, cream, jelly, or liposome.

In one example the pharmaceutical composition includes an siRNA compound mixed with a topical delivery agent. The topical delivery agent can be a plurality of microscopic vesicles. The microscopic vesicles can be liposomes. In some embodiments the liposomes are cationic liposomes.

In another aspect, the pharmaceutical composition includes an siRNA compound, *e.g.,* a double-stranded siRNA compound, or ssiRNA compound (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) admixed with a topical penetration enhancer. In one embodiment, the topical penetration enhancer is a fatty acid. The fatty acid can be arachidonic acid, oleic acid, lauric acid, caprylic acid, capric acid, myristic acid, palmitic acid, stearic acid, linoleic acid, linolenic acid, dicaprate, tricaprate, monolein, dilaurin, glyceryl 1-monocaprate, 1-dodecylazacycloheptan-2-one, an acylcarnitine, an acylcholine, or a C₁₋₁₀ alkyl ester, monoglyceride, diglyceride or pharmaceutically acceptable salt thereof.

In another embodiment, the topical penetration enhancer is a bile salt. The bile salt can be cholic acid, dehydrocholic acid, deoxycholic acid, glucholic acid, glycholic acid, glycodeoxycholic acid, taurocholic acid, taurodeoxycholic acid, chenodeoxycholic acid, ursodeoxycholic acid, sodium tauro-24,25-dihydro-fusidate, sodium glycodihydrofusidate, polyoxyethylene-9-lauryl ether or a pharmaceutically acceptable salt thereof.

In another embodiment, the penetration enhancer is a chelating agent. The chelating agent can be EDTA, citric acid, a salicyclate, a N-acyl derivative of collagen, laureth-9, an N-amino acyl derivative of a beta-diketone or a mixture thereof.

In another embodiment, the penetration enhancer is a surfactant, *e.g.,* an ionic or nonionic surfactant. The surfactant can be sodium lauryl sulfate, polyoxyethylene-9-lauryl ether, polyoxyethylene-20-cetyl ether, a perfluorchemical emulsion or mixture thereof.

In another embodiment, the penetration enhancer can be selected from a group consisting of unsaturated cyclic ureas, 1-alkyl-alkones, 1-alkenylazacyclo-alakanones, steroidal antiinflammatory agents and mixtures thereof. In yet another embodiment the penetration enhancer can be a glycol, a pyrrol, an azone, or a terpenes.

In one aspect, described herein is a pharmaceutical composition including an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in a form suitable for oral delivery. In one embodiment, oral delivery can be used to deliver an siRNA compound composition to a cell or a region of the gastro-intestinal tract, *e.g.,* small intestine, colon (*e.g.,* to treat a colon cancer), and so forth. The oral delivery form can be tablets, capsules or gel capsules. In one embodiment, the siRNA compound of the pharmaceutical composition modulates expression of a cellular adhesion protein, modulates a rate of cellular proliferation, or has biological activity against eukaryotic pathogens or retroviruses. In another embodiment, the pharmaceutical composition includes an enteric material that substantially prevents dissolution of the tablets, capsules or gel capsules in a mammalian stomach. In some embodiments the enteric material is a coating. The coating can be acetate phthalate, propylene glycol, sorbitan monoleate, cellulose acetate trimellitate, hydroxy propyl methylcellulose phthalate or cellulose acetate phthalate.

In another embodiment, the oral dosage form of the pharmaceutical composition includes a penetration enhancer. The penetration enhancer can be a bile salt or a fatty acid. The bile salt can be ursodeoxycholic acid, chenodeoxycholic acid, and salts thereof. The fatty acid can be capric acid, lauric acid, and salts thereof.

In another embodiment, the oral dosage form of the pharmaceutical composition includes an excipient. In one example the excipient is polyethyleneglycol. In another example the excipient is precirol.

In another embodiment, the oral dosage form of the pharmaceutical composition includes a plasticizer. The plasticizer can be diethyl phthalate, triacetin dibutyl sebacate, dibutyl phthalate or triethyl citrate.

In one aspect, described herein is a pharmaceutical composition including an siRNA compound and a delivery vehicle. In one embodiment, the siRNA compound is (a) is 19-25 nucleotides long, for example, 21-23 nucleotides, (b) is complementary to an endogenous target RNA, and, optionally, (c) includes at least one 3' overhang 1-5 nucleotides long.

In one embodiment, the delivery vehicle can deliver an siRNA compound, e.g., a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) to a cell by a topical route of administration. The delivery vehicle can be microscopic vesicles. In one example the microscopic vesicles are liposomes. In some embodiments the liposomes are cationic liposomes. In another example the microscopic vesicles are micelles. In one aspect, described herein is a pharmaceutical composition including an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in an injectable dosage form. In one embodiment, the injectable dosage form of the pharmaceutical composition includes sterile aqueous solutions or dispersions and sterile powders. In some embodiments the sterile solution can include a diluent such as water; saline solution; fixed oils, polyethylene glycols, glycerin, or propylene glycol.

In one aspect, described herein is a pharmaceutical composition including an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in oral dosage form. In one embodiment, the oral dosage form is selected from the group consisting of tablets, capsules and gel capsules. In another embodiment, the pharmaceutical composition includes an enteric material that substantially prevents dissolution of the tablets, capsules or gel capsules in a mammalian stomach. In some embodiments the enteric material is a coating. The coating can be acetate phthalate, propylene glycol, sorbitan monoleate, cellulose acetate trimellitate, hydroxy propyl methyl cellulose phthalate or cellulose acetate phthalate. In one embodiment, the oral dosage form of the pharmaceutical composition includes a penetration enhancer, *e.g.,* a penetration enhancer described herein.

In another embodiment, the oral dosage form of the pharmaceutical composition includes an excipient. In one example the excipient is polyethyleneglycol. In another example the excipient is precirol.

In another embodiment, the oral dosage form of the pharmaceutical composition includes a plasticizer. The plasticizer can be diethyl phthalate, triacetin dibutyl sebacate, dibutyl phthalate or triethyl citrate.

In one aspect, described herein is a pharmaceutical composition including an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in a rectal dosage form. In one embodiment, the rectal dosage form is an enema. In another embodiment, the rectal dosage form is a suppository.

In one aspect, described herein is a pharmaceutical composition including an siRNA compound, *e.g.,* a double-stranded siRNA compound, or ssiRNA compound, (*e.g*., a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in a vaginal dosage form. In one embodiment, the vaginal dosage form is a suppository. In another embodiment, the vaginal dosage form is a foam, cream, or gel.

In one aspect, described herein is a pharmaceutical composition including an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) in a pulmonary or nasal dosage form. In one embodiment, the siRNA compound is incorporated into a particle, *e.g.,* a macroparticle, *e.g.,* a microsphere. The particle can be produced by spray drying, lyophilization, evaporation, fluid bed drying, vacuum drying, or a combination thereof. The microsphere can be formulated as a suspension, a powder, or an implantable solid.

### Methods of Treatment of Conditions and Diseases

A subject can be treated by administering a defined amount of an siRNA compound, *e.g.,* a double-stranded siRNA compound, or ssiRNA compound, (*e.g.,* a precursor, *e.g.,* a larger siRNA compound which can be processed into a ssiRNA compound) composition that is in a powdered form, e.g., a collection of microparticles, such as crystalline particles. The composition can include a plurality of siRNA compounds, *e.g.,* specific for one or more different endogenous target RNAs. The method can include other features described herein.

A subject can be treated by administering a defined amount of an siRNA compound composition that is prepared by a method that includes spray-drying, *i.e.,* atomizing a liquid solution, emulsion, or suspension, immediately exposing the droplets to a drying gas, and collecting the resulting porous powder particles. The composition can include a plurality of siRNA compounds, *e.g.,* specific for one or more different endogenous target RNAs. The method can include other features described herein.

In one aspect, described herein is a method of treating a subject at risk for or afflicted with a disease that may benefit from the administration of the siRNA described herein. The method comprises administering the siRNA to a subject in need thereof, thereby treating the subject. The nucleic acid that is administered will depend on the condition or disease being treated.

*Genes.* Gene expression in a subject can be modulated by administering a pharmaceutical composition including an siRNA compound.

The transcriptional complex hypoxia inducible factor (HIF) is a key regulator of oxygen homeostasis. Hypoxia induces the expression of genes participating in many cellular and physiological processes, including oxygen transport and iron metabolism, erythropoiesis, angiogenesis, glycolysis and glucose uptake, transcription, metabolism, pH regulation, growth-factor signaling, response to stress and cell adhesion. These gene products participate in either increasing oxygen delivery to hypoxic tissues or activating an alternative metabolic pathway (glycolysis) which does not require oxygen. Hypoxia-induced pathways, in addition to being required for normal cellular processes, can also aid tumor growth by allowing or aiding angiogenesis, immortalization, genetic instability, tissue invasion and metastasis (Harris, Nat. Rev. Cancer, 2002, 2, 38-47; Maxwell et al., Curr. Opin. Genet. Dev., 2001, 11, 293-299). The transcription factor hypoxia-inducible factor 1 (HIF-1) plays an essential role in homeostatic responses to hypoxia by binding to the DNA sequence 5'-TACGTGCT-3' and activating the transcription of dozens of genes in vivo under hypoxic conditions (Wang and Semenza, J. Biol. Chem., 1995, 270, 1230-1237). Hypoxia-inducible factor-1 alpha is a heterodimer composed of a 120 kDa alpha subunit complexed with a 91 to 94 kDa beta subunit, both of which contain a basic helix-loop-helix. The gene encoding hypoxia-inducible factor-1 alpha (HIF1α also called HIF-1 alpha, HIF1A, HIF-1A, HIF1-A, and MOP1) was cloned in 1995 (Wang et al., Proc. Natl. Acad. Sci. U.S.A., 1995, 92, 5510-5514). A nucleic acid sequence encoding HIF1α, is disclosed and claimed in U.S. Pat. No. 5,882,914, as are expression vectors expressing the recombinant DNA, and host cells containing said vectors (Semenza, 1999). US Patent 7,217,572 discloses at SEQ ID NO: 1.89 the antisense oligonucleotides sequence: GTGCAGTATT GTAGCCAGGC (SEQ ID NO: 1), and discloses at SEQ ID NO: 446 the antisense oligonucleotide sequence: CCTCATGGTC ACATGGATGA (SEQ ID NO: 2).

Aberrant expression of or constitutive expression of STAT3 is associated with a number of disease processes. STAT3 has been shown to be involved in cell transformation. Constitutive activation and/or overexpression of STAT3 appears to be involved in several forms of cancer, including myeloma, breast carcinomas, prostate cancer, brain tumors, head and neck carcinomas, melanoma, leukemias and lymphomas, particularly chronic myelogenous leukemia and multiple myeloma. Niu et al., Cancer Res., 1999, 59, 5059-5063. Breast cancer cell lines that overexpress EGFR constitutively express phosphorylated STAT3 (Sartor, C. I., et al., Cancer Res., 1997, 57, 978-987; Garcia, R., et al., Cell Growth and Differentiation, 1997, 8, 1267-1276). Activated STAT3 levels were also found to be elevated in low grade glioblastomas and medulloblastomas (Cattaneo, E., et al., Anticancer Res., 1998, 18, 2381-2387). US Patent 7,307,069 discloses at SEQ ID NO: 184 the antisense oligonucleotide sequence: TTGGCTTCTC AAGATACCTG (SEQ ID NO: 3), and discloses at SEQ ID NO: 342 the antisense oligonucleotides sequence: GACTCTTGCA GGAAGCGGCT (SEQ ID NO: 4).

Huntington's disease is a progressive neurodegenerative disorder characterized by motor disturbance, cognitive loss and psychiatric manifestations (Martin and Gusella, N. Engl. J. Med. 315:1267-1276 (1986). Although an actual mechanism for Huntington's disease remains elusive, Huntington's disease has been shown to be an autosomal dominant neurodegenerative disorder caused by an expanding glutamine repeat in a gene termed IT15 or Huntingtin (HD). Although this gene is widely expressed and is required for normal development, the pathology of Huntington's disease is restricted to the brain, for reasons that remain poorly understood. The Huntingtin gene product is expressed at similar levels in patients and controls, and the genetics of the disorder suggest that the expansion of the polyglutamine repeat induces a toxic gain of function, perhaps through interactions with other cellular proteins. US Patent 7,320,965 discloses an antisense strand for inhibiting the expression of a human Huntingtin gene at SEQ ID NO: 793: CUGCACGGUU CUUUGUGACT T (SEQ ID NO: 5).

The intracellular transport of proteins, lipids, and mRNA to specific locations within the cell, as well as the proper alignment and separation of chromosomes in dividing cells, is essential to the functioning of the cell. The superfamily of proteins called kinesins (KIF), along with the myosins and dyneins, function as molecular engines to bind and transport vesicles and organelles along microtubules with energy supplied by ATP. KIFs have been identified in many species ranging from yeast to humans. The amino acid sequences which comprise the motor domain are highly conserved among eukaryotic phyla, while the region outside of the motor domain serves to bind to the cargo and varies in amino acid sequence among KIFs. The movement of a kinesin along a microtubule can occur in either the plus or minus direction, but any given kinesin can only travel in one direction, an action that is mediated by the polarity of the motor and the microtubule. The KIFs have been grouped into three major types depending on the position of the motor domain: the amino-terminal domain, the middle motor domain, and the carboxyl-terminal domain, referred to respectively as N-kinesin, M-kinesin, and C-kinesins. These are further classified into 14 classes based on a phylogenetic analysis of the 45 known human and mouse kinesin genes (Miki et al., Proc. Natl. Acad. Sci. U.S.A., 2001, 98, 7004-7011). One such kinesin, kinesin-like 1, a member of the N-2 (also called bimC) family of kinesins and is involved in separating the chromosomes by directing their movement along microtubules in the bipolar spindle. During mitosis, the microtubule bipolar spindle functions to distribute the duplicated chromosomes equally to daughter cells. Kinesin-like 1 is first phosphorylated by the kinase p34^{cdc2} and is essential for centrosome separation and assembly of bipolar spindles at prophase (Blangy et al., Cell, 1995, 83, 1159-1169). In rodent neurons, kinesin-like 1 is expressed well past their terminal mitotic division, and has been implicated in regulating microtubule behaviors within the developing axons and dendrites (Ferhat et al., J. Neurosci., 1998, 18, 7822-7835). The gene encoding human kinesin-like 1 (also called KNSL1, Eg5, HsEg5, HKSP, KIF11, thyroid interacting protein 5, and TRIP5) was cloned in 1995 (Blangy et al., Cell, 1995, 83, 1159-1169). Inhibition of kinesin-like 1 has been suggested as a target for arresting cellular proliferation in cancer because of the central role kinesin-like 1 holds in mitosis. Expression of kinesin-like 1 may also contribute to other disease states. A contribution of kinesin-like 1 to B-cell leukemia has been demonstrated in mice as a result of upregulated expression of kinesin-like 1 following a retroviral insertion mutation in the proximity of the kinesin-like 1 gene (Hansen and Justice, Oncogene, 1999, 18, 6531-6539). Autoantibodies to a set of proteins in the mitotic spindle assembly have been detected in human sera and these autoantibodies have been associated with autoimmune diseases including carpal tunnel syndrome, Raynaud's phenomenon, systemic sclerosis, Sjorgren's syndrome, rheumatoid arthritis, polymyositis, and polyarteritis. One of these autoantigens is kinesin-like 1 and has been identified in systemic lupus erythematosus (Whitehead et al., Arthritis Rheum., 1996, 39, 1635-1642). US Patent 7,199,107 discloses an antisense strand for inhibiting the expression of a human kinesin-1 gene at NO: 122: ACGTGGAATT ATACCAGCCA (SEQ ID NO: 6).

A number of therapeutic strategies exist for inhibiting aberrant angiogenesis, which attempt to reduce the production or effect of VEGF. For example, anti-VEGF or anti-VEGF receptor antibodies (Kim E S et al. (2002), PNAS USA 99: 11399-11404), and soluble VEGF "traps" which compete with endothelial cell receptors for VEGF binding (Holash J et al. (2002), PNAS USA 99: 11393-11398) have been developed. Classical VEGF "antisense" or aptamer therapies directed against VEGF gene expression have also been proposed (U.S. published application 2001/0021772 of Uhlmann et al.*.* However, the anti-angiogenic agents used in these therapies can produce only a stoichiometric reduction in VEGF or VEGF receptor, and the agents are typically overwhelmed by the abnormally high production of VEGF by the diseased tissue. The results achieved with available anti-angiogenic therapies have therefore been unsatisfactory. US Patent 7,345,027 discloses an antisense strand for inhibiting the expression of a human VEGF gene at SEQ ID NO: 78: GUGCUGGCCUUGGUGAGGU***TT*** (The terminal two Ts are overhangs; SEQ ID NO: 7).

The NF-κB or nuclear factor κB is a transcription factor that plays a critical role in inflammatory diseases by inducing the expression of a large number of proinflammatory and anti-apoptotic genes. These include cytokines such as IL-1, IL-2, IL-11, TNF-α and IL-6, chemokines including IL-8, GRO1 and RANTES, as well as other proinflammatory molecules including COX-2 and cell adhesion molecules such as ICAM-1, VCAM-1, and E-selectin. Pahl H L, (1999) Oncogene 18, 6853-6866; Jobin et al, (2000) Am. J. Physiol. Cell. Physiol. 278: 451-462. Under resting conditions, NF-κB is present in the cytosol of cells as a complex with IκB. The IκB family of proteins serve as inhibitors of NF-κB, interfering with the function of its nuclear localization signal (see for example U. Siebenlist et al, (1994) Ann. Rev. Cell Bio., 10: 405). Upon disruption of the IκB-NF-κB complex following cell activation, NF-κB translocates to the nucleus and activates gene transcription. Disruption of the IκB-NF-κB complex and subsequent activation of NF-κB is initiated by degradation of IκB. Activators of NF-κB mediate the site-specific phosphorylation of two amino terminal serines in each IκB which makes nearby lysines targets for ubiquitination, thereby resulting in IκB proteasomal destruction. NF-κB is then free to translocate to the nucleus and bind DNA leading to the activation of a host of inflammatory response target genes. (Baldwin, A., Jr., (1996) Annu Rev Immunol 14: 649-683, Ghosh, S. et al, (1998) Annu Rev Immunol 16, 225-260.) Recent evidence has shown that NF-κB subunits dynamically shuttle between the cytoplasm and the nucleus but a dominant acting nuclear export signal in IκBα ensures their transport back to the cytoplasm. Even though NF-κB is largely considered to be a transcriptional activator, under certain circumstances it can also be involved in directly repressing gene expression (reviewed in Ghosh, S. et al. (1998) Annu. Rev. Immunol., 16: 225-260). US Patent 7,235,654 discloses an siRNA at SEQ ID NO: 3: GUCUGUGUAU CACGUGACGN N (wherein N is a 2'-deoxy-thymidine; SEQ ID NO: 8).

Control of the risk factors involved in hypercholesterolemia and cardiovascular disease has been the focus of much research in academia and industry. Because an elevated level of circulating plasma low-density lipoprotein cholesterol has been identified as an independent risk factor in the development of hypercholesterolemia and cardiovascular disease, many strategies have been directed at lowering the levels of cholesterol carried in this atherogenic lipoprotein. AcylCoA cholesterol acyltransferase (ACAT) enzymes catalyze the synthesis of cholesterol esters from free cholesterol and fatty acyl-CoA. These enzymes are also involved in regulation of the concentration of cellular free sterols (Buhman et al., Biochim. Biophys. Acta, 2000, 1529, 142-154; Burnett et al., Clin. Chim. Acta, 1999, 286, 231-242; Chang et al., Annu. Rev. Biochem., 1997, 66, 613-638; Rudel et al., Curr. Opin. Lipidol., 2001, 12, 121-127; Rudel and Shelness, Nat. Med., 2000, 6, 1313-1314). Chang *et al.* cloned the first example of a human ACAT gene in 1993 (Chang et al., J. Biol. Chem., 1993, 268, 20747-20755). This original ACAT enzyme is now known as ACAT-1. Subsequently, the work of Meiner *et al.* suggested the presence of more than one ACAT gene in mammals (Meiner et al., J. Lipid Res., 1997, 38, 1928-1933). The cloning and expression of a second human ACAT isoform now known as acyl CoA cholesterol acyltransferase-2, was accomplished recently (Oelkers et al., J. Biol. Chem., 1998, 273, 26765-26771). Murine acyl CoA cholesterol acyltransferase-2 has also been identified and cloned (Cases et al., J. Biol. Chem., 1998, 273, 26755-26764). US Patent 7,335,764 discloses siRNAs targeted to a nucleic acid molecule encoding acyl CoA cholesterol acyltransferase-2 at SEQ ID NOs: 25 (GCACGAAGGA TCCCAGGCAC (SEQ ID NO: 9)), 26 (GGATCCCCTC ACCTCGTCTG (SEQ ID NO: 10)) and 27 (GTTCTTGGCC ACATAATTCC (SEQ ID NO: 11)).

Lp(a) contains two disulfide-linked distinct proteins, apolipoprotein(a) (or ApoA) and apolipoprotein B (or ApoB) (Rainwater and Kammerer, J. Exp. Zool., 1998, 282, 54-61). Apolipoprotein(a) is a unique apolipoprotein encoded by the LPA gene which has been shown to exclusively control the physiological concentrations of Lp(a) (Rainwater and Kammerer, J. Exp. Zool., 1998, 282, 54-61). It varies in size due to interallelic differences in the number of tandemly repeated Kringle 4-encoding 5.5 kb sequences in the LPA gene (Rainwater and Kammerer, J. Exp. Zool., 1998, 282, 54-61). Elevated plasma levels of Lp(a), caused by increased expression of apolipoprotein(a), are associated with increased risk for atherosclerosis and its manifestations, which include hypercholesterolemia (Seed et al., N. Engl. J. Med., 1990, 322, 1494-1499), myocardial infarction (Sandkamp et al., Clin. Chem., 1990, 36, 20-23), and thrombosis (Nowak-Gottl et al., Pediatrics, 1997, 99, E11). Moreover, the plasma concentration of Lp(a) is strongly influenced by heritable factors and is refractory to most drug and dietary manipulation (Katan and Beynen, Am. J. Epidemiol., 1987, 125, 387-399; Vessby et al., Atherosclerosis, 1982, 44, 61-71.). Pharmacologic therapy of elevated Lp(a) levels has been only modestly successful and apheresis remains the most effective therapeutic modality (Hajjar and Nachman, Annu. Rev. Med., 1996, 47, 423-442). US Patent 7,259,150 discloses an siRNA for inhibiting the expression of apolipoprotein(a) at SEQ ID NO: 23 (ACCTGACACC GGGATCCCTC (SEQ ID NO: 12)).

In certain embodiments, the siRNA compound (e.g., the siRNA in a composition described herein) silences a growth factor or growth factor receptor gene, a kinase, e.g., a protein tyrosine, serine or threonine kinase gene, an adaptor protein gene, a gene encoding a G protein superfamily molecule, or a gene encoding a transcription factor.

In some embodiments the siRNA compound (e.g., the siRNA in a composition described herein) silences the PDGF beta gene, and thus can be used to treat a subject having or at risk for a disorder characterized by unwanted PDGF beta expression, e.g., testicular and lung cancers.

In some embodiments the siRNA compound (e.g., the siRNA in a composition described herein) silences the Erb-B gene, and thus can be used to treat a subject having or at risk for a disorder characterized by unwanted Erb-B expression, e.g., breast cancer.

In some embodiments the siRNA compound (e.g., the siRNA in a composition described herein) silences the Src gene, and thus can be used to treat a subject having or at risk for a disorder characterized by unwanted Src expression, e.g., colon cancers.

In some embodiments the siRNA compound (e.g., the siRNA in a composition described herein) silences the CRK gene, and thus can be used to treat a subject having or at risk for a disorder characterized by unwanted CRK expression, e.g., colon and lung cancers.

In some embodiments the siRNA compound (e.g., the siRNA in a composition described herein) silences the GRB2 gene, and thus can be used to treat a subject having or at risk for a disorder characterized by unwanted GRB2 expression, e.g., squamous cell carcinoma.

In another embodiment the siRNA compound (e.g., the siRNA in a composition described herein) silences the RAS gene, and thus can be used to treat a subject having or at risk for a disorder characterized by unwanted RAS expression, e.g., pancreatic, colon and lung cancers, and chronic leukemia.

In another embodiment the siRNA compound (e.g., the siRNA in a composition described herein) silences the MEKK gene, and thus can be used to treat a subject having or at risk for a disorder characterized by unwanted MEKK expression, e.g., squamous cell carcinoma, melanoma or leukemia.

In another embodiment the siRNA compound (e.g., the siRNA in a composition described herein) silences the JNK gene, and thus can be used to treat a subject having or at risk for a disorder characterized by unwanted JNK expression, e.g., pancreatic or breast cancers.

In some embodiments the siRNA compound (e.g., the siRNA in a composition described herein) silences the RAF gene, and thus can be used to treat a subject having or at risk for a disorder characterized by unwanted RAF expression, e.g., lung cancer or leukemia.

In some embodiments the siRNA compound (e.g., the siRNA in a composition described herein) silences the Erkl/2 gene, and thus can be used to treat a subject having or at risk for a disorder characterized by unwanted Erkl/2 expression, e.g., lung cancer.

In another embodiment the siRNA compound (e.g., the siRNA in a composition described herein) silences the PCNA(p21) gene, and thus can be used to treat a subject having or at risk for a disorder characterized by unwanted PCNA expression, e.g., lung cancer.

In some embodiments the siRNA compound (e.g., the siRNA in a composition described herein) silences the MYB gene, and thus can be used to treat a subject having or at risk for a disorder characterized by unwanted MYB expression, e.g., colon cancer or chronic myelogenous leukemia.

In some embodiments the siRNA compound (e.g., the siRNA in a composition described herein) silences the c-MYC gene, and thus can be used to treat a subject having or at risk for a disorder characterized by unwanted c-MYC expression, e.g., Burkitt's lymphoma or neuroblastoma.

In another embodiment the siRNA compound (e.g., the siRNA in a composition described herein) silences the JUN gene, and thus can be used to treat a subject having or at risk for a disorder characterized by unwanted JUN expression, e.g., ovarian, prostate or breast cancers.

In another embodiment the siRNA compound (e.g., the siRNA in a composition described herein) silences the FOS gene, and thus can be used to treat a subject having or at risk for a disorder characterized by unwanted FOS expression, e.g., skin or prostate cancers.

In some embodiments the siRNA compound (e.g., the siRNA in a composition described herein) silences the BCL-2 gene, and thus can be used to treat a subject having or at risk for a disorder characterized by unwanted BCL-2 expression, e.g., lung or prostate cancers or Non-Hodgkin lymphoma.

In some embodiments the siRNA compound (e.g., the siRNA in a composition described herein) silences the Cyclin D gene, and thus can be used to treat a subject having or at risk for a disorder characterized by unwanted Cyclin D expression, e.g., esophageal and colon cancers.

In some embodiments the siRNA compound (e.g., the siRNA in a composition described herein) silences the VEGF gene, and thus can be used to treat a subject having or at risk for a disorder characterized by unwanted VEGF expression, e.g., esophageal and colon cancers.

In some embodiments the siRNA compound (e.g., the siRNA in a composition described herein) silences the EGFR gene, and thus can be used to treat a subject having or at risk for a disorder characterized by unwanted EGFR expression, e.g., breast cancer.

In another embodiment the siRNA compound (e.g., the siRNA in a composition described herein) silences the Cyclin A gene, and thus can be used to treat a subject having or at risk for a disorder characterized by unwanted Cyclin A expression, e.g., lung and cervical cancers.

In another embodiment the siRNA compound (e.g., the siRNA in a composition described herein) silences the Cyclin E gene, and thus can be used to treat a subject having or at risk for a disorder characterized by unwanted Cyclin E expression, e.g., lung and breast cancers.

In another embodiment the siRNA compound (e.g., the siRNA in a composition described herein) silences the WNT-1 gene, and thus can be used to treat a subject having or at risk for a disorder characterized by unwanted WNT-1 expression, e.g., basal cell carcinoma.

In another embodiment the siRNA compound (e.g., the siRNA in a composition described herein) silences the beta-catenin gene, and thus can be used to treat a subject having or at risk for a disorder characterized by unwanted beta-catenin expression, e.g., adenocarcinoma or hepatocellular carcinoma.

In another embodiment the siRNA compound (e.g., the siRNA in a composition described herein) silences the c-MET gene, and thus can be used to treat a subject having or at risk for a disorder characterized by unwanted c-MET expression, e.g., hepatocellular carcinoma.

In another embodiment the siRNA compound (e.g., the siRNA in a composition described herein) silences the PKC gene, and thus can be used to treat a subject having or at risk for a disorder characterized by unwanted PKC expression, e.g., breast cancer.

In some embodiments the siRNA compound (e.g., the siRNA in a composition described herein) silences the NFKB gene, and thus can be used to treat a subject having or at risk for a disorder characterized by unwanted NFKB expression, e.g., breast cancer.

In some embodiments the siRNA compound (e.g., the siRNA in a composition described herein) silences the STAT3 gene, and thus can be used to treat a subject having or at risk for a disorder characterized by unwanted STAT3 expression, e.g., prostate cancer.

In another embodiment the siRNA compound (e.g., the siRNA in a composition described herein) silences the survivin gene, and thus can be used to treat a subject having or at risk for a disorder characterized by unwanted survivin expression, e.g., cervical or pancreatic cancers.

In another embodiment the siRNA compound (e.g., the siRNA in a composition described herein) silences the Her2/Neu gene, and thus can be used to treat a subject having or at risk for a disorder characterized by unwanted Her2/Neu expression, e.g., breast cancer.

In another embodiment the siRNA compound (e.g., the siRNA in a composition described herein) silences the topoisomerase I gene, and thus can be used to treat a subject having or at risk for a disorder characterized by unwanted topoisomerase I expression, e.g., ovarian and colon cancers.

In some embodiments the siRNA compound (e.g., the siRNA in a composition described herein) silences the topoisomerase II alpha gene, and thus can be used to treat a subject having or at risk for a disorder characterized by unwanted topoisomerase II expression, e.g., breast and colon cancers.

In some embodiments the siRNA compound (e.g., the siRNA in a composition described herein) silences mutations in the p73 gene, and thus can be used to treat a subject having or at risk for a disorder characterized by unwanted p73 expression, e.g., colorectal adenocarcinoma.

In some embodiments the siRNA compound (e.g., the siRNA in a composition described herein) silences mutations in the p21(WAF1/CIP1) gene, and thus can be used to treat a subject having or at risk for a disorder characterized by unwanted p21(WAF1/CIP1) expression, e.g., liver cancer.

In some embodiments the siRNA compound (e.g., the siRNA in a composition described herein) silences mutations in the p27(KIP1) gene, and thus can be used to treat a subject having or at risk for a disorder characterized by unwanted p27(KIP1) expression, e.g., liver cancer.

In some embodiments the siRNA compound (e.g., the siRNA in a composition described herein) silences mutations in the PPM1D gene, and thus can be used to treat a subject having or at risk for a disorder characterized by unwanted PPM1D expression, e.g., breast cancer.

In some embodiments the siRNA compound (e.g., the siRNA in a composition described herein) silences mutations in the RAS gene, and thus can be used to treat a subject having or at risk for a disorder characterized by unwanted RAS expression, e.g., breast cancer.

In another embodiment the siRNA compound (e.g., the siRNA in a composition described herein) silences mutations in the caveolin I gene, and thus can be used to treat a subject having or at risk for a disorder characterized by unwanted caveolin I expression, e.g., esophageal squamous cell carcinoma.

In another embodiment the siRNA compound (e.g., the siRNA in a composition described herein) silences mutations in the MIB I gene, and thus can be used to treat a subject having or at risk for a disorder characterized by unwanted MIB I expression, e.g., male breast carcinoma (MBC).

In another embodiment the siRNA compound (e.g., the siRNA in a composition described herein) silences mutations in the MTAI gene, and thus can be used to treat a subject having or at risk for a disorder characterized by unwanted MTAI expression, e.g., ovarian carcinoma.

In another embodiment the siRNA compound (e.g., the siRNA in a composition described herein) silences mutations in the M68 gene, and thus can be used to treat a subject having or at risk for a disorder characterized by unwanted M68 expression, e.g., human adenocarcinomas of the esophagus, stomach, colon, and rectum.

In certain embodiments the siRNA compound (e.g., the siRNA in a composition described herein) silences mutations in tumor suppressor genes, and thus can be used as a method to promote apoptotic activity in combination with chemotherapeutics.

In some embodiments the siRNA compound (e.g., the siRNA in a composition described herein) silences mutations in the p53 tumor suppressor gene, and thus can be used to treat a subject having or at risk for a disorder characterized by unwanted p53 expression, e.g., gall bladder, pancreatic and lung cancers.

In some embodiments the siRNA compound (e.g., the siRNA in a composition described herein) silences mutations in the p53 family member DN-p63, and thus can be used to treat a subject having or at risk for a disorder characterized by unwanted DN-p63 expression, e.g., squamous cell carcinoma

In some embodiments the siRNA compound (e.g., the siRNA in a composition described herein) silences mutations in the pRb tumor suppressor gene, and thus can be used to treat a subject having or at risk for a disorder characterized by unwanted pRb expression, e.g., oral squamous cell carcinoma

In some embodiments the siRNA compound (e.g., the siRNA in a composition described herein) silences mutations in the APC1 tumor suppressor gene, and thus can be used to treat a subject having or at risk for a disorder characterized by unwanted APC1 expression, e.g., colon cancer.

In some embodiments the siRNA compound (e.g., the siRNA in a composition described herein) silences mutations in the BRCA1 tumor suppressor gene, and thus can be used to treat a subject having or at risk for a disorder characterized by unwanted BRCA1 expression, e.g., breast cancer.

In some embodiments the siRNA compound (e.g., the siRNA in a composition described herein) silences mutations in the PTEN tumor suppressor gene, and thus can be used to treat a subject having or at risk for a disorder characterized by unwanted PTEN expression, e.g., hamartomas, gliomas, and prostate and endometrial cancers.

In some embodiments the siRNA compound (e.g., the siRNA in a composition described herein) silences MLL fusion genes, e.g., MLL-AF9, and thus can be used to treat a subject having or at risk for a disorder characterized by unwanted MLL fusion gene expression, e.g., acute leukemias

In another embodiment the siRNA compound (e.g., the siRNA in a composition described herein) silences the BCR/ABL fusion gene, and thus can be used to treat a subject having or at risk for a disorder characterized by unwanted BCR/ABL fusion gene expression, e.g., acute and chronic leukemias.

In another embodiment the siRNA compound (e.g., the siRNA in a composition described herein) silences the TEL/AML1 fusion gene, and thus can be used to treat a subject having or at risk for a disorder characterized by unwanted TEL/AML1 fusion gene expression, e.g., childhood acute leukemia.

In another embodiment the siRNA compound (e.g., the siRNA in a composition described herein) silences the EWS/FLI1 fusion gene, and thus can be used to treat a subject having or at risk for a disorder characterized by unwanted EWS/FLI1 fusion gene expression, e.g., Ewing Sarcoma.

In another embodiment the siRNA compound (e.g., the siRNA in a composition described herein) silences the TLS/FUS1 fusion gene, and thus can be used to treat a subject having or at risk for a disorder characterized by unwanted TLS/FUS1 fusion gene expression, e.g., Myxoid liposarcoma.

In another embodiment the siRNA compound (e.g., the siRNA in a composition described herein) silences the PAX3/FKHR fusion gene, and thus can be used to treat a subject having or at risk for a disorder characterized by unwanted PAX3/FKHR fusion gene expression, e.g., Myxoid liposarcoma.

In another embodiment the siRNA compound (e.g., the siRNA in a composition described herein) silences the AML1/ETO fusion gene, and thus can be used to treat a subject having or at risk for a disorder characterized by unwanted AML1/ETO fusion gene expression, e.g., acute leukemia.

*Angiogenesis.* In another aspect, described herein is a method of treating a subject, e.g., a human, at risk for or afflicted with a disease or disorder that may benefit by angiogenesis inhibition, e.g., cancer. The method comprises administering the siRNA described herein to a subject in need thereof, thereby treating the subject. The nucleic acid that is administered will depend on the type of angiogenesis-related gene being treated.

In some embodiments the siRNA compound (e.g., the siRNA in a composition described herein) silences the alpha v-integrin gene, and thus can be used to treat a subject having or at risk for a disorder characterized by unwanted alpha V integrin, e.g., brain tumors or tumors of epithelial origin.

In some embodiments the siRNA compound (e.g., the siRNA in a composition described herein) silences the Flt-1 receptor gene, and thus can be used to treat a subject having or at risk for a disorder characterized by unwanted Flt-1 receptors, eg. cancer and rheumatoid arthritis.

In some embodiments the siRNA compound (e.g., the siRNA in a composition described herein) silences the tubulin gene, and thus can be used to treat a subject having or at risk for a disorder characterized by unwanted tubulin, eg. cancer and retinal neovascularization.

In some embodiments the siRNA compound (e.g., the siRNA in a composition described herein) silences the tubulin gene, and thus can be used to treat a subject having or at risk for a disorder characterized by unwanted tubulin, eg. cancer and retinal neovascularization.

*Viral Diseases.* In yet another aspect, described herein is a method of treating a subject infected with a virus or at risk for or afflicted with a disorder or disease associated with a viral infection. The method comprises administering the siRNA described herein to a subject in need thereof, thereby treating the subject. The nucleic acid that is administered will depend on the type of viral disease being treated. In some embodiments, the nucleic acid may target a viral gene. In other embodiments, the nucleic acid may target a host gene.

Thus, described herein is a method of treating patients infected by the Human Papilloma Virus (HPV) or at risk for or afflicted with a disorder mediated by HPV, e.g, cervical cancer. HPV is linked to 95% of cervical carcinomas and thus an antiviral therapy is an attractive method to treat these cancers and other symptoms of viral infection. In some embodiments, the expression of a HPV gene is reduced. In another embodiment, the HPV gene is one of the group of E2, E6, or E7. In some embodiments the expression of a human gene that is required for HPV replication is reduced.

Described herein is also a method of treating patients infected by the Human Immunodeficiency Virus (HIV) or at risk for or afflicted with a disorder mediated by HIV, e.g., Acquired Immune Deficiency Syndrome (AIDS). In some embodiments, the expression of a HIV gene is reduced. In another embodiment, the HIV gene is CCR5, Gag, or Rev. In some embodiments the expression of a human gene that is required for HIV replication is reduced. In another embodiment, the gene is CD4 or Tsg101.

Described herein is also a method for treating patients infected by the Hepatitis B Virus (HBV) or at risk for or afflicted with a disorder mediated by HBV, e.g., cirrhosis and heptocellular carcinoma. In some embodiments, the expression of a HBV gene is reduced. In another embodiment, the targeted HBV gene encodes one of the group of the tail region of the HBV core protein, the pre-cregious (pre-c) region, or the cregious (c) region. In another embodiment, a targeted HBV-RNA sequence is comprised of the poly(A) tail. In certain embodiment the expression of a human gene that is required for HBV replication is reduced.

Described herein is also a method of treating patients infected by the Hepatitis A Virus (HAV), or at risk for or afflicted with a disorder mediated by HAV. In some embodiments the expression of a human gene that is required for HAV replication is reduced.

Described herein is a method of treating patients infected by the Hepatitis C Virus (HCV), or at risk for or afflicted with a disorder mediated by HCV, e.g., cirrhosis. In some embodiments, the expression of a HCV gene is reduced. In another embodiment the expression of a human gene that is required for HCV replication is reduced.

Described herein is also a method of treating patients infected by the any of the group of Hepatitis Viral strains comprising hepatitis D, E, F, G, or H, or patients at risk for or afflicted with a disorder mediated by any of these strains of hepatitis. In some embodiments, the expression of a Hepatitis, D, E, F, G, or H gene is reduced. In another embodiment the expression of a human gene that is required for hepatitis D, E, F, G or H replication is reduced.

Methods described herein also provide for treating patients infected by the Respiratory Syncytial Virus (RSV) or at risk for or afflicted with a disorder mediated by RSV, e.g, lower respiratory tract infection in infants and childhood asthma, pneumonia and other complications, e.g., in the elderly. In some embodiments, the expression of a RSV gene is reduced. In another embodiment, the targeted HBV gene encodes one of the group of genes N, L, or P. In some embodiments the expression of a human gene that is required for RSV replication is reduced.

Methods described herein provide for treating patients infected by the Herpes Simplex Virus (HSV) or at risk for or afflicted with a disorder mediated by HSV, e.g, genital herpes and cold sores as well as life-threatening or sight-impairing disease mainly in immunocompromised patients. In some embodiments, the expression of a HSV gene is reduced. In another embodiment, the targeted HSV gene encodes DNA polymerase or the helicase-primase. In some embodiments the expression of a human gene that is required for HSV replication is reduced.

Described herein is also a method for treating patients infected by the herpes Cytomegalovirus (CMV) or at risk for or afflicted with a disorder mediated by CMV, e.g., congenital virus infections and morbidity in immunocompromised patients. In some embodiments, the expression of a CMV gene is reduced. In some embodiments the expression of a human gene that is required for CMV replication is reduced.

Methods described herein also provide for a method of treating patients infected by the herpes Epstein Barr Virus (EBV) or at risk for or afflicted with a disorder mediated by EBV, e.g., NK/T-cell lymphoma, non-Hodgkin lymphoma, and Hodgkin disease. In some embodiments, the expression of a EBV gene is reduced. In some embodiments the expression of a human gene that is required for EBV replication is reduced.

Methods described herein also provide for treating patients infected by Kaposi's Sarcoma-associated Herpes Virus (KSHV), also called human herpesvirus 8, or patients at risk for or afflicted with a disorder mediated by KSHV, e.g., Kaposi's sarcoma, multicentric Castleman's disease and AIDS-associated primary effusion lymphoma. In some embodiments, the expression of a KSHV gene is reduced. In some embodiments the expression of a human gene that is required for KSHV replication is reduced.

Described herein is also a method for treating patients infected by the JC Virus (JCV) or a disease or disorder associated with this virus, e.g., progressive multifocal leukoencephalopathy (PML). In some embodiments, the expression of a JCV gene is reduced. In certain embodiments the expression of a human gene that is required for JCV replication is reduced.

Methods described herein also provide for treating patients infected by the myxovirus or at risk for or afflicted with a disorder mediated by myxovirus, e.g., influenza. In some embodiments, the expression of a myxovirus gene is reduced. In some embodiments the expression of a human gene that is required for myxovirus replication is reduced.

Methods described herein also provide for treating patients infected by the rhinovirus or at risk for of afflicted with a disorder mediated by rhinovirus, e.g., the common cold. In some embodiments, the expression of a rhinovirus gene is reduced. In certain embodiments the expression of a human gene that is required for rhinovirus replication is reduced.

Methods described herein also provide for treating patients infected by the coronavirus or at risk for of afflicted with a disorder mediated by coronavirus, e.g., the common cold. In some embodiments, the expression of a coronavirus gene is reduced. In certain embodiments the expression of a human gene that is required for coronavirus replication is reduced.

Methods described herein also provide for treating patients infected by the flavivirus West Nile or at risk for or afflicted with a disorder mediated by West Nile Virus. In some embodiments, the expression of a West Nile Virus gene is reduced. In another embodiment, the West Nile Virus gene is E, NS3, or NS5. In some embodiments the expression of a human gene that is required for West Nile Virus replication is reduced.

Methods described herein also provide for treating patients infected by the St. Louis Encephalitis flavivirus, or at risk for or afflicted with a disease or disorder associated with this virus, e.g., viral haemorrhagic fever or neurological disease. In some embodiments, the expression of a St. Louis Encephalitis gene is reduced. In some embodiments the expression of a human gene that is required for St. Louis Encephalitis virus replication is reduced.

Methods described herein also provide for treating patients infected by the Tick-borne encephalitis flavivirus, or at risk for or afflicted with a disorder mediated by Tick-borne encephalitis virus, e.g., viral haemorrhagic fever and neurological disease. In some embodiments, the expression of a Tick-borne encephalitis virus gene is reduced. In some embodiments the expression of a human gene that is required for Tick-borne encephalitis virus replication is reduced.

Methods described herein also provide for methods of treating patients infected by the Murray Valley encephalitis flavivirus, which commonly results in viral haemorrhagic fever and neurological disease. In some embodiments, the expression of a Murray Valley encephalitis virus gene is reduced. In some embodiments the expression of a human gene that is required for Murray Valley encephalitis virus replication is reduced.

Described herein are also methods for treating patients infected by the dengue flavivirus, or a disease or disorder associated with this virus, e.g., dengue haemorrhagic fever. In some embodiments, the expression of a dengue virus gene is reduced. In some embodiments the expression of a human gene that is required for dengue virus replication is reduced.

Methods described herein also provide for treating patients infected by the Simian Virus 40 (SV40) or at risk for or afflicted with a disorder mediated by SV40, e.g., tumorigenesis. In some embodiments, the expression of a SV40 gene is reduced. In some embodiments the expression of a human gene that is required for SV40 replication is reduced.

Described herein are also methods for treating patients infected by the Human T Cell Lymphotropic Virus (HTLV), or a disease or disorder associated with this virus, e.g., leukemia and myelopathy. In some embodiments, the expression of a HTLV gene is reduced. In another embodiment the HTLV1 gene is the Tax transcriptional activator. In some embodiments the expression of a human gene that is required for HTLV replication is reduced.

Methods described herein also provide for treating patients infected by the Moloney-Murine Leukemia Virus (Mo-MuLV) or at risk for or afflicted with a disorder mediated by Mo-MuLV, e.g., T-cell leukemia. In some embodiments, the expression of a Mo-MuLV gene is reduced. In some embodiments the expression of a human gene that is required for Mo-MuLV replication is reduced.

Methods described herein also provide for treating patients infected by the encephalomyocarditis virus (EMCV) or at risk for or afflicted with a disorder mediated by EMCV, e.g., myocarditis. EMCV leads to myocarditis in mice and pigs and is capable of infecting human myocardial cells. This virus is therefore a concern for patients undergoing xenotransplantation. In some embodiments, the expression of a EMCV gene is reduced. In some embodiments the expression of a human gene that is required for EMCV replication is reduced.

Described herein is also a method for treating patients infected by the measles virus (MV) or at risk for or afflicted with a disorder mediated by MV, e.g., measles. In some embodiments, the expression of a MV gene is reduced. In some embodiments the expression of a human gene that is required for MV replication is reduced.

Described herein is also a method for treating patients infected by the Vericella zoster virus (VZV) or at risk for or afflicted with a disorder mediated by VZV, e.g., chicken pox or shingles (also called zoster). In some embodiments, the expression of a VZV gene is reduced. In some embodiments the expression of a human gene that is required for VZV replication is reduced.

Described herein is also a method for treating patients infected by an adenovirus or at risk for or afflicted with a disorder mediated by an adenovirus, e.g., respiratory tract infection. In some embodiments, the expression of an adenovirus gene is reduced. In some embodiments the expression of a human gene that is required for adenovirus replication is reduced.

Described herein is a method for treating patients infected by a yellow fever virus (YFV) or at risk for or afflicted with a disorder mediated by a YFV, e.g., respiratory tract infection. In some embodiments, the expression of a YFV gene is reduced. In another embodiment, the gene may be one of a group that includes the E, NS2A, or NS3 genes. In some embodiments the expression of a human gene that is required for YFV replication is reduced.

Methods described herein also provide for treating patients infected by the poliovirus or at risk for or afflicted with a disorder mediated by poliovirus, e.g., polio. In some embodiments, the expression of a poliovirus gene is reduced. In some embodiments the expression of a human gene that is required for poliovirus replication is reduced.

Methods described herein also provide for treating patients infected by a poxvirus or at risk for or afflicted with a disorder mediated by a poxvirus, e.g., smallpox. In some embodiments, the expression of a poxvirus gene is reduced. In some embodiments the expression of a human gene that is required for poxvirus replication is reduced.

*Other Pathogens.* In another aspect, described herein are methods of treating a subject infected with a pathogen, e.g., a bacterial, amoebic, parasitic, or fungal pathogen. The method comprises administering the siRNA described herein to a subject in need thereof, thereby treating the subject. The nucleic acid that is administered will depend on the type of pathogen being treated. In some embodiments, the nucleic acid may target a pathogen gene. In other embodiments, the nucleic acid may target a host gene.

The target gene can be one involved in growth, cell wall synthesis, protein synthesis, transcription, energy metabolism, e.g., the Krebs cycle, or toxin production.

Thus, described herein is a method of treating patients infected by a plasmodium that causes malaria. In some embodiments, the expression of a plasmodium gene is reduced. In another embodiment, the gene is apical membrane antigen 1 (AMA1). In some embodiments the expression of a human gene that is required for plasmodium replication is reduced.

Described herein are also methods for treating patients infected by the Mycobacterium ulcerans, or a disease or disorder associated with this pathogen, e.g., Buruli ulcers. In some embodiments, the expression of a Mycobacterium ulcerans gene is reduced. In some embodiments the expression of a human gene that is required for Mycobacterium ulcerans replication is reduced.

Described herein are also methods for treating patients infected by the Mycobacterium tuberculosis, or a disease or disorder associated with this pathogen, e.g., tuberculosis. In some embodiments, the expression of a Mycobacterium tuberculosis gene is reduced. In some embodiments the expression of a human gene that is required for Mycobacterium tuberculosis replication is reduced.

Described herein are also methods for treating patients infected by the Mycobacterium leprae, or a disease or disorder associated with this pathogen, e.g., leprosy. In some embodiments, the expression of a Mycobacterium leprae gene is reduced. In some embodiments the expression of a human gene that is required for Mycobacterium leprae replication is reduced.

Described herein are also methods for treating patients infected by the bacteria Staphylococcus aureus, or a disease or disorder associated with this pathogen, e.g., infections of the skin and muscous membranes. In some embodiments, the expression of a Staphylococcus aureus gene is reduced. In some embodiments the expression of a human gene that is required for Staphylococcus aureus replication is reduced.

Described herein are also methods for treating patients infected by the bacteria Streptococcus pneumoniae, or a disease or disorder associated with this pathogen, e.g., pneumonia or childhood lower respiratory tract infection. In some embodiments, the expression of a Streptococcus pneumoniae gene is reduced. In some embodiments the expression of a human gene that is required for Streptococcus pneumoniae replication is reduced.

Described herein are also methods for treating patients infected by the bacteria Streptococcus pyogenes, or a disease or disorder associated with this pathogen, e.g., Strep throat or Scarlet fever. In some embodiments, the expression of a Streptococcus pyogenes gene is reduced. In some embodiments the expression of a human gene that is required for Streptococcus pyogenes replication is reduced.

Described herein are also methods for treating patients infected by the bacteria Chlamydia pneumoniae, or a disease or disorder associated with this pathogen, e.g., pneumonia or childhood lower respiratory tract infection. In some embodiments, the expression of a Chlamydia pneumoniae gene is reduced. In some embodiments the expression of a human gene that is required for Chlamydia pneumoniae replication is reduced.

Described herein are also methods for treating patients infected by the bacteria Mycoplasma pneumoniae, or a disease or disorder associated with this pathogen, e.g., pneumonia or childhood lower respiratory tract infection. In some embodiments, the expression of a Mycoplasma pneumoniae gene is reduced. In some embodiments the expression of a human gene that is required for Mycoplasma pneumoniae replication is reduced.

*Immune Disorders.* In one aspect, described herein is a method of treating a subject, e.g., a human, at risk for or afflicted with a disease or disorder characterized by an unwanted immune response, e.g., an inflammatory disease or disorder, or an autoimmune disease or disorder. The method comprises administering the siRNA described herein to a subject in need thereof, thereby treating the subject. The nucleic acid that is administered will depend on the type of immune disorder being treated.

In some embodiments the disease or disorder is an ischemia or reperfusion injury, e.g., ischemia or reperfusion injury associated with acute myocardial infarction, unstable angina, cardiopulmonary bypass, surgical intervention e.g., angioplasty, e.g., percutaneous transluminal coronary angioplasty, the response to a transplantated organ or tissue, e.g., transplanted cardiac or vascular tissue; or thrombolysis.

In some embodiments the disease or disorder is restenosis, e.g., restenosis associated with surgical intervention e.g., angioplasty, e.g., percutaneous transluminal coronary angioplasty.

In certain embodiments the disease or disorder is Inflammatory Bowel Disease, e.g., Crohn Disease or Ulcerative Colitis.

In certain embodiments the disease or disorder is inflammation associated with an infection or injury.

In certain embodiments the disease or disorder is asthma, lupus, multiple sclerosis, diabetes, e.g., type II diabetes, arthritis, e.g., rheumatoid or psoriatic.

In certain other embodiments, the siRNA compound (e.g., the siRNA in a composition described herein) silences an integrin or co-ligand thereof, e.g., VLA4, VCAM, ICAM.

In certain other embodiments, the siRNA compound (e.g., the siRNA in a composition described herein) silences a selectin or co-ligand thereof, e.g., P-selectin, E-selectin (ELAM), I-selectin, P-selectin glycoprotein-1 (PSGL-1).

In certain other embodiments the siRNA compound (e.g., the siRNA in a composition described herein) silences a component of the complement system, e.g., C3, C5, C3aR, C5aR, C3 convertase, C5 convertase.

In certain other embodiments, the siRNA compound (e.g., the siRNA in a composition described herein) silences a chemokine or receptor thereof, e.g., TNFI, TNFJ, IL-1I, IL-1J, IL - 2, IL-2R, IL-4, IL-4R, IL-5, IL-6, IL-8, TNFRI, TNFRII, IgE, SCYA11, CCR3.

In other embodiments the siRNA compound (e.g., the siRNA in a composition described herein) silences GCSF, Gro1, Gro2, Gro3, PF4, MIG, Pro-Platelet Basic Protein (PPBP), MIP-1I, MIP-1J, RANTES, MCP-1, MCP-2, MCP-3, CMBKR1, CMBKR2, CMBKR3, CMBKR5, AIF-1, I-309.

*Pain.* In one aspect, described herein is a method of treating a subject, e.g., a human, at risk for or afflicted with acute pain or chronic pain. The method comprises administering the siRNA of the invention to a subject in need thereof, thereby treating the subject. The nucleic acid that is administered will depend on the type of pain being treated.

In certain other embodiments, the siRNA compound (e.g., the siRNA in a composition described herein) silences a component of an ion channel.

In certain other embodiments, the siRNA compound (e.g., the siRNA in a composition described herein) silences a neurotransmitter receptor or ligand.

In one aspect, described herein is a method of treating a subject, e.g., a human, at risk for or afflicted with a neurological disease or disorder. The method includes: providing an siRNA compound (e.g., the siRNA in a composition described herein) homologous to and can silence, e.g., by cleavage, a gene which mediates a neurological disease or disorder, and administering the siRNA compound to a subject, thereby treating the subject.

*Necrological Disorders.* In certain embodiments the disease or disorder is a neurological disorder, including Alzheimer's Disease or Parkinson Disease. The method comprises administering the siRNA described herein to a subject in need thereof, thereby treating the subject. The nucleic acid that is administered will depend on the type of neurological disorder being treated.

In certain other embodiments, the siRNA compound (e.g., the siRNA in a composition described herein) silences an amyloid-family gene, e.g., APP; a presenilin gene, e.g., PSEN1 and PSEN2, or I-synuclein.

In some embodiments the disease or disorder is a neurodegenerative trinucleotide repeat disorder, e.g., Huntington disease, dentatorubral pallidoluysian atrophy or a spinocerebellar ataxia, e.g., SCA1, SCA2, SCA3 (Machado-Joseph disease), SCA7 or SCA8.

In certain other embodiments, the siRNA compound (e.g., the siRNA in a composition described herein) silences HD, DRPLA, SCA1, SCA2, MJD1, CACNL1A4, SCA7, SCA8.

*Loss of Heterozygosity.* The loss of heterozygosity (LOH) can result in hemizygosity for sequence, e.g., genes, in the area of LOH. This can result in a significant genetic difference between normal and disease-state cells, e.g., cancer cells, and provides a useful difference between normal and disease-state cells, e.g., cancer cells. This difference can arise because a gene or other sequence is heterozygous in euploid cells but is hemizygous in cells having LOH. The regions of LOH will often include a gene, the loss of which promotes unwanted proliferation, e.g., a tumor suppressor gene, and other sequences including, e.g., other genes, in some cases a gene which is essential for normal function, e.g., growth. Methods described herein rely, in part, on the specific cleavage or silencing of one allele of an essential gene with an siRNA compound (e.g., the siRNA in a composition described herein) described herein. The siRNA compound (e.g., the siRNA in a composition described herein) is selected such that it targets the single allele of the essential gene found in the cells having LOH but does not silence the other allele, which is present in cells which do not show LOH. In essence, it discriminates between the two alleles, preferentially silencing the selected allele. In essence polymorphisms, e.g., SNPs of essential genes that are affected by LOH, are used as a target for a disorder characterized by cells having LOH, e.g., cancer cells having LOH.

One of ordinary skill in the art can identify essential genes which are in proximity to tumor suppressor genes, and which are within a LOH region which includes the tumor suppressor gene. The gene encoding the large subunit of human RNA polymerase II, POLR2A, a gene located in close proximity to the tumor suppressor gene p53, is such a gene. It frequently occurs within a region of LOH in cancer cells. Other genes that occur within LOH regions and are lost in many cancer cell types include the group comprising replication protein A 70-kDa subunit, replication protein A 32-kD, ribonucleotide reductase, thymidilate synthase, TATA associated factor 2H, ribosomal protein S 14, eukaryotic initiation factor 5A, alanyl tRNA synthetase, cysteinyl tRNA synthetase, NaK ATPase, alpha-1 subunit, and transferrin receptor.

Accordingly, described herein is a method of treating a disorder characterized by LOH, e.g., cancer. The method comprises optionally, determining the genotype of the allele of a gene in the region of LOH and determining the genotype of both alleles of the gene in a normal cell; providing an siRNA compound (e.g., the siRNA in a composition described herein) which preferentially cleaves or silences the allele found in the LOH cells; and administering the iRNA to the subject, thereby treating the disorder.

Described herein is also a siRNA compound (e.g., the siRNA in a composition described herein) disclosed herein, e.g, an siRNA compound (e.g., the siRNA in a composition described herein) which can preferentially silence, e.g., cleave, one allele of a polymorphic gene.

In another aspect, described herein is a method of cleaving or silencing more than one gene with an siRNA compound (e.g., the siRNA in a composition described herein). In these embodiments the siRNA compound (e.g., the siRNA in a composition described herein) is elected so that it has sufficient homology to a sequence found in more than one gene. For example, the sequence AAGCTGGCCCTGGACATGGAGAT (SEQ ID NO: 13) is conserved between mouse lamin B1, lamin B2, keratin complex 2-gene 1 and lamin A/C. Thus an siRNA compound (e.g., the siRNA in a composition described herein) targeted to this sequence would effectively silence the entire collection of genes.

Described herein is also an siRNA compound (e.g., the siRNA in a composition described herein) disclosed herein, which can silence more than one gene.

### Routes of Delivery

For ease of exposition the formulations, compositions and methods in this section are discussed largely with regard to modified siRNA compounds. It may be understood, however, that these formulations, compositions and methods can be practiced with other siRNA compounds, *e.g.,* unmodified siRNA compounds, and such practice is described herein. A composition that includes a iRNA can be delivered to a subject by a variety of routes. Exemplary routes include: intravenous, topical, rectal, anal, vaginal, nasal, pulmonary, ocular.

The iRNA molecules described herein can be incorporated into pharmaceutical compositions suitable for administration. Such compositions typically include one or more species of iRNA and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The pharmaceutical compositions described herein may be administered in a number of ways depending upon whether local or systemic treatment is desired and upon the area to be treated. Administration may be topical (including ophthalmic, vaginal, rectal, intranasal, transdermal), oral or parenteral. Parenteral administration includes intravenous drip, subcutaneous, intraperitoneal or intramuscular injection, or intrathecal or intraventricular administration.

The route and site of administration may be chosen to enhance targeting. For example, to target muscle cells, intramuscular injection into the muscles of interest would be a logical choice. Lung cells might be targeted by administering the iRNA in aerosol form. The vascular endothelial cells could be targeted by coating a balloon catheter with the iRNA and mechanically introducing the DNA.

Formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Compositions for oral administration include powders or granules, suspensions or solutions in water, syrups, elixirs or non-aqueous media, tablets, capsules, lozenges, or troches. In the case of tablets, carriers that can be used include lactose, sodium citrate and salts of phosphoric acid. Various disintegrants such as starch, and lubricating agents such as magnesium stearate, sodium lauryl sulfate and talc, are commonly used in tablets. For oral administration in capsule form, useful diluents are lactose and high molecular weight polyethylene glycols. When aqueous suspensions are required for oral use, the nucleic acid compositions can be combined with emulsifying and suspending agents. If desired, certain sweetening and/or flavoring agents can be added.

Compositions for intrathecal or intraventricular administration may include sterile aqueous solutions which may also contain buffers, diluents and other suitable additives.

Formulations for parenteral administration may include sterile aqueous solutions which may also contain buffers, diluents and other suitable additives. Intraventricular injection may be facilitated by an intraventricular catheter, for example, attached to a reservoir. For intravenous use, the total concentration of solutes may be controlled to render the preparation isotonic.

For ocular administration, ointments or droppable liquids may be delivered by ocular delivery systems known to the art such as applicators or eye droppers. Such compositions can include mucomimetics such as hyaluronic acid, chondroitin sulfate, hydroxypropyl methylcellulose or poly(vinyl alcohol), preservatives such as sorbic acid, EDTA or benzylchronium chloride, and the usual quantities of diluents and/or carriers.

In one embodiment, the administration of the siRNA compound, *e.g.,* a double-stranded siRNA compound, or ssiRNA compound, composition is parenteral, *e.g.,* intravenous (*e.g.,* as a bolus or as a diffusible infusion), intradermal intraperitoneal, intramuscular, intrathecal, intraventricular, intracranial, subcutaneous, transmucosal, buccal, sublingual, endoscopic, rectal, oral, vaginal, topical, pulmonary, intranasal, urethral or ocular. Administration can be provided by the subject or by another person, *e.g.,* a health care provider. The medication can be provided in measured doses or in a dispenser which delivers a metered dose. Selected modes of delivery are discussed in more detail below.

*Rectal Administration.* Described herein are also methods, compositions, and kits, for rectal administration or delivery of siRNA compounds described herein.

Accordingly, an siRNA compound, *e.g.,* a double-stranded siRNA compound, or ssiRNA compound, (*e.g.,* a precursor, *e.g.,* a larger siRNA compound which can be processed into a ssiRNA compound , or a DNA which encodes a an siRNA compound, *e.g.,* a double-stranded siRNA compound, or ssiRNA compound, or precursor thereof) described herein, *e.g.,* a therapeutically effective amount of a siRNA compound described herein, *e.g.,* a siRNA compound having a double stranded region of less than 40, and, for example, less than 30 nucleotides and having one or two 1-3 nucleotide single strand 3' overhangs can be administered rectally, *e.g.,* introduced through the rectum into the lower or upper colon. This approach is particularly useful in the treatment of, inflammatory disorders, disorders characterized by unwanted cell proliferation, *e.g.,* polyps, or colon cancer.

The medication can be delivered to a site in the colon by introducing a dispensing device, *e.g.,* a flexible, camera-guided device similar to that used for inspection of the colon or removal of polyps, which includes means for delivery of the medication.

The rectal administration of the siRNA compound is by means of an enema. The siRNA compound of the enema can be dissolved in a saline or buffered solution. The rectal administration can also by means of a suppository, which can include other ingredients, e.g., an excipient, e.g., cocoa butter or hydropropylmethylcellulose.

*Ocular Delivery.* Any of the siRNA compounds described herein can be administered to ocular tissue. For example, the medications can be applied to the surface of the eye or nearby tissue, *e.g.,* the inside of the eyelid. They can be applied topically, *e.g.,* by spraying, in drops, as an eyewash, or an ointment. Administration can be provided by the subject or by another person, *e.g.,* a health care provider. The medication can be provided in measured doses or in a dispenser which delivers a metered dose. The medication can also be administered to the interior of the eye, and can be introduced by a needle or other delivery device which can introduce it to a selected area or structure. Ocular treatment is particularly desirable for treating inflammation of the eye or nearby tissue.

*Topical Delivery.* Any of the siRNA compounds described herein can be administered directly to the skin. For example, the medication can be applied topically or delivered in a layer of the skin, *e.g.,* by the use of a microneedle or a battery of microneedles which penetrate into the skin, but, for example, not into the underlying muscle tissue. Administration of the siRNA compound composition can be topical. Topical applications can, for example, deliver the composition to the dermis or epidermis of a subject. Topical administration can be in the form of transdermal patches, ointments, lotions, creams, gels, drops, suppositories, sprays, liquids or powders. A composition for topical administration can be formulated as a liposome, micelle, emulsion, or other lipophilic molecular assembly. The transdermal administration can be applied with at least one penetration enhancer, such as iontophoresis, phonophoresis, and sonophoresis.

For ease of exposition the formulations, compositions and methods in this section are discussed largely with regard to modified siRNA compounds. It may be understood, however, that these formulations, compositions and methods can be practiced with other siRNA compounds, *e.g.,* unmodified siRNA compounds, and such practice is described herein. In some embodiments, an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) is delivered to a subject via topical administration. "Topical administration" refers to the delivery to a subject by contacting the formulation directly to a surface of the subject. The most common form of topical delivery is to the skin, but a composition disclosed herein can also be directly applied to other surfaces of the body, *e.g.,* to the eye, a mucous membrane, to surfaces of a body cavity or to an internal surface. As mentioned above, the most common topical delivery is to the skin. The term encompasses several routes of administration including, but not limited to, topical and transdermal. These modes of administration typically include penetration of the skin's permeability barrier and efficient delivery to the target tissue or stratum. Topical administration can be used as a means to penetrate the epidermis and dermis and ultimately achieve systemic delivery of the composition. Topical administration can also be used as a means to selectively deliver oligonucleotides to the epidermis or dermis of a subject, or to specific strata thereof, or to an underlying tissue.

The term "skin," as used herein, refers to the epidermis and/or dermis of an animal. Mammalian skin consists of two major, distinct layers. The outer layer of the skin is called the epidermis. The epidermis is comprised of the stratum corneum, the stratum granulosum, the stratum spinosum, and the stratum basale, with the stratum corneum being at the surface of the skin and the stratum basale being the deepest portion of the epidermis. The epidermis is between 50 µm and 0.2 mm thick, depending on its location on the body.

Beneath the epidermis is the dermis, which is significantly thicker than the epidermis. The dermis is primarily composed of collagen in the form of fibrous bundles. The collagenous bundles provide support for, inter alia, blood vessels, lymph capillaries, glands, nerve endings and immunologically active cells.

One of the major functions of the skin as an organ is to regulate the entry of substances into the body. The principal permeability barrier of the skin is provided by the stratum comeum, which is formed from many layers of cells in various states of differentiation. The spaces between cells in the stratum corneum is filled with different lipids arranged in lattice-like formations that provide seals to further enhance the skins permeability barrier.

The permeability barrier provided by the skin is such that it is largely impermeable to molecules having molecular weight greater than about 750 Da. For larger molecules to cross the skin's permeability barrier, mechanisms other than normal osmosis must be used.

Several factors determine the permeability of the skin to administered agents. These factors include the characteristics of the treated skin, the characteristics of the delivery agent, interactions between both the drug and delivery agent and the drug and skin, the dosage of the drug applied, the form of treatment, and the post treatment regimen. To selectively target the epidermis and dermis, it is sometimes possible to formulate a composition that comprises one or more penetration enhancers that will enable penetration of the drug to a preselected stratum.

Transdermal delivery is a valuable route for the administration of lipid soluble therapeutics. The dermis is more permeable than the epidermis and therefore absorption is much more rapid through abraded, burned or denuded skin. Inflammation and other physiologic conditions that increase blood flow to the skin also enhance transdermal adsorption. Absorption via this route may be enhanced by the use of an oily vehicle (inunction) or through the use of one or more penetration enhancers. Other effective ways to deliver a composition disclosed herein via the transdermal route include hydration of the skin and the use of controlled release topical patches. The transdermal route provides a potentially effective means to deliver a composition disclosed herein for systemic and/or local therapy.

In addition, iontophoresis (transfer of ionic solutes through biological membranes under the influence of an electric field) (Lee et al., Critical Reviews in Therapeutic Drug Carrier Systems, 1991, p. 163), phonophoresis or sonophoresis (use of ultrasound to enhance the absorption of various therapeutic agents across biological membranes, notably the skin and the cornea) (Lee et al., Critical Reviews in Therapeutic Drug Carrier Systems, 1991, p. 166), and optimization of vehicle characteristics relative to dose position and retention at the site of administration (Lee et al., Critical Reviews in Therapeutic Drug Carrier Systems, 1991, p. 168) may be useful methods for enhancing the transport of topically applied compositions across skin and mucosa sites.

The compositions and methods provided may also be used to examine the function of various proteins and genes *in vitro* in cultured or preserved dermal tissues and in animals. The subject matter described herein can be thus applied to examine the function of any gene. The methods described herein can also be used therapeutically or prophylactically. For example, for the treatment of animals that are known or suspected to suffer from diseases such as psoriasis, lichen planus, toxic epidermal necrolysis, ertythema multiforme, basal cell carcinoma, squamous cell carcinoma, malignant melanoma, Paget's disease, Kaposi's sarcoma, pulmonary fibrosis, Lyme disease and viral, fungal and bacterial infections of the skin.

*Pulmoncuy Delivery.* Any of the siRNA compounds described herein can be administered to the pulmonary system. Pulmonary administration can be achieved by inhalation or by the introduction of a delivery device into the pulmonary system, *e.g.,* by introducing a delivery device which can dispense the medication. Certain embodiments may use a method of pulmonary delivery by inhalation. The medication can be provided in a dispenser which delivers the medication, *e.g.,* wet or dry, in a form sufficiently small such that it can be inhaled. The device can deliver a metered dose of medication. The subject, or another person, can administer the medication. Pulmonary delivery is effective not only for disorders which directly affect pulmonary tissue, but also for disorders which affect other tissue. siRNA compounds can be formulated as a liquid or nonliquid, *e.g.,* a powder, crystal, or aerosol for pulmonary delivery.

For ease of exposition the formulations, compositions and methods in this section are discussed largely with regard to modified siRNA compounds. It may be understood, however, that these formulations, compositions and methods can be practiced with other siRNA compounds, *e.g.,* unmodified siRNA compounds, and such practice is described herein. A composition that includes an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof) can be administered to a subject by pulmonary delivery. Pulmonary delivery compositions can be delivered by inhalation by the patient of a dispersion so that the composition, for example, iRNA, within the dispersion can reach the lung where it can be readily absorbed through the alveolar region directly into blood circulation. Pulmonary delivery can be effective both for systemic delivery and for localized delivery to treat diseases of the lungs.

Pulmonary delivery can be achieved by different approaches, including the use of nebulized, aerosolized, micellular and dry powder-based formulations. Delivery can be achieved with liquid nebulizers, aerosol-based inhalers, and dry powder dispersion devices. Metered-dose devices are may be used. One of the benefits of using an atomizer or inhaler is that the potential for contamination is minimized because the devices are self contained. Dry powder dispersion devices, for example, deliver drugs that may be readily formulated as dry powders. A iRNA composition may be stably stored as lyophilized or spray-dried powders by itself or in combination with suitable powder carriers. The delivery of a composition for inhalation can be mediated by a dosing timing element which can include a timer, a dose counter, time measuring device, or a time indicator which when incorporated into the device enables dose tracking, compliance monitoring, and/or dose triggering to a patient during administration of the aerosol medicament.

The term "powder" means a composition that consists of finely dispersed solid particles that are free flowing and capable of being readily dispersed in an inhalation device and subsequently inhaled by a subject so that the particles reach the lungs to permit penetration into the alveoli. Thus, the powder is said to be "respirable." For example, the average particle size is less than about 10 µm in diameter with a relatively uniform spheroidal shape distribution. In some embodiments, the diameter is less than about 7.5 µm and in some embodiments less than about 5.0 µm. Usually the particle size distribution is between about 0.1 µm and about 5 µm in diameter, sometimes about 0.3 µm to about 5 µm.

The term "dry" means that the composition has a moisture content below about 10% by weight (% w) water, usually below about 5% w and in some cases less it than about 3% w. A dry composition can be such that the particles are readily dispersible in an inhalation device to form an aerosol.

The term "therapeutically effective amount" is the amount present in the composition that is needed to provide the desired level of drug in the subject to be treated to give the anticipated physiological response.

The term "physiologically effective amount" is that amount delivered to a subject to give the desired palliative or curative effect.

The term "pharmaceutically acceptable carriers" means that the carrier can be taken into the lungs with no significant adverse toxicological effects on the lungs.

The types of pharmaceutical excipients that are useful as carrier include stabilizers such as human serum albumin (HSA), bulking agents such as carbohydrates, amino acids and polypeptides; pH adjusters or buffers; salts such as sodium chloride; and the like. These carriers may be in a crystalline or amorphous form or may be a mixture of the two.

Bulking agents that are particularly valuable include compatible carbohydrates, polypeptides, amino acids or combinations thereof. Suitable carbohydrates include monosaccharides such as galactose, D-mannose, sorbose, and the like; disaccharides, such as lactose, trehalose, and the like; cyclodextrins, such as 2-hydroxypropyl-.beta.-cyclodextrin; and polysaccharides, such as raffinose, maltodextrins, dextrans, and the like; alditols, such as mannitol, xylitol, and the like. A group of carbohydrates, may include lactose, threhalose, raffinose maltodextrins, and mannitol. Suitable polypeptides include aspartame. Amino acids include alanine and glycine, with glycine being used in some embodiments.

Additives, which are minor components of the composition described herein, may be included for conformational stability during spray drying and for improving dispersibility of the powder. These additives include hydrophobic amino acids such as tryptophan, tyrosine, leucine, phenylalanine, and the like.

Suitable pH adjusters or buffers include organic salts prepared from organic acids and bases, such as sodium citrate, sodium ascorbate, and the like; sodium citrate may be used in some embodiments.

Pulmonary administration of a micellar iRNA formulation may be achieved through metered dose spray devices with propellants such as tetrafluoroethane, heptafluoroethane, dimethylfluoropropane, tetrafluoropropane, butane, isobutane, dimethyl ether and other non-CFC and CFC propellants.

*Oral or Nasal Delivery.* Any of the siRNA compounds described herein can be administered orally, e.g., in the form of tablets, capsules, gel capsules, lozenges, troches or liquid syrups. Further, the composition can be applied topically to a surface of the oral cavity.

Any of the siRNA compounds described herein can be administered nasally. Nasal administration can be achieved by introduction of a delivery device into the nose, *e.g.,* by introducing a delivery device which can dispense the medication. Methods of nasal delivery include spray, aerosol, liquid, *e.g.,* by drops, or by topical administration to a surface of the nasal cavity. The medication can be provided in a dispenser with delivery of the medication, *e.g.,* wet or dry, in a form sufficiently small such that it can be inhaled. The device can deliver a metered dose of medication. The subject, or another person, can administer the medication.

Nasal delivery is effective not only for disorders which directly affect nasal tissue, but also for disorders which affect other tissue siRNA compounds can be formulated as a liquid or nonliquid, *e.g.,* a powder, crystal, or for nasal delivery. As used herein, the term "crystalline'' describes a solid having the structure or characteristics of a crystal, *i.e.,* particles of three-dimensional structure in which the plane faces intersect at definite angles and in which there is a regular internal structure. The compositions described herein may have different crystalline forms. Crystalline forms can be prepared by a variety of methods, including, for example, spray drying.

For ease of exposition the formulations, compositions and methods in this section are discussed largely with regard to modified siRNA compounds. It may be understood, however, that these formulations, compositions and methods can be practiced with other siRNA compounds, *e.g.,* unmodified siRNA compounds, and such practice is described herein. Both the oral and nasal membranes offer advantages over other routes of administration. For example, drugs administered through these membranes have a rapid onset of action, provide therapeutic plasma levels, avoid first pass effect of hepatic metabolism, and avoid exposure of the drug to the hostile gastrointestinal (GI) environment. Additional advantages include easy access to the membrane sites so that the drug can be applied, localized and removed easily.

In oral delivery, compositions can be targeted to a surface of the oral cavity, *e.g.,* to sublingual mucosa which includes the membrane of ventral surface of the tongue and the floor of the mouth or the buccal mucosa which constitutes the lining of the cheek. The sublingual mucosa is relatively permeable thus giving rapid absorption and acceptable bioavailability of many drugs. Further, the sublingual mucosa is convenient, acceptable and easily accessible.

The ability of molecules to permeate through the oral mucosa appears to be related to molecular size, lipid solubility and peptide protein ionization. Small molecules, less than 1000 daltons appear to cross mucosa rapidly. As molecular size increases, the permeability decreases rapidly. Lipid soluble compounds are more permeable than non-lipid soluble molecules. Maximum absorption occurs when molecules are un-ionized or neutral in electrical charges. Therefore charged molecules present the biggest challenges to absorption through the oral mucosae.

A pharmaceutical composition of iRNA may also be administered to the buccal cavity of a human being by spraying into the cavity, without inhalation, from a metered dose spray dispenser, a mixed micellar pharmaceutical formulation as described above and a propellant. In one embodiment, the dispenser is first shaken prior to spraying the pharmaceutical formulation and propellant into the buccal cavity. For example, the medication can be sprayed into the buccal cavity or applied directly, *e.g.,* in a liquid, solid, or gel form to a surface in the buccal cavity. This administration is particularly desirable for the treatment of inflammations of the buccal cavity, *e.g.,* the gums or tongue, *e.g.,* in one embodiment, the buccal administration is by spraying into the cavity, *e.g.,* without inhalation, from a dispenser, *e.g.,* a metered dose spray dispenser that dispenses the pharmaceutical composition and a propellant.

### Devices

In another aspect, described herein is a device, *e.g.,* an implantable device, wherein the device can dispense or administer a composition that includes an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof), *e.g.,* a siRNA compound that silences an endogenous transcript. In one embodiment, the device is coated with the composition. In another embodiment the siRNA compound is disposed within the device. In another embodiment, the device includes a mechanism to dispense a unit dose of the composition. In other embodiments the device releases the composition continuously, *e.g.,* by diffusion. Exemplary devices include stents, catheters, pumps, artificial organs or organ components (*e.g.,* artificial heart, a heart valve, etc.), and sutures.

For ease of exposition the devices, formulations, compositions and methods in this section are discussed largely with regard to modified siRNA compounds. It may be understood, however, that these devices, formulations, compositions and methods can be practiced with other siRNA compounds, *e.g.,* unmodified siRNA compounds, and such practice is described herein. An siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-strarided siRNA compound, or ssiRNA compound, or precursor thereof) can be disposed on or in a device, *e.g.,* a device which implanted or otherwise placed in a subject. Exemplary devices include devices which are introduced into the vasculature, *e.g.,* devices inserted into the lumen of a vascular tissue, or which devices themselves form a part of the vasculature, including stents, catheters, heart valves, and other vascular devices. These devices, *e.g.,* catheters or stents, can be placed in the vasculature of the lung, heart, or leg.

Other devices include non-vascular devices, *e.g.,* devices implanted in the peritoneum, or in organ or glandular tissue, *e.g.,* artificial organs. The device can release a therapeutic substance in addition to an siRNA, *e.g.,* a device can release insulin.

Other devices include artificial joints, *e.g.,* hip joints, and other orthopedic implants.

In one embodiment, unit doses or measured doses of a composition that includes iRNA are dispensed by an implanted device. The device can include a sensor that monitors a parameter within a subject. For example, the device can include pump, *e.g.,* and, optionally, associated electronics.

Tissue, *e.g.,* cells or organs can be treated with an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof), ex vivo and then administered or implanted in a subject.

The tissue can be autologous, allogeneic, or xenogeneic tissue. *E.g.*, tissue can be treated to reduce graft versus host disease. In other embodiments, the tissue is allogeneic and the tissue is treated to treat a disorder characterized by unwanted gene expression in that tissue. *E.g.,* tissue, *e.g.,* hematopoietic cells, *e.g.,* bone marrow hematopoietic cells, can be treated to inhibit unwanted cell proliferation.

Introduction of treated tissue, whether autologous or transplant, can be combined with other therapies.

In some implementations, the iRNA treated cells are insulated from other cells, *e.g.,* by a semi-permeable porous barrier that prevents the cells from leaving the implant, but enables molecules from the body to reach the cells and molecules produced by the cells to enter the body. In one embodiment, the porous barrier is formed from alginate.

In one embodiment, a contraceptive device is coated with or contains an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof). Exemplary devices include condoms, diaphragms, IUD (implantable uterine devices, sponges, vaginal sheaths, and birth control devices. In one embodiment, the iRNA is chosen to inactive sperm or egg. In another embodiment, the iRNA is chosen to be complementary to a viral or pathogen RNA, *e.g.,* an RNA of an STD. In some instances, the iRNA composition can include a spermicide.

### Dosage

The dosage of a pharmaceutical composition including a siRNA compound can be administered in order to alleviate the symptoms of a disease state, *e.g.,* cancer or a cardiovascular disease. A subject can be treated with the pharmaceutical composition by any of the methods mentioned above.

In one aspect, described herein is a method of administering an siRNA compound, *e.g.,* a double-stranded siRNA compound, or ssiRNA compound, to a subject (*e.g.,* a human subject). The method includes administering a unit dose of the siRNA compound, *e.g.,* a ssiRNA compound, *e.g.,* double stranded ssiRNA compound that (a) the double-stranded part is 19-25 nucleotides (nt) long, for example, 21-23 nt, (b) is complementary to a target RNA (*e.g.,* an endogenous or pathogen target RNA), and, optionally, (c) includes at least one 3' overhang 1-5 nucleotide long. In one embodiment, the unit dose is less than 1.4 mg per kg of bodyweight, or less than 10, 5, 2, 1, 0.5, 0.1, 0.05, 0.01, 0.005, 0.001, 0.0005, 0.0001, 0.00005 or 0.00001 mg per kg of bodyweight, and less than 200 nmole of RNA agent (*e.g.,* about 4.4 x 10¹⁶ copies) per kg of bodyweight, or less than 1500, 750, 300, 150, 75, 15, 7.5, 1.5, 0.75, 0.15, 0.075, 0.015, 0.0075, 0.0015, 0.00075, 0.00015 nmole of RNA agent per kg of bodyweight.

The defined amount can be an amount effective to treat or prevent a disease or disorder, *e.g.,* a disease or disorder associated with the target RNA. The unit dose, for example, can be administered by injection (*e.g.,* intravenous or intramuscular), an inhaled dose, or a topical application. In some embodiments dosages may be less than 2, 1, or 0.1 mg/kg of body weight.

In some embodiments, the unit dose is administered less frequently than once a day, *e.g.,* less than every 2, 4, 8 or 30 days. In another embodiment, the unit dose is not administered with a frequency (*e.g.,* not a regular frequency). For example, the unit dose may be administered a single time.

RNAi silencing persists for several days after administering an siRNA or siNA composition so, in many instances, it is possible to administer the composition with a frequency of less than once per day, or, for some instances, only once for the entire therapeutic regimen. For example, treatment of some cancer cells may be mediated by a single bolus administration, whereas a chronic viral infection may require regular administration, *e.g.,* once or more per week or once or less per month.

In one embodiment, the effective dose is administered with other traditional therapeutic modalities. In one embodiment, the subject has a viral infection and the modality is an antiviral agent other than an siRNA compound, *e.g.,* other than a double-stranded siRNA compound, or ssiRNA compound. In another embodiment, the subject has atherosclerosis and the effective dose of an siRNA compound, *e.g.,* a double-stranded siRNA compound, or ssiRNA compound, is administered in combination with, *e.g.,* after surgical intervention, *e.g.,* angioplasty.

In one embodiment, a subject is administered an initial dose and one or more maintenance doses of an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof). The maintenance dose or doses are generally lower than the initial dose, *e.g.,* one-half less of the initial dose. A maintenance regimen can include treating the subject with a dose or doses ranging from 0.01 µg to 1.4 mg/kg of body weight per day, *e.g*., 10, 1, 0.1, 0.01, 0.001, or 0.00001 mg per kg of bodyweight per day. The maintenance doses are, for example, administered no more than once every 5, 10, or 30 days. Further, the treatment regimen may last for a period of time which will vary depending upon the nature of the particular disease, its severity and the overall condition of the patient. In certain embodiments the dosage may be delivered no more than once per day, e.g., no more than once per 24, 36, 48, or more hours, e.g., no more than once for every 5 or 8 days. Following treatment, the patient can be monitored for changes in his condition and for alleviation of the symptoms of the disease state. The dosage of the compound may either be increased in the event the patient does not respond significantly to current dosage levels, or the dose may be decreased if an alleviation of the symptoms of the disease state is observed, if the disease state has been ablated, or if undesired side-effects are observed.

The effective dose can be administered in a single dose or in two or more doses, as desired or considered appropriate under the specific circumstances. If desired to facilitate repeated or frequent infusions, implantation of a delivery device, e.g., a pump, semi-permanent stent (e.g., intravenous, intraperitoneal, intracisternal or intracapsular), or reservoir may be advisable.

In one embodiment, the siRNA compound pharmaceutical composition includes a plurality of siRNA compound species. In another embodiment, the siRNA compound species has sequences that are non-overlapping and non-adjacent to another species with respect to a naturally occurring target sequence. In another embodiment, the plurality of siRNA compound species is specific for different naturally occurring target genes. In another embodiment, the siRNA compound is allele specific.

In some cases, a patient is treated with a siRNA compound in conjunction with other therapeutic modalities. For example, a patient being treated for a viral disease, e.g., an HIV associated disease (e.g., AIDS), may be administered a siRNA compound specific for a target gene essential to the virus in conjunction with a known antiviral agent (e.g., a protease inhibitor or reverse transcriptase inhibitor). In another example, a patient being treated for cancer may be administered a siRNA compound specific for a target essential for tumor cell proliferation in conjunction with a chemotherapy.

Following successful treatment, it may be desirable to have the patient undergo maintenance therapy to prevent the recurrence of the disease state, wherein the compound described herein is administered in maintenance doses, ranging from 0.01 µg to 100 g per kg of body weight (see US 6,107,094).

The concentration of the siRNA compound composition is an amount sufficient to be effective in treating or preventing a disorder or to regulate a physiological condition in humans. The concentration or amount of siRNA compound administered will depend on the parameters determined for the agent and the method of administration, e.g., nasal, buccal, pulmonary. For example, nasal formulations tend to require much lower concentrations of some ingredients in order to avoid irritation or burning of the nasal passages. It is sometimes desirable to dilute an oral formulation up to 10-100 times in order to provide a suitable nasal formulation.

Certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an siRNA compound, e.g., a double-stranded siRNA compound, or ssiRNA compound, (e.g., a precursor, e.g., a larger siRNA compound which can be processed into a ssiRNA compound, or a DNA which encodes an siRNA compound, e.g., a double-stranded siRNA compound, or ssiRNA compound, or precursor thereof) can include a single treatment or, for example, can include a series of treatments. It will also be appreciated that the effective dosage of a siRNA compound such as a ssiRNA compound used for treatment may increase or decrease over the course of a particular treatment. Changes in dosage may result and become apparent from the results of diagnostic assays as described herein. For example, the subject can be monitored after administering a siRNA compound composition. Based on information from the monitoring, an additional amount of the siRNA compound composition can be administered.

Dosing is dependent on severity and responsiveness of the disease condition to be treated, with the course of treatment lasting from several days to several months, or until a cure is effected or a diminution of disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual compounds, and can generally be estimated based on EC50s found to be effective in in vitro and in vivo animal models. In some embodiments, the animal models include transgenic animals that express a human gene, e.g., a gene that produces a target RNA. The transgenic animal can be deficient for the corresponding endogenous RNA. In another embodiment, the composition for testing includes a siRNA compound that is complementary, at least in an internal region, to a sequence that is conserved between the target RNA in the animal model and the target RNA in a human.

The inventors have discovered that siRNA compounds described herein can be administered to mammals, particularly large mammals such as nonhuman primates or humans in a number of ways.

### Kits

In certain other aspects, described herein are kits that include a suitable container containing a pharmaceutical formulation of an siRNA compound, *e.g.,* a double-stranded siRNA compound, or ssiRNA compound, (*e.g.,* a precursor, *e.g.,* a larger siRNA compound which can be processed into a ssiRNA compound, or a DNA which encodes an siRNA compound, *e.g.,* a double-stranded siRNA compound, or ssiRNA compound, or precursor thereof). In certain embodiments the individual components of the pharmaceutical formulation may be provided in one container. Alternatively, it may be desirable to provide the components of the pharmaceutical formulation separately in two or more containers, *e.g.,* one container for an siRNA compound preparation, and at least another for a carrier compound. The kit may be packaged in a number of different configurations such as one or more containers in a single box. The different components can be combined, *e.g.,* according to instructions provided with the kit. The components can be combined according to a method described herein, *e.g.,* to prepare and administer a pharmaceutical composition. The kit can also include a delivery device.

The invention is further illustrated by the following examples, which should not be construed as further limiting.

### EXEMPLIFICATION

The invention now being generally described, it will be more readily understood by reference to the following examples which are included merely for purposes of illustration of certain aspects and embodiments of the present invention, and are not intended to limit the invention.

### Example 1 - siRNAs targeting firefly luciferase and containing mismatch base pairings in the sense strand. (Reference Example)

Double stranded siRNA agents containing mismatch base pairings (also termed "duplexes" herein) and a control, unmodified siRNA sequence (Duplex ID 1000) without modification are provided below in Table 1. In the following tables, strand S corresponds to the sense strand, and strand AS corresponds to the antisense strand.

**Table 1. siRNA duplexes targeting firefly luciferase and containing mismatch base parings in positions 9-12 in the sense strand.**

| **Duplex ID** | **Strand** | **Sequence 5' to 3'** | **Modifications** | **SEQ ID NO.** |
|---|---|---|---|---|
| AD-3224 | S | CUU ACG CUA AGU ACU UCG AdTdT UCG AAG UAC UCA GCG UAA GdTdT | G9->A | 14 |
| | AS | | none | 15 |
| AD-3225 | S | CUU ACG CUC AGU ACU UCG AdTdT UCG AAG UAC UCA GCG UAA GdTdT | G9 -> C | 16 |
| | AS | | none | 15 |
| AD-3226 | S | CUU ACG CUU AGU ACU UCG AdTdT UCG AAG UAC UCA GCG UAA GdTdT | G9 -> U | 17 |
| | AS | | none | 15 |
| AD-3227 | S | CUU ACG CUG GGU ACU UCG AdTdT UCG AAG UAC UCA GCG UAA GdTdT | A10 -> G | 18 |
| | AS | | none | 15 |
| AD-3228 | S | CUU ACG CUG CGU ACU UCG AdTdT UCG AAG UAC UCA GCG UAA GdTdT | A10 -> C | 19 |
| | AS | | none | 15 |
| AD-3229 | S | CUU ACG CUG UGU ACU UCG AdTdT UCG AAG UAC UCA GCG UAA GdTdT | A10 -> U | 20 |
| | AS | | none | 15 |
| AD-3230 | S | CUU ACG CUG AAU ACU UCG AdTdT UCG AAG UAC UCA GCG UAA GdTdT | G11 -> A | 21 |
| | AS | | none | 15 |
| AD-3231 | S | CUU ACG CUG ACU ACU UCG AdTdT UCG AAG UAC UCA GCG UAA GdTdT | G11 -> C | 22 |
| | AS | | none | 15 |
| AD-3232 | S | CUU ACG CUG AUU ACU UCG AdTdT UCG AAG UAC UCA GCG UAA GdTdT | G11 -> U | 23 |
| | AS | | none | 15 |
| AD-3233 | S | CUU ACG CUG AGA ACU UCG AdTdT UCG AAG UAC UCA GCG UAA GdTdT | U12 -> A | 24 |
| | AS | | none | 15 |
| AD-3234 | S | CUU ACG CUG AGG ACU UCG AdTdT UCG AAG UAC UCA GCG UAA GdTdT | U12 -> G | 25 |
| | AS | | none | 15 |
| AD-3235 | S | CUU ACG CUG AGC ACU UCG AdTdT UCG AAG UAC UCA GCG UAA GdTdT | U12 -> C | 26 |
| | AS | | none | 15 |

| | | | | |
|---|---|---|---|---|
| *Mismatches are shown in bold.* | | | | |

**Table 2. siRNA duplexes targeting firefly luciferase and containing mismatch base parings in positions 1-8 and 13-19 in the sense strand.**

| **Duplex ID** | **Strand** | **Sequence 5' to 3'** | **Modification** | **SEQ ID NO.** |
|---|---|---|---|---|
| AD-15959 | S | AUU ACG CUG AGU ACU UCG AdTdT UCG AAG UAC UCA GCG UAA GdTdT | C1 -> A | 27 |
| | AS | | none | 15 |
| AD-15960 | S | GUU ACG CUG AGU ACU UCG AdTdT UCG AAG UAC UCA GCG UAA GdTdT | C1 -> G | 28 |
| | AS | | none | 15 |
| AD-1.5961 | S | UUU ACG CUG AGU ACU UCG AdTdT UCG AAG UAC UCA GCG UAA GdTdT | C1 -> U | 29 |
| | AS | | none | 15 |
| AD-15962 | S | CAU ACG CUG AGU ACU UCG AdTdT UCG AAG UAC UCA GCG UAA GdTdT | U2 -> A | 30 |
| | AS | | none | 15 |
| AD-15963 | S | CCU ACG CUG AGU ACU UCG AdTdT UCG AAG UAC UCA GCG UAA GdTdT | U2 -> C | 31 |
| | AS | | none | 15 |
| AD-15964 | S | CGU ACG CUG AGU ACU UCG AdTdT UCG AAG UAC UCA GCG UAA GdTdT | U2 -> G | 32 |
| | AS | | none | 15 |
| AD-15965 | S | CUA ACG CUG AGU ACU UCG AdTdT UCG AAG UAC UCA GCG UAA GdTdT | U3 -> A | 33 |
| | AS | | none | 15 |
| AD-15966 | S | CUC ACG CUG AGU ACU UCG AdTdT UCG AAG UAC UCA GCG UAA GdTdT | U3 -> C | 34 |
| | AS | | none | 15 |
| AD-15967 | S | CUG ACG CUG AGU ACU UCG AdTdT UCG AAG UAC UCA GCG UAA GdTdT | U3 -> G | 35 |
| | AS | | none | 15 |
| AD-15968 | S | CUU GCG CUG AGU ACU UCG AdTdT UCG AAG UAC UCA GCG UAA GdTdT | A4 -> G | 36 |
| | AS | | none | 15 |
| AD-15969 | S | CUU CCG CUG AGU ACU UCG AdTdT UCG AAG UAC UCA GCG UAA GdTdT | A4 -> C | 37 |
| | AS | | none | 15 |
| AD-15970 | S | CUU UCG CUG AGU ACU UCG AdTdT UCG AAG UAC UCA GCG UAA GdTdT | A4 -> U | 38 |
| | AS | | none | 15 |
| AD-15971 | S | CUU AAG CUG AGU ACU UCG AdTdT UCG AAG UAC UCA GCG UAA GdTdT | C5 -> A | 39 |
| | AS | | none | 15 |
| AD-15972 | S | CUU AGG CUG AGU ACU UCG AdTdT UCG AAG UAC UCA GCG UAA GdTdT | C5 -> G | 40 |
| | AS | | none | 15 |
| AD-15973 | S | CUU AUG CUG AGU ACU UCG AdTdT UCG AAG UAC UCA GCG UAA GdTdT | C5 -> U | 41 |
| | AS | | none | 15 |
| AD-15974 | S | CUU ACA CUG AGU ACU UCG AdTdT UCG AAG UAC UCA GCG UAA GdTdT | G6 -> A | 42 |
| | AS | | none | 15 |
| AD-15975 | S | CUU ACC CUG AGU ACU UCG AdTdT UCG AAG UAC UCA GCG UAA GdTdT | G6 -> C | 43 |
| | AS | | none | 15 |
| AD-15976 | S | CUU ACU CUG AGU ACU UCG AdTdT UCG AAG UAC UCA GCG UAA GdTdT | G6 -> U | 44 |
| | AS | | none | 15 |
| AD-15977 | S | CUU ACG AUG AGU ACU UCG AdTdT UCG AAG UAC UCA GCG UAA GdTdT | C7 -> A | 45 |
| | AS | | none | 15 |
| AD-15978 | S | CUU ACG GUG AGU ACU UCG AdTdT UCG AAG UAC UCA GCG UAA GdTdT | C7 -> G | 46 |
| | AS | | none | 15 |
| AD-15979 | S | CUU ACG UUG AGU ACU UCG AdTdT UCG AAG UAC UCA GCG UAA GdTdT | C7 -> U | 47 |
| | AS | | none | 15 |
| AD-15980 | S | CUU ACG CAG AGU ACU UCG AdTdT UCG AAG UAC UCA GCG UAA GdTdT | U8 -> A | 48 |
| | AS | | none | 15 |
| AD-15981 | S | CUU ACG CCG AGU ACU UCG AdTdT | U8 -> C | 49 |
| | AS | UCG AAG UAC UCA GCG UAA GdTdT | none | 15 |
| AD-15982 | S | CUU ACG CGG AGU ACU UCG AdTdT UCG AAG UAC UCA GCG UAA GdTdT | U8 -> G | 50 |
| | AS | | none | 15 |
| AD-15983 | S | CUU ACG CUG AGU CCU UCG AdTdT UCG AAG UAC UCA GCG UAA GdTdT | A13 -> C | 51 |
| | AS | | none | 15 |
| AD-15984 | S | CUU ACG CUG AGU GCU UCG AdTdT UCG AAG UAC UCA GCG UAA GdTdT | A13 -> G | 52 |
| | AS | | none | 15 |
| AD-15985 | S | CUU ACG CUG AGU UCU UCG AdTdT UCG AAG UAC UCA GCG UAA GdTdT | A13 -> U | 53 |
| | AS | | none | 15 |
| AD-15986 | S | CUU ACG CUG AGU AAU UCG AdTdT UCG AAG UAC UCA GCG UAA GdTdT | C14 -> A | 54 |
| | AS | | none | 15 |
| AD-15987 | S | CUU ACG CUG AGU AGU UCG AdTdT UCG AAG UAC UCA GCG UAA GdTdT | C14 -> G | 55 |
| | AS | | none | 15 |
| AD-15988 | S | CUU ACG CUG AGU AUU UCG AdTdT UCG AAG UAC UCA GCG UAA GdTdT | C14 -> U | 56 |
| | AS | | none | 15 |
| AD-15989 | S | CUU ACG CUG AGU ACA UCG AdTdT UCG AAG UAC UCA GCG UAA GdTdT | U15 -> A | 57 |
| | AS | | none | 15 |
| AD-15990 | S | CUU ACG CUG AGU ACC UCG AdTdT UCG AAG UAC UCA GCG UAA GdTdT | U15 -> C | 58 |
| | AS | | none | 15 |
| AD-15991 | S | CUU ACG CUG AGU ACG UCG AdTdT UCG AAG UAC UCA GCG UAA GdTdT | U15 -> G | 59 |
| | AS | | none | 15 |
| AD-15992 | S | CUU ACG CUG AGU ACU ACG AdTdT UCG AAG UAC UCA GCG UAA GdTdT | U16 -> A | 60 |
| | AS | | none | 15 |
| AD-15993 | S | CUU ACG CUG AGU ACU CCG AdTdT UCG AAG UAC UCA GCG UAA GdTdT | U16 -> C | 61 |
| | AS | | none | 15 |
| AD-15994 | S | CUU ACG CUG AGU ACU GCG AdTdT UCG AAG UAC UCA GCG UAA GdTdT | U16 -> G | 62 |
| | AS | | none | 15 |
| AD-15995 | S | CUU ACG CUG AGU ACU UAG AdTdT UCG AAG UAC UCA GCG UAA GdTdT | C17 -> A | 63 |
| | AS | | none | 15 |
| AD-15996 | S | CUU ACG CUG AGU ACU UGG AdTdT UCG AAG UAC UCA GCG UAA GdTdT | C17 -> G | 64 |
| | AS | | none | 15 |
| AD-15997 | S | CUU ACG CUG AGU ACU UUG AdTdT UCG AAG UAC UCA GCG UAA GdTdT | C17 -> U | 65 |
| | AS | | none | 15 |
| AD-15998 | S | CUU ACG CUG AGU ACU UCA AdTdT UCG AAG UAC UCA GCG UAA GdTdT | G18 -> A | 66 |
| | AS | | none | 15 |
| AD-15999 | S | CUU ACG CUG AGU ACU UCC AdTdT UCG AAG UAC UCA GCG UAA GdTdT | G18 -> C | 67 |
| | AS | | none | 15 |
| AD-16000 | S | CUU ACG CUG AGU ACU UCU AdTdT UCG AAG UAC UCA GCG UAA GdTdT | G18 -> U | 68 |
| | AS | | none | 15 |
| AD-16001 | S | CUU ACG CUG AGU ACU UCG CdTdT UCG AAG UAC UCA GCG UAA GdTdT | A19 -> C | 69 |
| | AS | | none | 15 |
| AD-16002 | S | CUU ACG CUG AGU ACU UCG GdTdT UCG AAG UAC UCA GCG UAA GdTdT | A19 -> G | 70 |
| | AS | | none | 15 |
| AD-16003 | S | CUU ACG CUG AGU ACU UCG UdTdT UCG AAG UAC UCA GCG UAA GdTdT | A19 -> U | 71 |
| | AS | | none | 15 |

| | | | | |
|---|---|---|---|---|
| *Mismatches are shown in bold.* | | | | |

### HeLa Dual Luciferase (Dual Luc or HLDL) Assay Procedure

HeLa cells stably expressing Firefly and Renilla luciferases were cultured in DMEM (Invitrogen) supplemented with 10% FBS and 1x Glutamax + Zeosin/Puromycin and plated in 96-well plates (opaque walls), 10K cells/well in DMEM / 10% FBS w/o antibiotics. Transfection was performed using Lipofectamine 24 hours after plating the cells and after another 24 h of incubation, the luciferase assay was performed as described briefly below:

Reagent preparation (all reagents were purchased from Promega):
1. The contents of one bottle of Dual-Glo Luciferase buffer were transferred to one bottle of Dual-Glo Luciferase substrate to create the Dual-Glo Luciferase Reagent. The solution was mixed by inversion until the substrate was completely dissolved and aliquoted into the amount needed for this experiment (45 mL) plus 9 mL portions, which were frozen for future use.
2. 0.450 mL of Dual Glo Stop & Glo substrate was diluted 1: 100 into 45 mL of Dual Glo Stop & Glo buffer in a 50 mL conical vial.
3. Both reagents were brought to room temperature (r.t.) before use. Similarly, the cells were equilibrated to r.t. before running the assay.

### Assay procedure:

1. The medium was removed from the plated cells by vacuum suction and replaced with 75 µL each well of Phenol-Red-free DMEM medium (Invitrogen).
2. 75 µL of Dual-Glo Luciferase Reagent was added and mixed well by agitating the plate.
3. The plate was shaken on shaker for 20 minutes, and then the firefly luminescence was measured in the luminometer; settings: Iva luminescence 96 wp.
4. 75 µL of Dual-Glo Stop & Glo Reagent was added to each well and mixed well by agitating the plate.
5. The plate was shaken on shaker for 15 min and the Renilla luminescence was measured in the luminometer; settings: Iva luminescence 96 wp
6. The data was exported into Excel and the ratio of luminescence from Firefly to Renilla was calculated and normalized to the results from the untreated control wells.

### Results

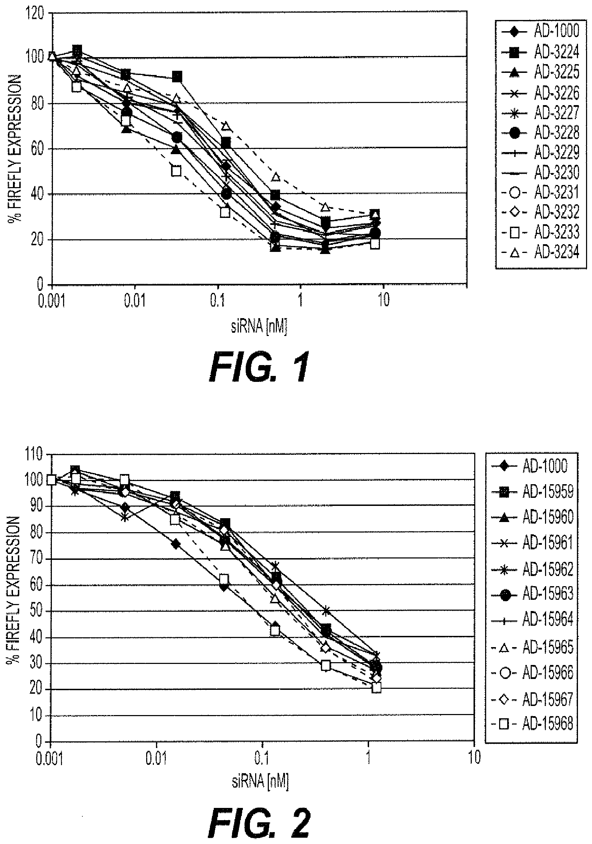

Tabulated below are IC50 values derived using results from various HeLa cell based dual luciferase assays, as described above. Figures 1-6 depict graphically the primary data obtained from the assays. The results demonstrate that the effect of the mismatch base pairing on siRNA potency is dependent on the mismatched base pair and its position on the sense strand. Apparently, local destabilization in the central region of the sense strand via mismatched base pairs (position 9-12) are more effective in enhancing potency than those outside of this region. For this siRNA, particularly mismatches in position 9 were found to significantly enhance potency over the parent compound.

**Table 3. Calculated IC50 values for the results shown in Figure 1.**

| **Duplex ID** | **IC 50 Value (nM)** |
|---|---|
| AD-1000 | 0.146 |
| AD-3224 | 0.033 |
| AD-3225 | 0.043 |
| AD-3226 | 0.069 |
| AD-3227 | 0.162 |
| AD-3228 | 0.065 |
| AD-3229 | 0.103 |
| AD-3230 | 0.106 |
| AD-3231 | 0.153 |
| AD-3232 | 0.139 |
| AD-3233 | 0.233 |
| AD-3234 | 0.42. |

**Table 4. Calculated IC50 values for the results shown in Figure 2.**

| **Duplex ID** | **IC 50 Value (nM)** |
|---|---|
| AD-1000 | 0.076 |
| AD-3202 | 0.044 |
| AD-15959 | 0.235 |
| AD-15960 | 0.16 |
| AD-15961 | 0.241 |
| AD-15962 | 0.351 |
| AD-15963 | 0.214 |
| AD-15964 | 0.195 |
| AD-15965 | 0.221 |
| AD-15966 | 0.151 |
| AD-15967 | 0.187 |
| AD-15968 | 0.083 |

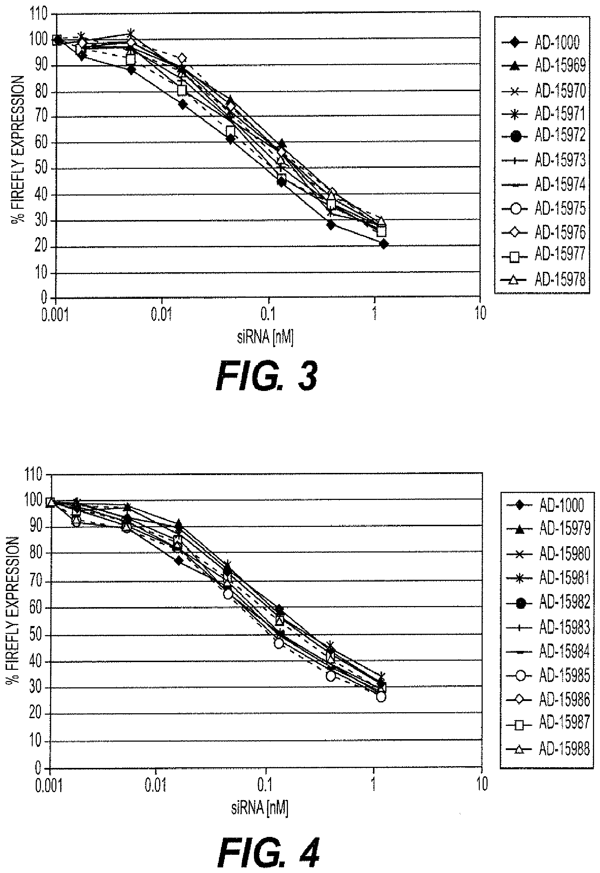

**Table 5. Calculated IC50 values for the results shown in Figure 3.**

| **Duplex ID** | **IC 50 Value (nM)** |
|---|---|
| AD-1000 | 0.08 |
| AD-3202 | 0.041 |
| AD-15969 | 0.188 |
| AD-15970 | 0.148 |
| AD-15971 | 0.139 |
| AD-15972 | 0.168 |
| AD-15973 | 0.125 |
| AD-15974 | 0.108 |
| AD-15975 | 0.156 |
| AD-15976 | 0.182 |
| AD-15977 | 0.102 |
| AD-159678 | 0.155 |

**Table 6. Calculated IC50 values for the results shown in Figure 4.**

| **Duplex ID** | **IC 50 Value (nM)** |
|---|---|
| AD-1000 | 0.124 |
| AD-3202 | 0.05 |
| AD-15979 | 0.188 |
| AD-15980 | 0.139 |
| AD-15981 | 0.214 |
| AD-15982 | 0.103 |
| AD-15983 | 0.178 |
| AD-15984 | 0.108 |
| AD-15985 | 0.119 |
| AD-15986 | 0.216 |
| AD-15987 | 0.167 |
| AD-15988 | 0.173 |

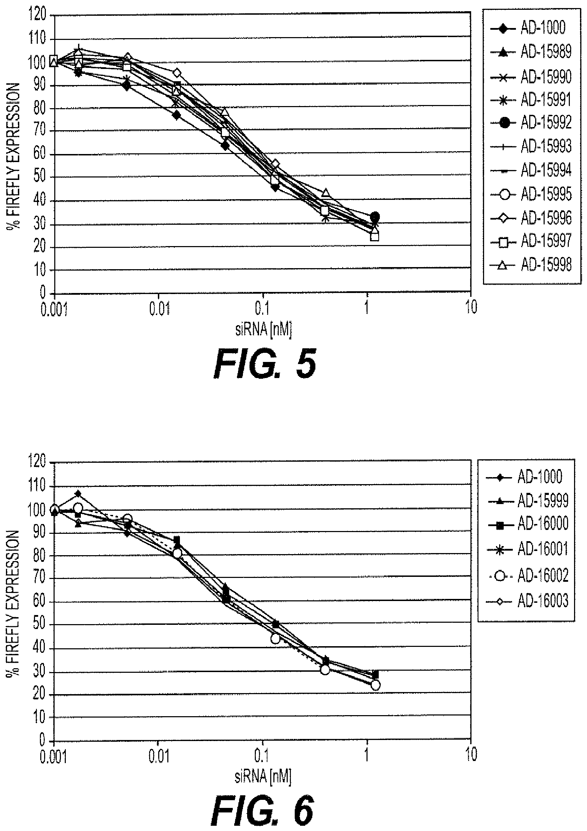

**Table 7. Calculated IC50 values for the results shown in Figures 5.**

| **Duplex ID** | **IC 50 Value (nM)** |
|---|---|
| AD-1000 | 0.091 |
| AD-3202 | 0.046 |
| AD-15989 | 0.143 |
| AD-15990 | 0.119 |
| AD-15991 | 0.108 |
| AD-15992 | 0.157 |
| AD-15993 | 0.125 |
| AD-15994 | 0.119 |
| AD-15995 | 0.156 |
| AD-15996 | 0.157 |
| AD-15997 | 0.117 |
| AD-15998 | 0.175 |

**Table 8. Calculated IC50 values for the results shown in Figure 6.**

| **Duplex ID** | **IC 50 Value (nM)** |
|---|---|
| AD-1000 | 0.096 |
| AD-3202 | 0.035 |
| AD-15999 | 0.124 |
| AD-16000 | 0.11 |
| AD-16001 | 0.087 |
| AD-16002 | 0.084 |
| AD-16003 | 0.078 |

### Example 2 - siRNAs containing modified nucleobases.

Based on the results described above in Example 1, also encompassed herein are other nucleoside isosteres and modifications, which will influence the local structure of the siRNA duplex and enhance the potency and activity of iRNA agents containing these modifications. Non-limiting examples are provided below and some siRNAs synthesized and tested listed in the following Tables 9-13:
2,4-difluorotoluyl ribo- and deoxyribonucleotide 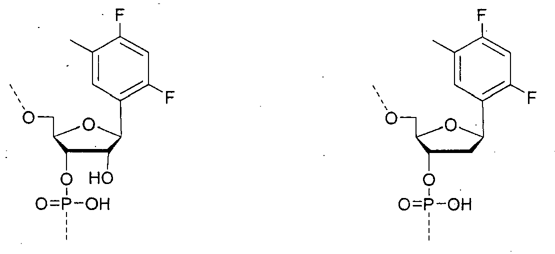
5-Nitroindole ribo- and deoxyribonucleotide 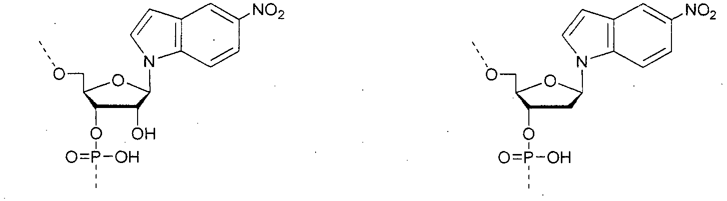
3-Nitropyrrole ribo- and deoxyribonucleotide 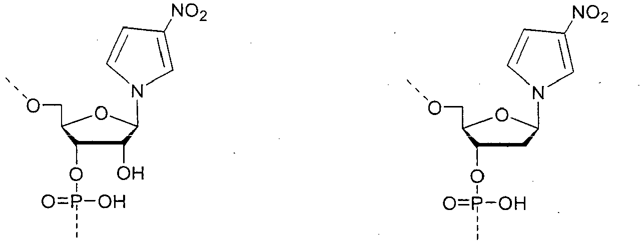
Ribo- and deoxyribonebularine 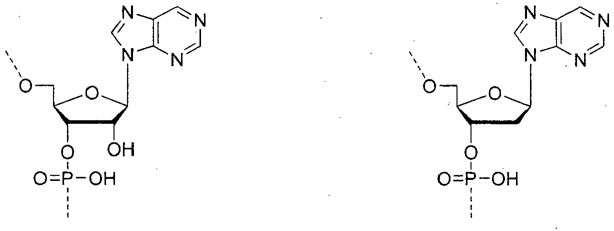
4-Fluoro-6-methylbenzimidazole ribo- and deoxyribonucleotide 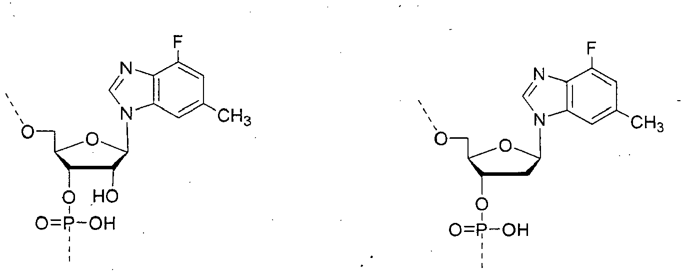
4-Methylbenzimidazole ribo- and deoxyribonucleotide 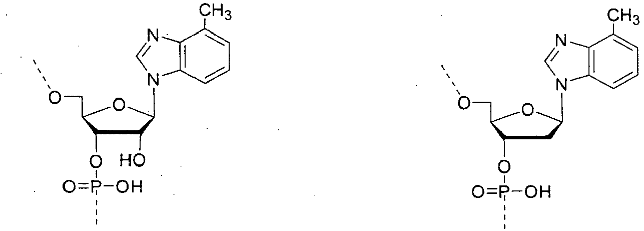
Ribo- and deoxyriboinosine

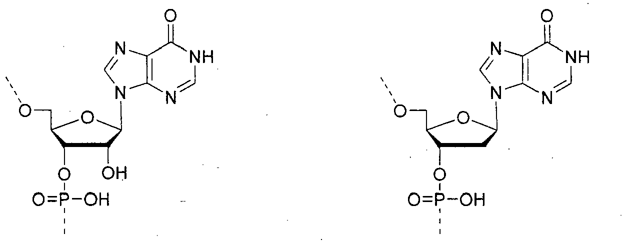

**Table 9. siRNA Duplexes targeting firefly luciferases and containing 2,4-difluorotoluyl deoxyribonucleotide or 2,4-difluorotoluyl nibonucleotide in the sense strand.**

| **Duplex ID** | **Strand** | **Sequence 5' to 3'** | **Modifications** | **SEQ ID NO.** |
|---|---|---|---|---|
| 1000 | S | CUU ACG CUG AGU ACU UCG AdTdT UCG AAG UAC UCA GCG UAA GdTdT | none | 72 |
| | AS | | none | 15 |
| 3200 | S | CUU ACG CY1G AGU ACU UCG AdTdT UCG AAG UAC UCA GCG UAA GdTdT | U8 -> rF | 73 |
| | AS | | none | 15 |
| 3201 | S | CUU ACG CUY1 AGU ACU UCG AdTdT UCG AAG UAC UCA GCG UAA GdTdT | G9 -> rF | 74 |
| | AS | | none | 15 |
| 3202 | S | CUU ACG CUG Y1GU ACU UCG AdTdT UCG AAG UAC UCA GCG UAA GdTdT | A10 -> rF | 75 |
| | AS | | none | 15 |
| 3203 | S | CUU ACG CUG AY1U ACU UCG AdTdT UCG AAG UAC UCA GCG UAA GdTdT | G11 -> rF | 76 |
| | AS | | none | 15 |
| 3204 | S | CUU ACG CUG AGY1 ACU UCG AdTdT UCG AAG UAC UCA GCG UAA GdTdT | U12 -> rF | 77 |
| | AS | | none | 15 |
| 3205 | S | CUU ACG CUG AGU Y1CU UCG AdTdT UCG AAG UAC UCA GCG UAA GdTdT | A13 -> rF | 78 |
| | AS | | none | 15 |
| 3206 | S | CUU ACG CUG AGU AY1U UCG AdTdT UCG AAG UAC UCA GCG UAA GdTdT | C14 -> rF | 79 |
| | AS | | none | 15 |
| 3207 | S | CUU ACG CUG AGU ACY1 UCG AdTdT UCG AAG UAC UCA GCG UAA GdTdT | U15 -> rF | 80 |
| | AS | | none | 15 |
| 3208 | S | CUU ACG CUG AGU ACU Y1CG AdTdT UCG AAG UAC UCA GCG UAA GdTdT | U16 -> rF | 81 |
| | AS | | none | 15 |
| 3209 | S | CUU ACG CY1G AGY1 ACU UCG AdTdT UCG AAG UAC UCA GCG UAA GdTdT | U8/U12 -> rF | 82 |
| | AS | | none | 15 |
| 3210 | S | CUU ACG CUG AGY1 ACY1 UCG AdTdT UCG AAG UAC UCA GCG UAA GdTdT | U12/U15 -> rF | 83 |
| | AS | | none | 15 |
| 3334 | S | CUU ACG CUY2 AGU ACU UCG AdTdT UCG AAG UAC UCA GCG UAA GdTdT | G9 -> Y2 | 84 |
| | AS | | none | 15 |
| 3335 | S | CUU ACG CUG Y2GU ACU UCG AdTdT UCG AAG UAC UCA GCG UAA GdTdT | A10 -> Y2 | 85 |
| | AS | | none | 15 |
| 3336 | S | CUU ACG CUG AY2U ACU UCG AdTdT UCG AAG UAC UCA GCG UAA GdTdT | G11 -> Y2 | 86 |
| | AS | | none | 15 |
| 3337 | S | CUU ACG CUG AGY2 ACU UCG AdTdT UCG AAG UAC UCA GCG UAA GdTdT | U12 -> Y2 | 87 |
| | AS | | none | 15 |

| | | | | |
|---|---|---|---|---|
| *Y1, 2,4-difluorotoluylribonucleotide; Y2, 2,4-difluorotoluyldeoxyribonucleotide* | | | | |

**Table 10. siRNA Duplexes targeting firefly luciferase and containing 5-nitroindole deoxyribonucleotide or 5-nitroindole ribonucleotide in the sense strand.**

| **Duplex ID** | **Strand** | **Sequence 5' to 3'** | **Modification** | **SEQ ID NO.** |
|---|---|---|---|---|
| AD-1000 | S | CUU ACG CUG AGU ACU UCG AdTdT UCG AAG UAC UCA GCG UAA GdTdT | none | 72 |
| | AS | | none | 15 |
| AD-3310 | S | CUU ACG CUY3 AGU ACU UCG AdTdT UCG AAG UAC UCA GCG UAA GdTdT | G9 -> Y3 | 88 |
| | AS | | none | 15 |
| AD-3311 | S | CUU ACG CUG Y3GU ACU UCG AdTdT UCG AAG UAC UCA GCG UAA GdTdT | A10 -> Y3 | 89 |
| | AS | | none | 15 |
| AD-3312 | S | CUU ACG CUG AY3U ACU UCG AdTdT UCG AAG UAC UCA GCG UAA GdTdT | G11 -> Y3 | 90 |
| | AS | | none | 15 |
| AD-3313 | S | CUU ACG CUG AGY3 ACU UCG AdTdT UCG AAG UAC UCA GCG UAA GdTdT | U12 -> Y3 | 91 |
| | AS | | none | 15 |
| AD-3314 | S | CUU ACG CUY4 AGU ACU UCG AdTdT UCG AAG UAC UCA GCG UAA GdTdT | G9 -> Y4 | 92 |
| | AS | | none | 15 |
| AD-3315 | S | CUU ACG CUG **Y4**GU ACU UCG AdTdT UCG AAG UAC UCA GCG UAA GdTdT | A10 -> Y4 | 93 |
| | AS | | none | 15 |
| AD-3316 | S | CUU ACG CUG AY4U ACU UCG AdTdT UCG AAG UAC UCA GCG UAA GdTdT | G11 -> Y4 | 94 |
| | AS | | none | 15 |
| AD-3317 | S | CUU ACG CUG AGY4 ACU UCG AdTdT UCG AAG UAC UCA GCG UAA GdTdT | U12 -> Y4 | 95 |
| | AS | | none | 15 |

| | | | | |
|---|---|---|---|---|
| *Y3, 5-nitroindole deoxyribonucleotide; Y4, 5-nitroindole ribonucleotide* | | | | |

**Table 11. siRNA Duplexes targeting firefly luciferase and containing ribo- or deoxyribonebularine in the sense strand.**

| **Duplex ID** | **Strand** | **Sequence 5' to 3'** | **Modification** | **SEQ ID NO.** |
|---|---|---|---|---|
| 1000 | S | CUU ACG CUG AGU ACU UCG AdTdT UCG AAG UAC UCA GCG UAA GdTdT | none | 72 |
| | AS | | none | 15 |
| AD-3318 | S | CUU ACG CUY17 AGU ACU UCG AdTdT UCG AAG UAC UCA GCG UAA GdTdT | G9 -> Y'17 | 96 |
| | AS | | none | 15 |
| AD-3319 | S | CUU ACG CUG Y17GU ACU UCG AdTdT UCG AAG UAC UCA GCG UAA GdTdT | A10 -> Y17 | 97 |
| | AS | | none | 15 |
| AD-3320 | S | CUU ACG CUG AY17U ACU UCG AdTdT UCG AAG UAC UCA GCG UAA GdTdT | G11 -> Y17 | 98 |
| | AS | | none | 15 |
| AD-3321 | S | CUU ACG CUG AGY17 ACU UCG AdTdT UCG AAG UAC UCA GCG UAA GdTdT | U12 -> Y17 | 99 |
| | AS | | none | 15 |
| AD-3322 | S | CUU ACG CUY20 AGU ACU UCG AdTdT UCG AAG UAC UCA GCG UAA GdTdT | G9 -> Y20 | 100 |
| | AS | | none | 15 |
| AD-3323 | S | CUU ACG CUG Y20GU ACU UCG AdTdT UCG AAG UAC UCA GCG UAA GdTdT | A10 -> Y20 | 101 |
| | AS | | none | 15 |
| AD-3324 | S | CUU ACG CUG AY20U ACU UCG AdTdT UCG AAG UAC UCA GCG UAA GdTdT | G11 -> Y20 | 102 |
| | AS | | none | 15 |
| AD-3325 | S | CUU ACG CUG AGY20 ACU UCG AdTdT UCG AAG UAC UCA GCG UAA GdTdT | U12 -> Y20 | 103 |
| | AS | | none | 15 |

| | | | | |
|---|---|---|---|---|
| *Y17, deoxyribonebularine; Y20, ribonebularine* | | | | |

**Table 12. siRNA Duplexes targeting firefly luciferase and containing ribo- or deoxyriboinosine in the sense strand.**

| **Duplex** | **Strand** | **Sequence 5' to 3'** | **Modifications** | **SEQ ID NO.** |
|---|---|---|---|---|
| AD-1000 | S | CUU ACG CUG AGU ACU UCG AdTdT UCG AAG UAC UCA GCG UAA GdTdT | none | 72 |
| | AS | | none | 15 |
| AD-3326 | S | CUU ACG CUdI AGU ACU UCG AdTdT UCG AAG UAC UCA GCG UAA GdTdT | G9 -> dl | 104. |
| | AS | | none | 15 |
| AD-3327 | S | CUU ACG CUG dIGU ACU UCG AdTdT UCG AAG UAC UCA GCG UAA GdTdT | A10 -> dl | 105 |
| | AS | | none | 15 |
| AD-3328 | S | CUU ACG CUG AdIU ACU UCG AdTdT UCG AAG UAC UCA GCG UAA GdTdT | G11 -> dl | 106 |
| | AS | | none | 15 |
| AD-3329 | S | CUU ACG CUG AGdI ACU UCG AdTdT UCG AAG UAC UCA GCG UAA GdTdT | U12 -> dl | 107 |
| | AS | | none | 15 |
| AD-3330 | S | CUU ACG CUI AGU ACU UCG AdTdT UCG AAG UAC UCA GCG UAA GdTdT | G9 -> I | 108 |
| | AS | | none | 15 |
| AD-3331 | S | CUU ACG CUG IGU ACU UCG AdTdT UCG AAG UAC UCA GCG UAA GdTdT | A10 -> I | 109 |
| | AS | | none | 15 |
| AD-3332 | S | CUU ACG CUG AIU ACU UCG AdTdT UCG AAG UAC UCA GCG UAA GdTdT | G11 -> I | 110 |
| | AS | | none | 15 |
| AD-3333 | S | CUU ACG CUG AGI ACU UCG AdTdT UCG AAG UAC UCA GCG UAA GdTdT | U12 -> I | 111 |
| | AS | | none | 15 |

| | | | | |
|---|---|---|---|---|
| *dI, deoxyinosine; 1, riboinosine* | | | | |

**Table 13. siRNA Duplexes targeting firefly luciferase and containing ribo- or deoxyribo-2-aminopurine in the sense strand.**

| **Duplex ID** | **Strand** | **Sequence 5' to 3'** | **Modifications** | **SEQ ID NO.** |
|---|---|---|---|---|
| AD-1000 | S | CUU ACG CUG AGU ACU UCG AdTdT UCG AAG UAC UCA GCG UAA GdTdT | none | 72 |
| | AS | | none | 15 |
| AD-3347 | S | CUU ACG CUY19 AGU ACU UCG AdTdT UCG AAG UAC UCA GCG UAA GdTdT | G9 -> Y19 | 112 |
| | AS | | none | 15 |
| AD-3348 | S | CUU ACG CUG Y19GU ACU UCG AdTdT UCG AAG UAC UCA GCG UAA GdTdT | A10 -> Y19 | 113 |
| | AS | | none | 15 |
| AD-3349 | S | CUU ACG CUG AY19U ACU UCG AdTdT UCG AAG UAC UCA GCG UAA GdTdT | G11 -> Y19 | 114 |
| | AS | | none | 15 |
| AD-3350 | S | CUU ACG CUG AGY19 ACU UCG AdTdT UCG AAG UAC UCA GCG UAA GdTdT | U12 -> Y19 | 115 |
| | AS | | none | 15 |
| AD-3351 | S | CUU ACG CUY18 AGU ACU UCG AdTdT UCG AAG UAC UCA GCG UAA GdTdT | G9 -> Y18 | 116 |
| | AS | | none | 15 |
| AD-3352 | S | CUU ACG CUG Y18GU ACU UCG AdTdT UCG AAG UAC UCA GCG UAA GdTdT | A10 -> Y18 | 117 |
| | AS | | none | 15 |
| AD-3353 | S | CUU ACG CUG AY18U ACU UCG AdTdT UCG AAG UAC UCA GCG UAA GdTdT | G11 -> Y18 | 118 |
| | AS | | none | 15 |
| AD-3354 | S | CUU ACG CUG AGY18 ACU UCG AdTdT UCG AAG UAC UCA GCG UAA GdTdT | U12 -> Y18 | 119 |
| | AS | | none | 15 |

| | | | | |
|---|---|---|---|---|
| *Y18, ribo-2-aminopurine; Y19,deoxyribo-2-aminopurine* | | | | |

### HeLa Dual Luciferase (Dual Luc or HLDL) Assay Procedure

HeLa cells stably expressing Firefly and Renilla luciferases were cultured and the assay was carried out as described under Example 1.

### Results

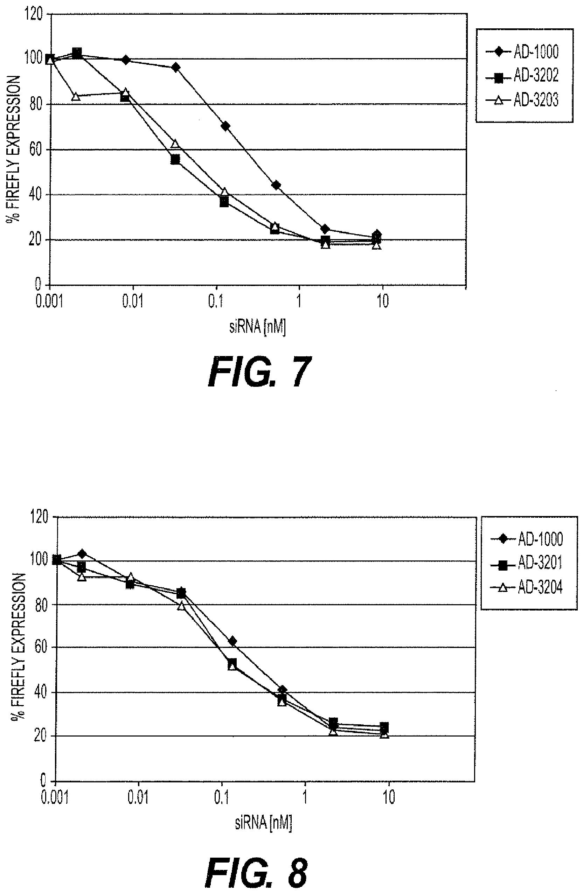

Tabulated below are IC50 values derived using results from various HeLa cell based dual luciferase assays, as described above. Figures 7-13 depict graphically the primary data obtained from the assays. The results demonstrate that the effect of the nucleobase modifications on siRNA potency is dependent on the modifications as well as its position on the sense strand. For this siRNA, particularly nucleobase modifications in position 10 were found to significantly enhance potency over the parent compound.

**Table 14. Calculated IC50 values for the results shown in Figure 7-9.**

| **Duplex ID** | **IC 50 Value (nM)** |
|---|---|
| AD-1000 | 0.327 |
| AD-3202 | 0.046 |
| AD-3203 | 0.073 |
| And-1000 | 0.245 |
| AD-3201 | 0.164 |
| AD-3204 | 0.160 |
| AD-1000 | 0.280 |
| AD-3334 | 0.144 |
| AD-3335 | 0.072 |
| AD-3336 | 0.158 |

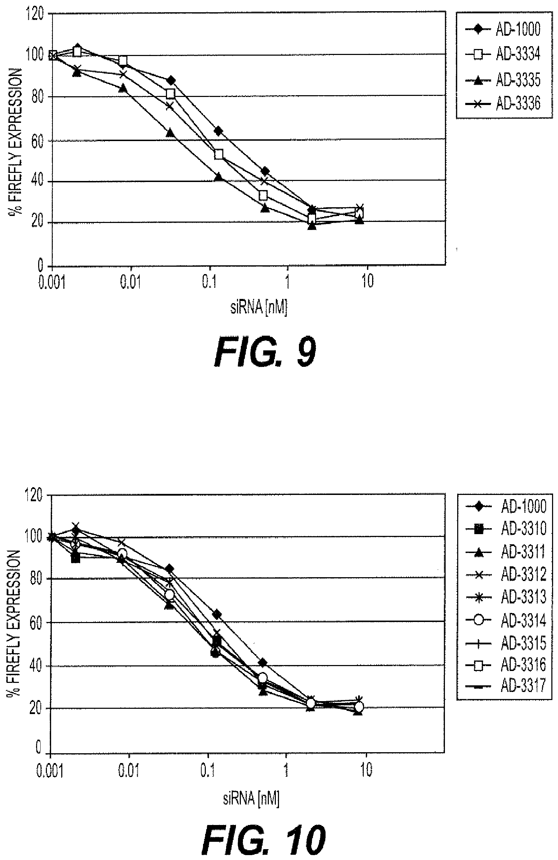

**Table 15. Calculated IC50 values for the results shown in Figure 10.**

| **Duplex ID** | **IC 50 Value (nM)** |
|---|---|
| AD-1000 | 0.245 |
| AD-3310 | 0.136 |
| AD-3311 | 0.098 |
| AD-3312 | 0.162 |
| AD-3313 | 0.134 |
| AD-3314 | 0.111 |
| AD-3314 | 0.098 |
| AD-3315 | 0.132 |
| AD-3316 | 0.132 |
| AD-3317 | 0.245 |

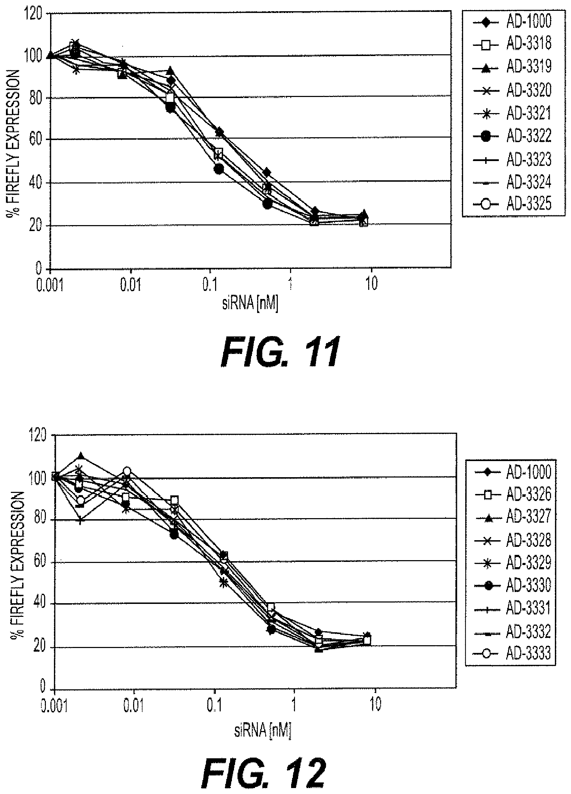

**Table 16. Calculated IC50 values for the results shown in Figure 11.**

| **Duplex ID** | **IC 50 Value (nM)** |
|---|---|
| AD-1000 | 0.28 |
| AD-3318 | 0.141 |
| AD-3319 | 0.243 |
| AD-3320 | 0.143 |
| AD-3321 | 0.138 |
| AD-3322 | 0.107 |
| AD-3323 | 0.24 |
| AD-3324 | 0.151 |
| AD-3325 | 0.154 |

**Table 17. Calculated IC50 values for the results shown in Figure 12.**

| **Duplex ID** | **IC 50 Value (nM)** |
|---|---|
| AD-1000 | 0.209 |
| AD-3326 | 0.229 |
| AD-3327 | 0.153 |
| AD-3328 | 0.155 |
| AD-3329 | 0.131 |
| AD-3330 | 0.132 |
| AD-3331 | 0.125 |
| AD-3332 | 0.188 |
| AD-3333 | 0.145 |

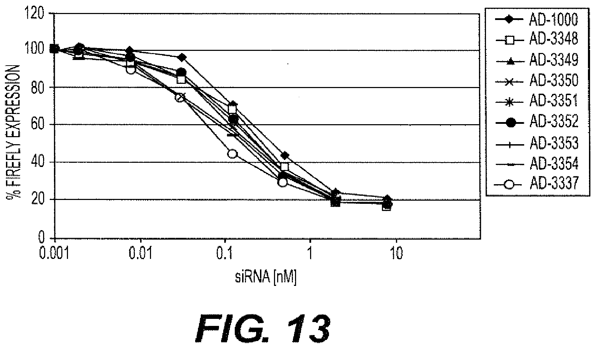

**Table 18. Calculated IC50 values for the results shown in Figure 13.**

| **Duplex ID** | **IC 50 Value (nM)** |
|---|---|
| AD-1000 | 0.327 |
| AD-3348 | 0.257 |
| AD-3349 | 0.188 |
| AD-3350 | 0.155 |
| AD-3351 | 0.182 |
| AD-3352 | 0.21 |
| AD-3353 | 0.215 |
| AD-3354 | 0.132 |

### Example 3 - Correlation of potency with thermal stability of the siRNA duplexes.

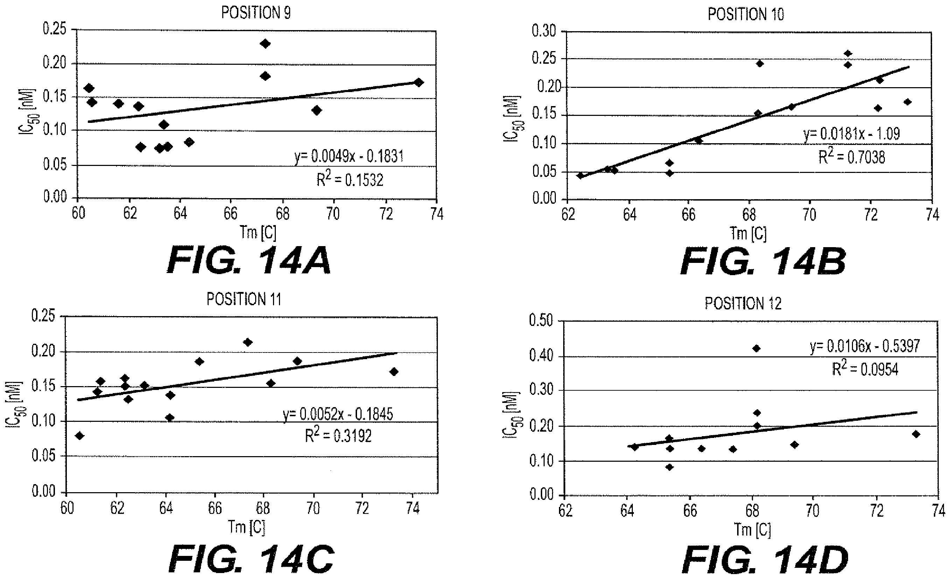

Based on the results described above in Example 1 and 2, the thermal stability of the siRNA duplexes was measured in 0.9% saline solution and plotted against the potency expressed by their corresponding IC50 values. Figure 14 shows the results for each of the central positions 9-12 of the sense strand. The results indicate that while there is no significant correlation between activity and thermal stability at positions 9, 11 and 12, the potency of the siRNAs appears to increase with decreased thermal stability due to modifications in position 10.

### Example 4 - siRNAs containing abasic modifications.

Based on the results described above in Example 1 and 2, also encompassed herein are abasic modifications, which will influence the local structure of the siRNA duplex and enhance the potency and activity of iRNA agents containing these modifications. Non-limiting examples are provided below:

### Abasic modifications (plus similar structures)

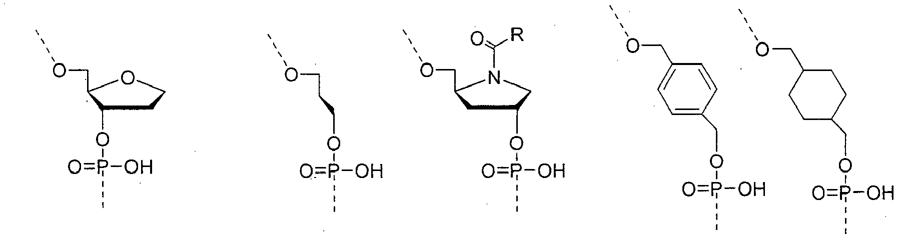

**Table 19. siRNA Duplexes targeting firefly luciferase and containing abasic modifications in the sense strand.**

| **Duplex ID** | **Strand** | **Sequence 5' to 3'** | **Modifications** | **SEQ ID NO.** |
|---|---|---|---|---|
| AD-1000 | S | CUU ACG CUG AGU ACU UCG AdTdT UCG AAG UAC UCA GCG UAA GdTdT | none | 72 |
| | AS | | none | 15 |
| AD-19040 | S | CUU ACG CUY16 AGU ACU UCG AdTdT UCG AAG UAC UCA GCG UAA GdTdT | G9 -> Y16 | 120 |
| | AS | | none | 15 |
| AD-19041 | S | CUU ACG CUG Y16GU ACU UCG AdTdT UCG AAG UAC UCA GCG UAA GdTdT | A10 -> Y16 | 121 |
| | AS | | none | 15 |
| AD-19042 | S | CUU ACG CUG AY16U ACU UCG AdTdT UCG AAG UAC UCA GCG UAA GdTdT | G11 -> Y16 | 122 |
| | AS | | none | 15 |
| AD-19043 | S | CUU ACG CUG AGY16 ACU UCG AdTdT UCG AAG UAC UCA GCG UAA GdTdT | U12 -> Y16 | 123 |
| | AS | | none | 15 |

| | | | | |
|---|---|---|---|---|
| *Y16, abasic modification (2-hydroxymethyl-tetrahydrojurane-3-phosphate)* | | | | |

### HeLa Dual Luciferase (Dual Luc or HLDL) Assay Procedure

HeLa cells stably expressing Firefly and Renilla luciferases were cultured and the assay was carried out as described under Example 1.

### Results

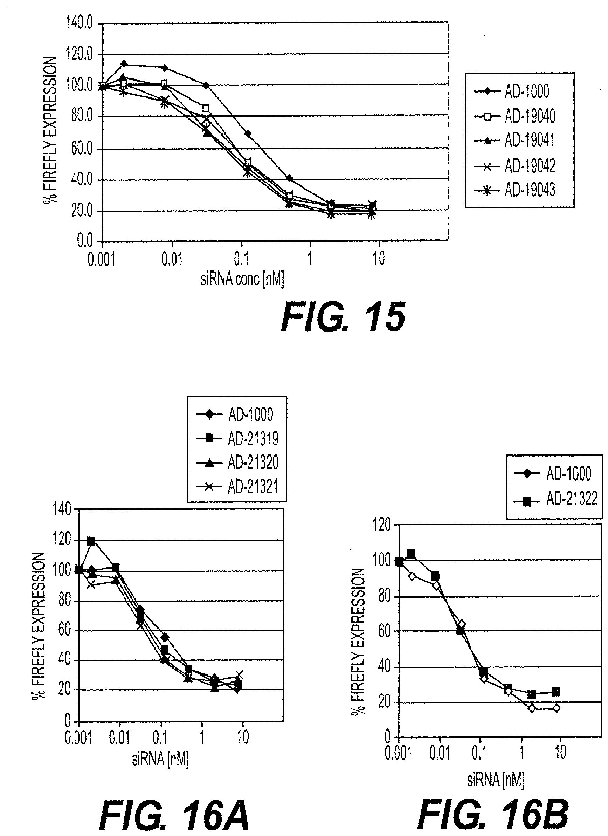

Tabulated below are IC50 values derived using results from HeLa cell based dual luciferase assays, as described above. Figure 15 depicts graphically the primary data obtained from the assay. The results demonstrate that the effect of the abasic modification on siRNA potency is dependent on its position on the sense strand. For this siRNA, particularly abasic modifications in position 10 and 12 were found to enhance potency over the parent compound.

**Table 20. Calculated IC50 values for the results shown in Figure 15.**

| **Duplex ID** | **IC 50 Value (nM)** |
|---|---|
| AD-1000 | 0.281 |
| AD-19040 | 0.126 |
| AD-19041 | 0.094 |
| AD-19042 | 0.126 |
| AD-19043 | 0.092 |

### Example 5 - siRNAs containing bulges. (Reference Example)

Based on the results described above in Example 1 and 2, also encompassed herein are bulges in the sense strand formed by the incorporation of additional nucleotides, which will influence the local structure of the siRNA duplex and enhance the potency and activity of iRNA agents containing these modifications. Non-limiting examples are provided below:

**Table 21. siRNA Duplexes targeting firefly luciferase and containing bulges in the sense strand.**

| **Duplex ID** | **Strand** | **Sequence 5' to 3'** | **Modifications** | **SEQ ID NO.** |
|---|---|---|---|---|
| AD-1000 | S | CUU ACG CUG AGU ACU UCG AdTdT UCG AAG UAC UCA GCG UAA GdTdT | none | 72 |
| | AS | | none | 15 |
| AD-21319 | S | CUU ACG CUAG AGU ACU UCG AdTdT UCG AAG UAC UCA GCG UAA GdTdT | U8-**A**-G9, single bulge | 124 |
| | AS | | none | 15 |
| AD-21320 | S | CUU ACG CUGC AGU ACU UCG AdTdT UCG AAG UAC UCA GCG UAA GdTdT | G9-**C**-A10, single bulge | 125 |
| | AS | | none | 15 |
| AD-21321 | S | CUU ACG CUG ACGU ACU UCG AdTdT UCG AAG UAC UCA GCG UAA GdTdT | A10-**C**-G11, single bulge | 126 |
| | AS | | none | 15 |
| AD-21322 | S | CUU ACG CUG AGA UAC UUC GAdT dT UCG AAG UAC UCA GCG UAA GdTdT | G11-**A**-U12, single bulge | 127 |
| | AS | | none | 15 |

### HeLa Dual Luciferase (Dual Luc or HLDL) Assay Procedure

HeLa cells stably expressing Firefly and Renilla luciferases were cultured and the assay was carried out as described under Example 1.

### Results

Tabulated below are IC50 values derived using results from HeLa cell based dual luciferase assays, as described above. Figure 16a,b depicts graphically the primary data obtained from the assay. The results demonstrate that the effect of the abasic modification on siRNA potency is dependent on its position on the sense strand. For this siRNA, particularly abasic modifications in position 10 and 12 were found to enhance potency over the parent compound.

**Table 22. Calculated IC50 values for the results shown in Figure 16.**

| **Duplex ID** | **IC 50 Value (nM)** |
|---|---|
| AD-1000 | 0.152 |
| AD-21319 | 0.089 |
| AD-21320 | 0.08 |
| AD-21321 | 0.059 |
| AD-1000 | 0.05 |
| AD-21322 | 0.052 |

### Example 6 - siRNAs targeting PTEN and containing mismatch base pairings and nucleobase modifications in the sense strand.

Based on the results described above in Examples 1 and 2, some of the modifications, which showed the most pronounced effect on siRNA potency were applied to a siRNA duplex targeting the endogenous gene PTEN and screened in HeLa cells. Some of the siRNAs synthesized and tested are listed in the Table 23.

**Table 23. siRNA Duplexes targeting PTEN and containing mismatch base parings and nucleobase modifications in the sense strand.**

| **Duplex ID** | **Strand** | **Sequence** | **Modification** | **SEQ ID NO.** |
|---|---|---|---|---|
| AD-19044 | S | AAG UAA GGA CCA GAG ACA AdTdT UUG UCU CUG GUC CUU ACU UdTdT | parent | 128 |
| | AS | | | 129 |
| AD-19045 | S | AAG UAA GGG CCA GAG ACA AdTdT UUG UCU CUG GUC CUU ACU UdTdT | A9-> G | 130 |
| | AS | | | 129 |
| AD-19046 | S | AAG UAA GGC CCA GAG ACA AdTdT UUG UCU CUG GUC CUU ACU UdTdT | A9-> C | 131 |
| | AS | | | 129 |
| A-19047 | S | AAG UAA GGU CCA GAG ACA AdTdT UUG UCU CUG GUC CUU ACU UdTdT | A9-> U | 132 |
| | AS | | | 129 |
| AD-19048 | S | AAG UAA GGA GCA GAG ACA AdTdT UUG UCU CUG GUC CUU ACU UdTdT | C10-> G | 133 |
| | AS | | | 129 |
| AD-19049 | S | AAG UAA GGA ACA GAG ACA AdTdT UUG UCU CUG GUC CUU ACU UdTdT | C10-> A | 134 |
| | AS | | | 129 |
| AD-19050 | S | AAG UAA GGA UCA GAG ACA AdTdT UUG UCU CUG GUC CUU ACU UdTdT | C10-> U | 135 |
| | AS | | | 129 |
| AD-19051 | S | AAG UAA GGY1 CCA GAG ACA AdTdT UUG UCU CUG GUC CUU ACU UdTdT | A9->Y1 | 136 |
| | AS | | | 129 |
| AD-19052 | S | AAG UAA GGA Y1CA GAG ACA AdTdT UUG UCU CUG GUC CUU ACU UdTdT | C10-> Y1 | 137 |
| | AS | | | 129 |
| AD-19053 | S | AAG UAA GGA CY1A GAG ACA AdTdT UUG UCU CUG GUC CUU ACU UdTdT | C11-> Y1 | 138 |
| | AS | | | 129 |
| AD-19054 | S | AAG UAA GGA CCY1 GAG ACA AdTdT UUG UCU CUG GUC CUU ACU UdTdT | A12-> Y1 | 139 |
| | AS | | | 129 |

### PTENAssay Procedure

HeLa cells were cultured in DMEM (Invitrogen) supplemented with 10% FBS and 1x Glutamax and plated in 96-well plates (opaque walls), 10K cells/well in DMEM / 10% FBS w/o antibiotics. Transfection was performed using Lipofectamine 24 hours after plating the cells and after another 24 h of incubation, PTEN assay was performed as described briefly below: Standard PTEN probe sets for use in QuantiGene 1.0 Reagent Systems were obtained from Panomics, Inc. The probe set for a target gene consists of three types of oligonucleotide probes that capture the target RNA to the surface of a plate well and then hybridize to DNA signal amplification molecules. For each target sequence, the software algorithm that designs the probe, identifies one or more continuous regions that can serve as annealing templates for CEs (capture extenders, 5-10 per gene), LEs (label extenders, 10-20 per gene), or BL (blocking probes). QuantiGene1.0 Reagent System was performed according to manufacturer's recommended protocols (Panomics, Inc.). Briefly, the probe set oligonucleotides (250 fmol CE, 500 fmol BL, and 1000 fmol LE) were mixed with the sample, and the mixture was added to an assay well in a 96-well plate covalently coated with capture probe Oligonucleotide. Target RNA was captured during an overnight incubation at 53°C. Unbound material was removed on day 2 by three washes with 200 to 300 µl of wash buffer (0.1x standard saline citrate containing 0.3 g/L lithium laurel sulfate) followed by sequential hybridization of DNA amplifier molecules, then 3'-alkaline phosphatase-conjugated Label Probe oligonucleotides, with three washes after each incubation. After the final wash, luminescent substrate Dioxetane was added to the wells, and following a short incubation the luminescent signal was measured by a luminometer. The "no template" background values were subtracted from each probe set signal. Values were normalized to the GAPDH (Glyceraldehyde-3-phosphate dehydrogenase) values, which is a housekeeping gene. Additionally, a ratio of normalized values was calculated for evaluating mRNA levels which correlates to the extent of gene silencing.

### Results

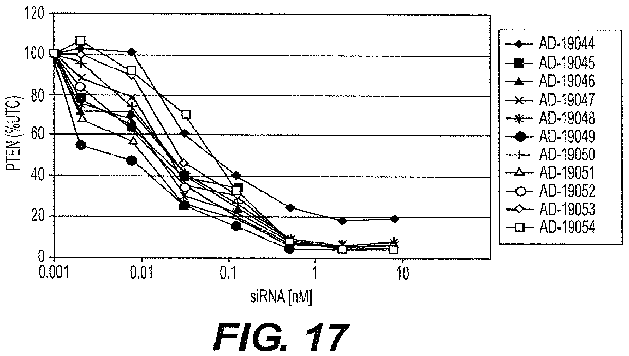

Tabulated below are IC50 values derived using results from HeLa cell based PTEN assay, as described above. Figure 17 depicts graphically the primary data obtained from the assay. The results confirm the findings obtained for the siRNA sequence targeting firefly luciferase described in Examples 1 and 2 in that local destabilization of the central region of the sense strand with mismatch base pairings or modified nucleobases can lead to substantial potency enhancements. For this particular sequence, mismatched base pairings or introduction of 2,4 difluorotoluyl ribonucleotide in positions 9 and 10 were found to significantly enhance potency over the parent unmodified compound.

**Table 24. Calculated IC50 values for the results shown in Figure 17.**

| **Duplex ID** | **IC 50 Value (nM)** |
|---|---|
| AD-19044 | 0.0640 |
| AD-19045 | 0.0240 |
| AD-19046 | 0.0250 |
| AD-19047 | 0.0266 |
| AD-19048 | 0.0169 |
| AD-19049 | 0.0044 |
| AD-19050 | 0.0260 |
| AD-19051 | 0.0108 |
| AD-19052 | 0.0170 |
| AD-19053 | 0.0360 |
| AD-19054 | 0.0620 |

### General References

The oligoribonucleotides and oligoribonucleosides used in accordance with this invention may be with solid phase synthesis, see for example "Oligonucleotide synthesis, a practical approach", Ed. M. J. Gait, IRL Press, 1984; "Oligonucleotides and Analogues, A Practical Approach", Ed. F. Eckstein, IRL Press, 1991 (especially Chapter 1, Modem machine-aided methods of oligodeoxyribonucleotide synthesis, Chapter 2, Oligoribonucleotide synthesis, Chapter 3, 2'-O-Methyloligoribonucleotides: synthesis and applications, Chapter 4, Phosphorothioate oligonucleotides, Chapter 5, Synthesis of oligonucleotide phosphorodithioates, Chapter 6, Synthesis of oligo-2'-deoxyribonucleoside methylphosphonates, and. Chapter 7, Oligodeoxynucleotides containing modified bases. Other particularly useful synthetic procedures, reagents, blocking groups and reaction conditions are described in Martin, P., Helv. Chim. Acta, 1995, 78, 486-504; Beaucage, S. L. and Iyer, R. P., Tetrahedron, 1992, 48, 2223-2311 and Beaucage, S. L. and Iyer, R. P., Tetrahedron, 1993, 49, 6123-6194, or references referred to therein. Modification described in WO 00/44895, WO01/75164, or WO02/44321 can be used herein.

### Phosphate Group References

The preparation of phosphinate oligoribonucleotides is described in U.S. Pat. No. 5,508,270. The preparation of alkyl phosphonate oligoribonucleotides is described in U.S. Pat. No. 4,469,863. The preparation of phosphoramidite oligoribonucleotides is described in U.S. Pat. No. 5,256,775 or U.S..Pat. No. 5,366,878. The preparation of phosphotriester oligoribonucleotides is described in U.S. Pat. No. 5,023,243. The preparation of borano phosphate oligoribonucleotide is described in U.S. Pat. Nos. 5,130,302 and 5,177,198.. The preparation of 3'-Deoxy-3'-amino phosphoramidate oligoribonucleotides is described in U.S. Pat. No. 5,476,925. 3'-Deoxy-3'-methylenephosphonate oligoribonucleotides is described in An, H, et al. J. Org. Chem. 2001, 66, 2789-2801. Preparation of sulfur bridged nucleotides is described in Sproat et al. Nucleosides Nucleotides 1988, 7,651 and Crosstick et al. Tetrahedron Lett. 1989, 30, 4693.

### Sugar Group References

Modifications to the 2' modifications can be found in Verma, S. et al. Annu. Rev. Biochem. 1998, 67, 99-134 and all references therein. Specific modifications to the ribose can be found in the following references: 2'-fluoro (Kawasaki et. al., J. Med. Chem., 1993, 36, 831-841), 2'-MOE (Martin, P. Helv. Chim. Acta 1996, 79, 1930-1938), "LNA" (Wengel, J. Acc. Chem. Res. 1999, 32, 301-310).

### Replacement of the Phosphate Group References

Methylenemethylimino linked oligoribonucleosides, also identified herein as MMI linked oligoribonucleosides, methylenedimethylhydrazo linked oligoribonucleosides, also identified herein as MDH linked oligoribonucleosides, and methylenecarbonylamino linked oligonucleosides, also identified herein as amide-3 linked oligoribonucleosides, and methyleneaminocarbonyl linked oligonucleosides, also identified herein as amide-4 linked oligoribonucleosides as well as mixed backbone compounds having, as for instance, alternating MMI and PO or PS linkages can be prepared as is described in U.S. Pat. Nos. 5,378,825, 5,386,023, 5,489,677 and in published PCT applications PCT/US92/04294 and PCT/US92/04305 (published as WO 92/20822 WO and 92/20823, respectively). Formacetal and thioformacetal linked oligoribonucleosides can be prepared as is described in U.S. Pat. Nos. 5,264,562 and 5,264,564. Ethylene oxide linked oligoribonucleosides can be prepared as is described in U.S. Pat. No. 5,223,618. Siloxane replacements are described in Cormier,J.F. et al. Nucleic Acids Res. 1988, 16, 4583. Carbonate replacements are described in Tittensor, J.R. J. Chem. Soc. C 1971, 1933. Carboxymethyl replacements are described in Edge, M.D. et al. J. Chem. Soc. Perkin Trans. 1 1972, 1991. Carbamate replacements are described in Stirchak, E.P. Nucleic Acids Res. 1989, 17, 6129.

### Replacement of the Phosphate-Ribose Backbone References

Cyclobutyl sugar surrogate compounds can be prepared as is described in U.S. Pat. No. 5,359,044. Pyrrolidine sugar surrogate can be prepared as is described in U.S. Pat. No. 5,519,134. Morpholino sugar surrogates can be prepared as is described in U.S. Pat. Nos. 5,142,047 and 5,235,033, and other related patent disclosures. Peptide Nucleic Acids (PNAs) are known per se and can be prepared in accordance with any of the various procedures referred to in Peptide Nucleic Acids (PNA): Synthesis, Properties and Potential Applications, Bioorganic & Medicinal Chemistry, 1996, 4, 5-23. They may also be prepared in accordance with U.S. Pat. No. 5,539,083.

### Terminal Modification References

Terminal modifications are described in Manoharan, M. et al. Antisense and Nucleic Acid Drug Development 12, 103-128 (2002) and references therein.

### Base References

N-2 substituted purine nucleoside amidites can be prepared as is described in U.S. Pat. No. 5,459,255. 3-Deaza purine nucleoside amidites can be prepared as is described in U.S. Pat. No. 5,457,191. 5,6-Substituted pyrimidine nucleoside amidites can be prepared as is described in U.S. Pat. No. 5,614,617. 5-Propynyl pyrimidine nucleoside amidites can be prepared as is described in U.S. Pat. No. 5,484,908. Additional references can be disclosed in the above section on base modifications.

## Claims

1. A double-stranded iRNA agent with increased RNAi silencing activity, wherein the iRNA agent comprises two 21 nucleotide-long strands, wherein the strands form a double-stranded region of 19 consecutive base pairs having a two-nucleotide overhang at the 3'-end, wherein the cleavage site region corresponds to positions 9-12 from the 5'-end of the sense strand, said double-stranded iRNA agent comprising:
a. an antisense strand that is complementary to a target gene; and
b. a sense strand that is complementary to said antisense strand and comprises at least one of the following modified nucleotides in the following positions from the 5' end:
(i) a ribonebularine in position 9;
(ii) a ribo-2-aminopurine in position 12; and
(iii) an abasic nucleotide in position 10.

## Patentansprüche

1. Doppelsträngiges iRNA-Mittel mit gesteigerter RNAi-Silencing-Aktivität, wobei das iRNA-Mittel zwei 21 Nukleotide lange Stränge umfasst, wobei die Stränge einen doppelsträngigen Bereich von 19 aufeinander folgenden Basenpaaren bilden, welcher einen Überhang von zwei Nukleotiden am 3'-Ende aufweist, wobei der Bereich der Spaltstelle den Positionen 9 bis 12 vom 5'-Ende des Sense-Stranges aus entspricht, wobei das doppelsträngige iRNA-Mittel umfasst:
a. einen Antisense-Strang, der komplementär zu einem Zielgen ist und
b. einen Sense-Strang, der komplementär zu dem Antisense-Strang ist und mindestens eines der folgenden modifizierten Nukleotide in den folgenden Positionen vom 5'-Ende aus umfasst:
(i) ein Ribonebularin in Position 9,
(ii) ein Ribo-2-aminopurin in Position 12 und
(iii) ein abasisches Nukleotid in Position 10.

## Revendications

1. Agent d'ARNi double brin doté d'une activité de silençage d'iARN augmentée, où l'agent d'ARNi comprend deux brins d'une longueur de 21 nucléotides, dans lequel les brins forment une région double brin de 19 paires de bases consécutives comportant un dépassement de deux nucléotides à l'extrémité 3', dans lequel la région du site de clivage correspond aux positions 9 à 12 à partir de l'extrémité 5' du brin sens, ledit agent d'ARNi double brin comprenant :
a. un brin antisens qui est complémentaire d'un gène cible ; et
b. un brin sens qui est complémentaire dudit brin antisens et comprend au moins l'un des nucléotides modifiés suivants dans les positions suivantes à partir de l'extrémité 5' :
(i) une ribonébularine en position 9 ;
(ii) une ribo-2-aminopurine en position 12 ; et
(iii) un nucléotide abasique en position 10.
